# EUROPEAN PATENT SPECIFICATION

(11) **EP 2 329 839 B1**
(45) Date of publication and mention of the grant of the patent: **16.09.2015**
(21) Application number: 10186174.8
(22) Date of filing: 10.01.2003
(51) Int. Cl.: A61P 3/04, A61K 38/26, A61K 38/22

(54) **Modification of feeding behavior by GLP-1 and PYY**
Änderung des Fütterverhaltens durch GLP-1 und PYY
Modification du comportement d'alimentation par GLP-1 et PYY

(30) Priority: 10.01.2002 GB 0200507; 28.06.2002 US 392109 P; 24.09.2002 WO PCT/US02/31944
(43) Date of publication of application: 08.06.2011
(62) Divisional of application: 03700848.9
(73) Proprietor: Imperial Innovations Limited, London SW7 2PG (GB); Oregon Health and Science University, Portland, Oregon 97201 (US)
(72) Inventor: Cowley, Michael, Balwyn, Victoria 3103 (AU); Cone, Roger, Oregon City, OR 97045 (US); Low, Malcolm, Lake Oswego, OR 97035 (US); Butler, Andrew, Portland, OR 97201 (US); Bloom, Stephen Robert, London, SW7 2QA (GB); Small, Caroline Jane, London, SW7 2QA (GB); Batterham, Rachel Louise, London, SW7 2QA (GB); Ghatei, Mohammad Ali, London, SW7 2QA (GB)
(74) Representative: Brady, Paul Andrew

(56) References cited:
- WETTERGREN A ET AL: "Glucagon-like peptide-1 7-36 amide and peptide YY have additive inhibitory effect on gastric acid secretion in man.", SCANDINAVIAN JOURNAL OF GASTROENTEROLOGY JUN 1997 LNKD- PUBMED:9200286, vol. 32, no. 6, June 1997 (1997-06), pages 552-555, XP009147027, ISSN: 0036-5521
- FUNG L C ET AL: "Glucagon-like peptide-1-(7-36) amide and peptide YY mediate intraduodenal fat-induced inhibition of acid secretion in dogs.", ENDOCRINOLOGY JAN 1998 LNKD- PUBMED:9421414, vol. 139, no. 1, January 1998 (1998-01), pages 189-194, XP002632655, ISSN: 0013-7227
- BATTERHAM R L ET AL: "Gut hormone PYY Ä3-36Ü physiologically inhibits food intake", NATURE, NATURE PUBLISHING GROUP, LONDON, GB, vol. 418, 8 August 2002 (2002-08-08), pages 650-654, XP002984562, ISSN: 0028-0836, DOI: DOI:10.1038/NATURE00887
- GUTZWILLER J-P ET AL: "GLUCAGON-LIKE PEPTIDE-1: A POTENT REGULATOR OF FOOD INTAKE IN HUMANS", GUT, BRITISH MEDICAL ASSOCIATION, LONDON, UK, vol. 44, 1 January 1999 (1999-01-01), pages 81-86, XP002918974, ISSN: 0017-5749
- STEINERT ROBERT E ET AL: "Oral administration of glucagon-like peptide 1 or peptide YY 3-36 affects food intake in healthy male subjects.", THE AMERICAN JOURNAL OF CLINICAL NUTRITION OCT 2010 LNKD- PUBMED:20720259, vol. 92, no. 4, October 2010 (2010-10), pages 810-817, XP009147021, ISSN: 1938-3207
- Nicola M. Neary ET AL: "Peptide YY 3-36 and Glucagon-Like Peptide-1 7-36 Inhibit Food Intake Additively", Endocrinology, vol. 146, no. 12, 1 December 2005 (2005-12-01), pages 5120-5127, XP055110181, ISSN: 0013-7227, DOI: 10.1210/en.2005-0237
- AKILA DE SILVA ET AL: "Gut Hormones and Appetite Control: A Focus on PYY and GLP-1 as Therapeutic Targets in Obesity", GUT AND LIVER, vol. 6, no. 1, 1 January 2012 (2012-01-01) , page 10, XP055110261, ISSN: 1976-2283, DOI: 10.5009/gnl.2012.6.1.10
- Akila De Silva ET AL: "Gut Hormones and Appetite Control: A Focus on PYY and GLP-1 as Therapeutic Targets in Obesity", Gut and Liver, vol. 6, no. 1, 1 January 2012 (2012-01-01) , page 10, XP055110261, ISSN: 1976-2283, DOI: 10.5009/gnl.2012.6.1.10

## Description

### FIELD

This application relates to agents for use in treating obesity and/or diabetes.

### BACKGROUND

According to the National Health and Nutrition Examination Survey (NHANES III, 1988 to 1994), between one third and one half of men and women in the United States are overweight. In the United States, sixty percent of men and fifty-one percent of women, of the age of 20 or older, are either overweight or obese. In addition, a large percentage of children in the United States are overweight or obese.

The cause of obesity is complex and multi-factorial. Increasing evidence suggests that obesity is not a simple problem of self-control but is a complex disorder involving appetite regulation and energy metabolism. In addition, obesity is associated with a variety of conditions associated with increased morbidity and mortality in a population. Although the etiology of obesity is not definitively established, genetic, metabolic, biochemical, cultural and psychosocial factors are believed to contribute. In general, obesity has been described as a condition in which excess body fat puts an individual at a health risk.

There is strong evidence that obesity is associated with increased morbidity and mortality. Disease risk, such as cardiovascular disease risk and type 2 diabetes disease risk, increases independently with increased body mass index (BMI). Indeed, this risk has been quantified as a five percent increase in the risk of cardiac disease for females, and a seven percent increase in the risk of cardiac disease for males, for each point of a BMI greater than 24.9 (see Kenchaiah et al., N. Engl. J. Med. 347:305, 2002; Massie, N. Engl. J. Med. 347:358, 2002). In addition, there is substantial evidence that weight loss in obese persons reduces important disease risk factors. Even a small weight loss, such as 10% of the initial body weight in both overweight and obese adults has been associated with a decrease in risk factors such as hypertension, hyperlipidemia, and hyperglycemia.

Although diet and exercise provide a simple process to decrease weight gain, overweight and obese individuals often cannot sufficiently control these factors to effectively lose weight. Pharmacotherapy is available; several weight loss drugs have been approved by the Food and Drug Administration that can be used as part of a comprehensive weight loss program. However, many of these drugs have serious adverse side effects. When less invasive methods have failed, and the patient is at high risk for obesity related morbidity or mortality, weight loss surgery is an option in carefully selected patients with clinically severe obesity. However, these treatments are high-risk, and suitable for use in only a limited number of patients. It is not only obese subjects who wish to lose weight. People with weight within the recommended range, for example, in the upper part of the recommended range, may wish to reduce their weight, to bring it closer to the ideal weight. Thus, a need remains for agents that can be used to effect weight loss in overweight and obese subjects.

Batterham et al, Nature, 418, 2002, 650 (XP 002984562) discloses that gut hormone PYY₃₋₃₆ physiologically inhibits food intake. Gutzwiller et al, Gut 1999 (444) 81-86 (XP 002918974) discloses that GLP-1 is a regulator of food intake in humans.

### SUMMARY

Disclosed herein are findings that peripheral administration of PYY, or an agonist thereof, and GLP-1, or an agonist thereof, to a subject results in decreased food intake, caloric intake, and appetite, and an alteration in energy metabolism. The subject is a human subject.

Also disclosed are findings that co-administration of PYY, or an agonist thereof, for example, PYY₃₋₃₆, with GLP-1 or an agonist thereof to a subject results in a greater decrease in food intake, caloric intake, and appetite, than when PYY or an agonist thereof is administered alone. The effect is synergistic.

The invention provides a Peptide YY (PYY) or an agonist thereof and a Glucagon-like peptide-1 (GLP-1) or an agonist thereof for use in treating obesity and/or diabetes in a human subject,
wherein the PYY or agonist thereof, and GLP-1 or agonist thereof are in a form suitable for peripheral administration, and
wherein the PYY or agonist thereof is for administration at a molar equivalent amount of a PYY₃₋₃₆ dose of no more than 2.2 nmol per kg body weight of said subject.

The invention also provides a non-therapeutic method for:
a) decreasing calorie intake or food intake,
b) reducing weight gain, or inducing weight loss,
c) controlling any one or more of appetite, satiety and hunger,
d) maintaining desired body weight, a desired Body Mass Index, and/or a desired appearance and good health,
e) improving lipid profile,
in a human subject comprising the administration of Peptide YY (PYY) or an agonist thereof and a Glucagon-like peptide-1 (GLP-1) or an agonist thereof, wherein the PYY or agonist thereof, and GLP-1 or agonist thereof are in a form suitable for peripheral administration,
wherein the PYY or agonist thereof is for administration at a molar equivalent amount of a PYY3-36 dose of no more than 2.2 nmol per kg body weight of a subject, and
wherein the subject desires to lose weight.

The GLP-1 or an agonist thereof may be administered simultaneously or substantially simultaneously as the PYY or agonist thereof, or sequentially, in any order. The PYY or agonist thereof and the GLP-1 or agonist thereof may be administered in a single pharmaceutical composition or in separate compositions, and they may be administered by the same route or my different routes.

The foregoing and other features and advantages will become more apparent from the following detailed description of several embodiments, which proceeds with reference to the accompanying figures.

### BRIEF DESCRIPTION OF THE FIGURES

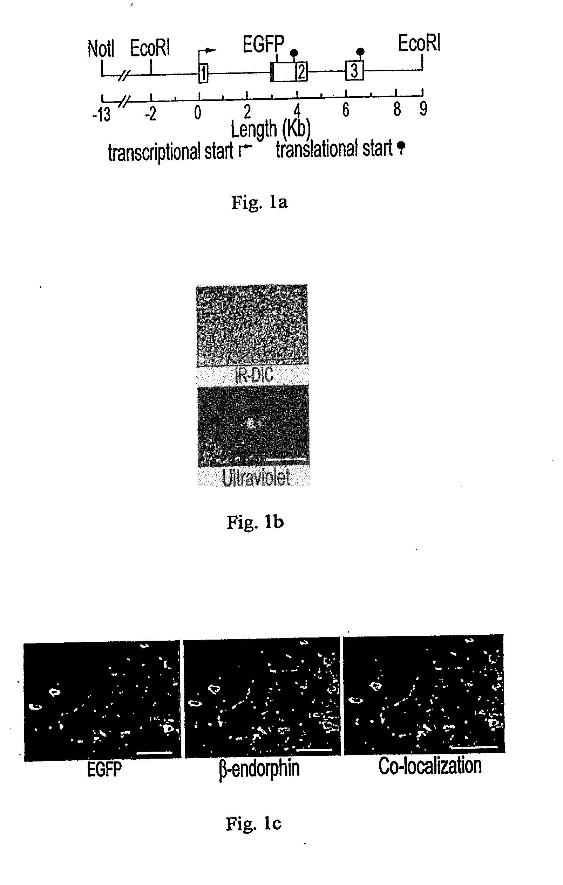

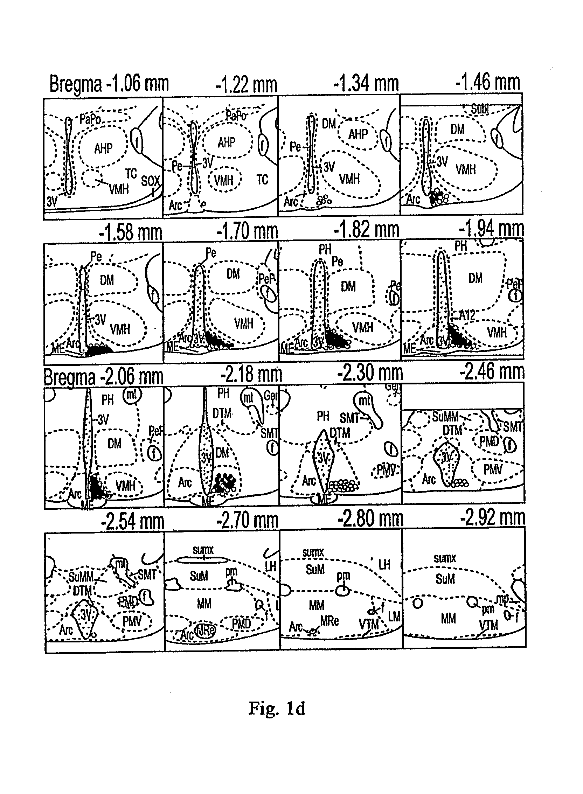

**Fig. 1** is a set of diagrams and digital images showing the generation of transgenic mice expressing EGFP in ARC POMC neurons. Fig. 1a is a schematic diagram of the structure of the POMC-EGFP transgene. Fig. 1b is a digital image showing the identification of a single POMC neuron (arrowhead on recording electrode tip) by EGFP fluorescence (upper) and IR-DIC microscopy (lower) in a living ARC slice prior to electrophysiological recordings. Fig. 1c is a set of digital images showing the co-localization (bright, on right) of EGFP (left) and ß-endorphin immunoreactivity (middle) in ARC POMC neurons. Scale bars: b & c, 50 µm. Fig. 1d is a set of diagrams showing the distribution of EGFP-positive neuronal soma throughout the ARC nucleus. **o** = 5 cells, ● = 10 cells.

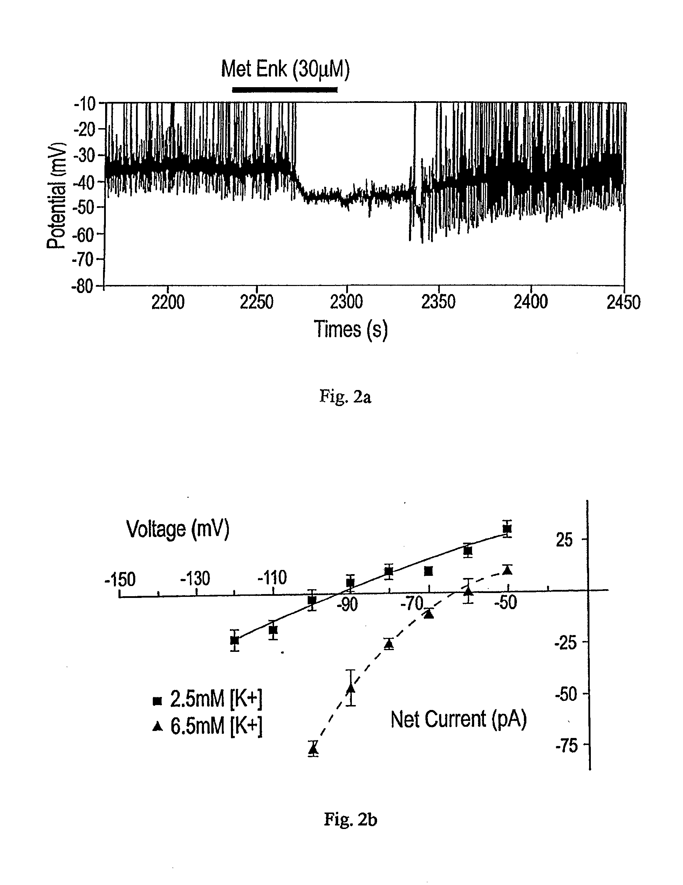

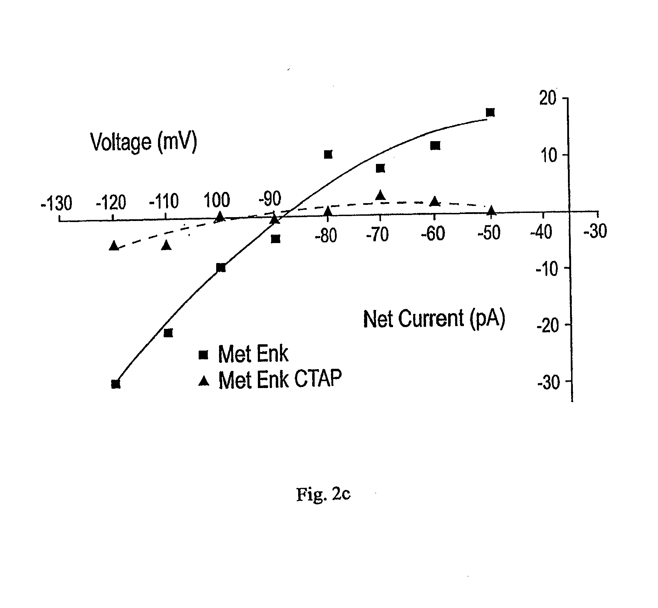

**Fig. 2** is a tracing and graphs showing activation of MOP-Rs hyperpolarizes the EGFP-labeled POMC neurons by opening G protein-coupled inwardly-rectifying potassium channels. Fig. 2a is a tracing showing met-enkephalin hyperpolarizes POMC neurons and inhibits all action potentials. The horizontal bar indicates the time when 30 µM Met-Enk was bath-applied to the slice. Fig. 2b is a graph showing met-enkephalin current and reversal potential is shifted by extracellular K⁺ concentration. Fig. 2c is a graph showing met-enkephalin activates MOP-Rs on POMC neurons. A Met-Enk (30 µM) current was observed and the MOP-R specific antagonist CTAP (1 µM) was applied for 1 minute. Following CTAP Met-Enk elicited no current. The figure is representative of three experiments.

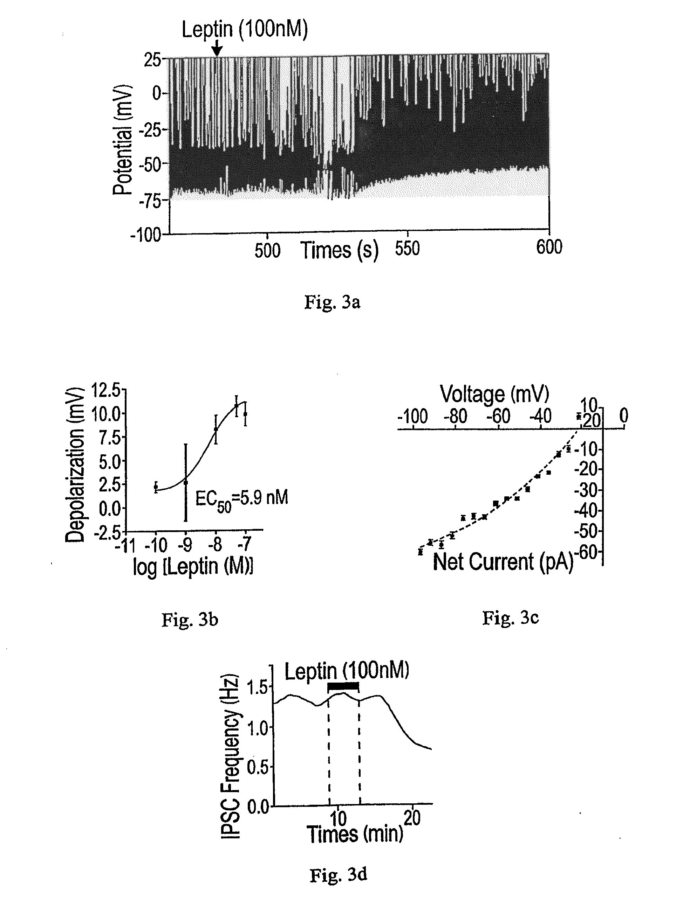

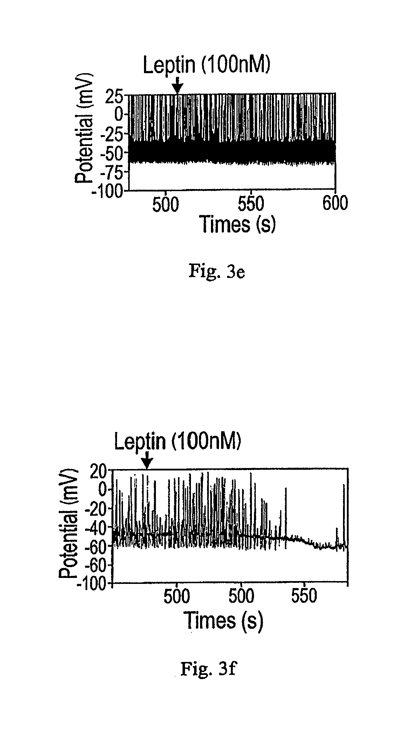

**Fig. 3** are tracings and graphs demonstrating that leptin depolarizes POMC neurons via a non-specific cation channel, and decreases GABAergic tone onto POMC cells. Fig. 3a is a tracing demonstrating that leptin depolarizes POMC neurons and increases the frequency of action potentials within 1 to 10 minutes of addition. The figure is a representative example of recordings made from 77 POMC neurons. Fig. 3b is a graph showing that leptin causes a concentration dependent depolarization of POMC cells. The depolarization caused by leptin was determined at 0.1, 1, 10, 50, and 100 nM (EC₅₀ =5.9 nM) in (8, 7, 9, 3, 45) cells respectively. Fig. 3c is a graph showing that leptin depolarizes POMC cells by activating a nonspecific cation current. The figure is representative of the response in 10 cells. Fig. 3d is a graph showing that leptin decreases the frequency of IPSCs in POMC cells. The figure is an example of 5 cells in which leptin (100 nM) decreased the frequency of IPSCs. Fig. 3e is a tracing demonstrating that leptin had no effect on 5 adjacent non-fluorescent ARC neurons. Fig. 3f is a tracing showing that leptin hyperpolarized 5 non-fluorescent ARC neurons.

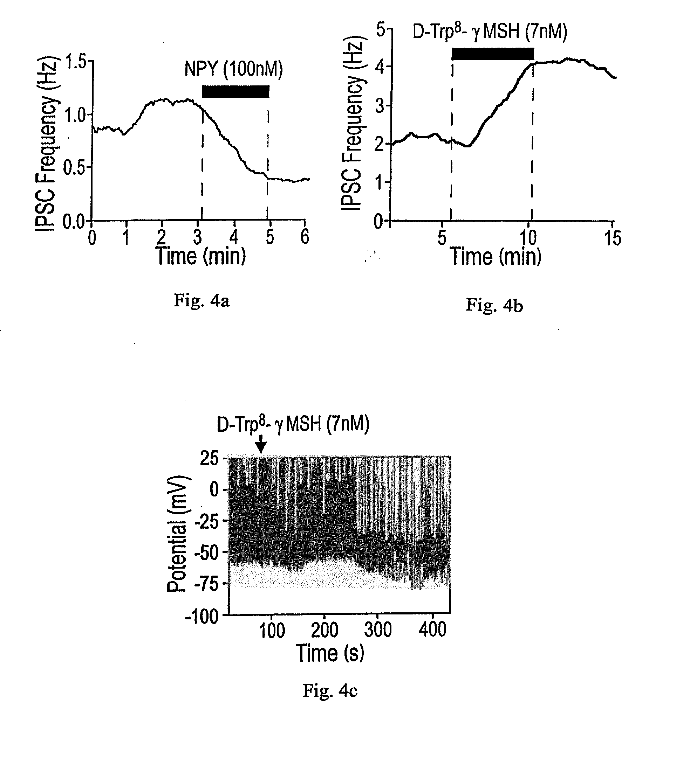

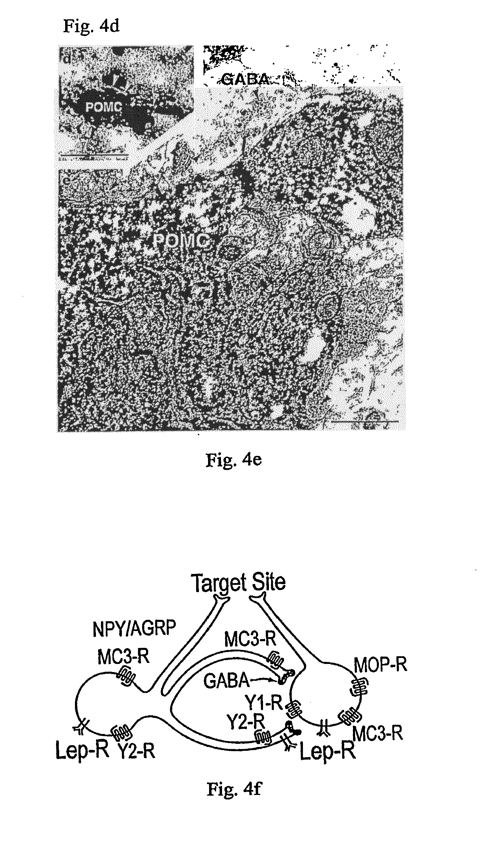

**Fig. 4** is a set of images showing that the GABAergic inputs to POMC cells are from NPY neurons that co-express GABA. Fig. 4a is a graph showing that NPY decreases the frequency of mini IPSCs in POMC neurons. Fig. 4b is a graph demonstrating that D-Trp⁸-γMSH (7nM), a dose that selectively activates MC3-R, increases the frequency of GABAergic IPSCs in POMC neurons. Fig. 4c is a tracing showing that D-Trp⁸-γMSH hyperpolarizes POMC neurons. Figs. 4a, 4b and 4c are representative. Fig. 4d is a set of digital images demonstrating that expression of NPY in nerve terminals adjacent to POMC neurons in the ARC. NPY nerve terminals (black, arrowheads); POMC neuronal soma (grey). Scale bar, 10 µm. Fig. 4e is a digital image showing expression of GABA and NPY in nerve terminals synapsing onto POMC neurons in the ARC. GABA immunoreactivity (10 nm gold particles, arrowheads without tail) and NPY immunoreactivity (25 nm gold particles, arrows with tail) are in separate vesicle populations co-localized within synaptic boutons that make direct contact with the soma of POMC neurons (DAB contrasted with uranyl acetate and lead citrate, diffuse black in cytoplasm). Scale bar, 1 µm. Fig. 4f is a diagram of the model of NPY/GABA and POMC neurons in the ARC.

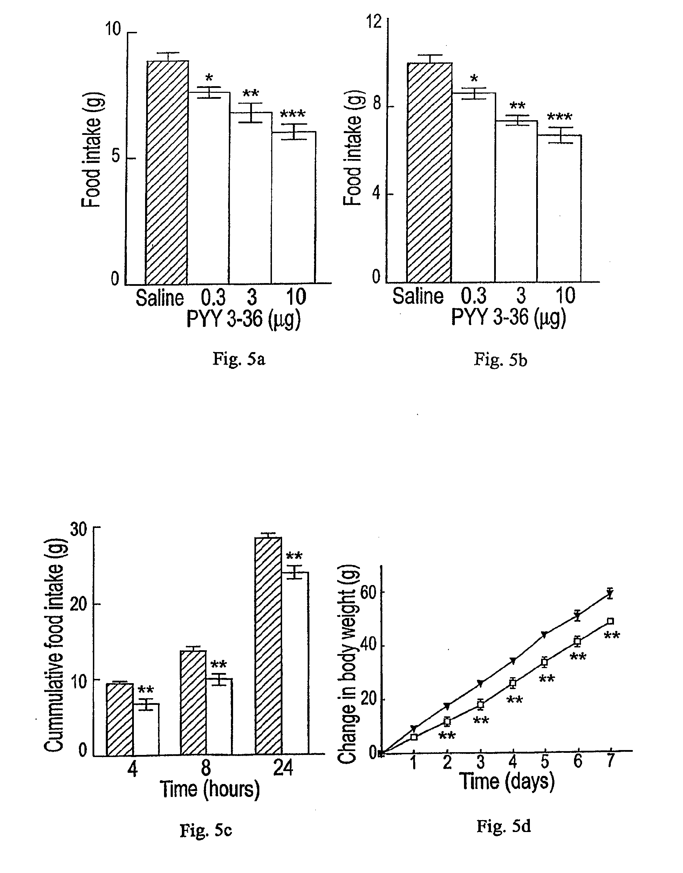

Fig. 5 is a set of graphs relating to the feeding response to PYY₃₋₃₆ in rats. Fig. 5a is a bar graph of dark-phase feeding tabulating food intake after intraperitoneal injection of PYY₃₋₃₆. Freely feeding rats were injected with PYY₃₋₃₆ at the doses indicated (µg/100g), or saline, just prior to 'lights off' and 4-hour cumulative food intake was measured. Results are the mean ± s.e.m. (n = 8 per group), * = p < 0.05, ** = p < 0.01, *** = < 0.001 compared to saline. Fig. 5b is a bar graph of food intake after intraperitoneal injection of PYY₃₋₃₆. Fasted rats were injected with PYY₃₋₃₆ at the doses indicated (µg/100g), or saline, and 4-hour cumulative food intake was measured. Results are shown as the mean ± s.e.m. (n = 8 per group), * = p < 0.05, ** = p < 0.01, *** = < 0.001 compared to saline. Fig. 5c is a bar graph of cumulative food intake after intraperitoneal injection of saline or PYY₃₋₃₆. Fasted rats were injected with either saline (closed bars) or PYY₃₋₃₆ 5µg/100g (open bars) and cumulative food intake measured at the time points indicated. Results are expressed as mean ± s.e.m. (n = 12 per group), ** = p < 0.01 compared to saline. Fig. 5d is a line graph of body weight gain during chronic treatment with PYY₃₋₃₆. Rats were injected intraperitoneally with PYY₃₋₃₆ 5µg/100g (open squares) or saline (filled inverted triangles) twice daily for 7 days. Body weight gain was calculated each day. Results are expressed as mean ± s.e.m. (n = 12 per group) ** = p < 0.01 compared to saline.

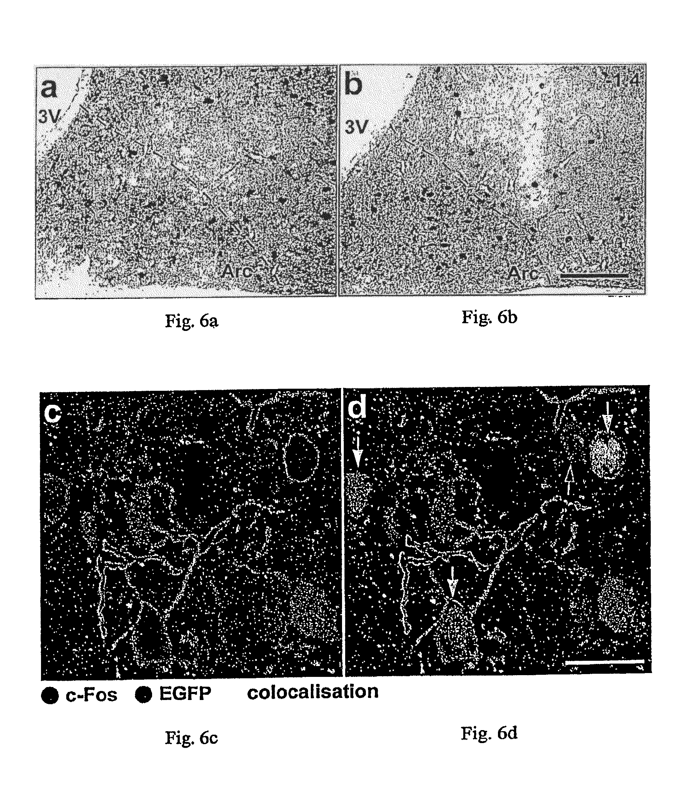

**Fig. 6** is a set of digital images of c-fos expression in *Pomc-EGFP* mice. Figs. 6a and 6b are digital images of representative sections (bregma -1.4 mm²²) of c-fos expression in the arcuate nucleus of *Pomc-EGFP* mice response to intraperitoneal saline (Fig. 6a) or PYY₃₋₃₆ (5µg/100g) (Fig. 6b). Scale bar 100 µm. 3V, third ventricle; Arc, arcuate nucleus. Figs. 6c and 6d are digital images of representative sections showing POMC-EGFP neurons (Fig. 6c) and c-fos immunoreactivity (Fig. 6d) either co-localizing (bright arrows) or alone (single darker arrow). Scale bar 25 µm.

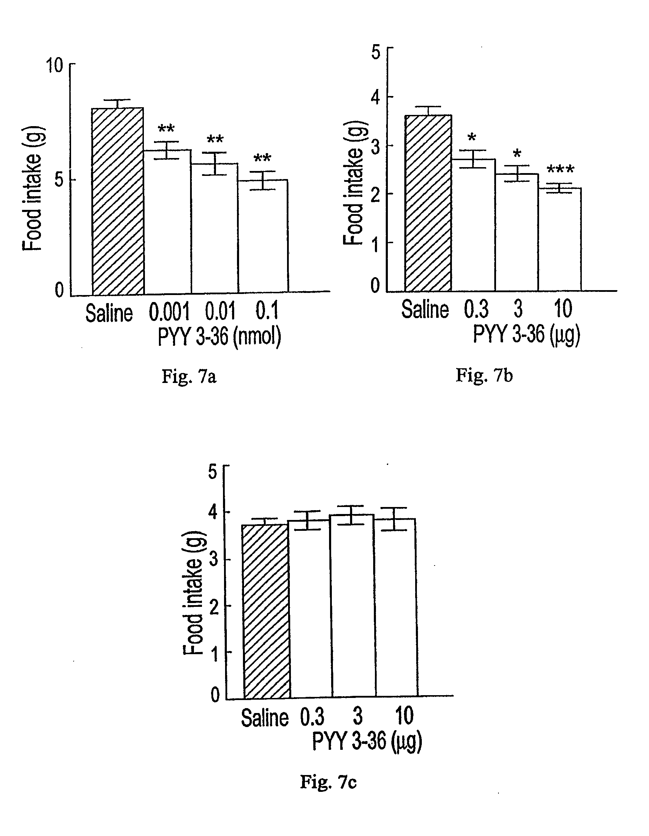

**Fig. 7** is a set of bar graphs relating to intra-arcuate PYY₃₋₃₆ in rats and feeding effects of IP PYY₃₋₃₆ in *Y2r-* null mice. Fig. 7a is a bar graph of food intake following intra-arcuate PYY₃₋₃₆ injection. Fasted rats were injected with saline or PYY₃₋₃₆ into the arcuate nucleus at the doses indicated. Post-injection 2-hour food intake was measured, ** = p < 0.01 compared to saline. Figs. 7b and 7c are bar graphs of feeding response to PYY₃₋₃₆ in Y2r-null mice following IP administration: wild type littermates mice (Fig. 7b) and *Y2r*-null mice (Fig. 7c), fasted for 24 hours, were injected with PYY₃₋₃₆ at the doses indicated (µg/100g), or saline, and 4-hour cumulative food intake was measured. Results are the mean ± s.e.m. (n = 5 per group), * = p < 0.05, ** = p < 0.01 compared to saline.

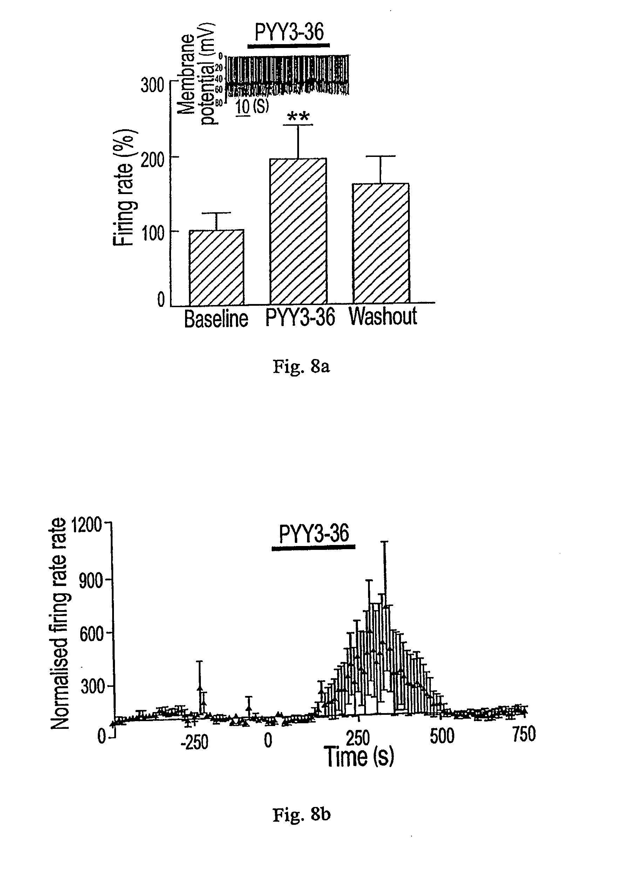

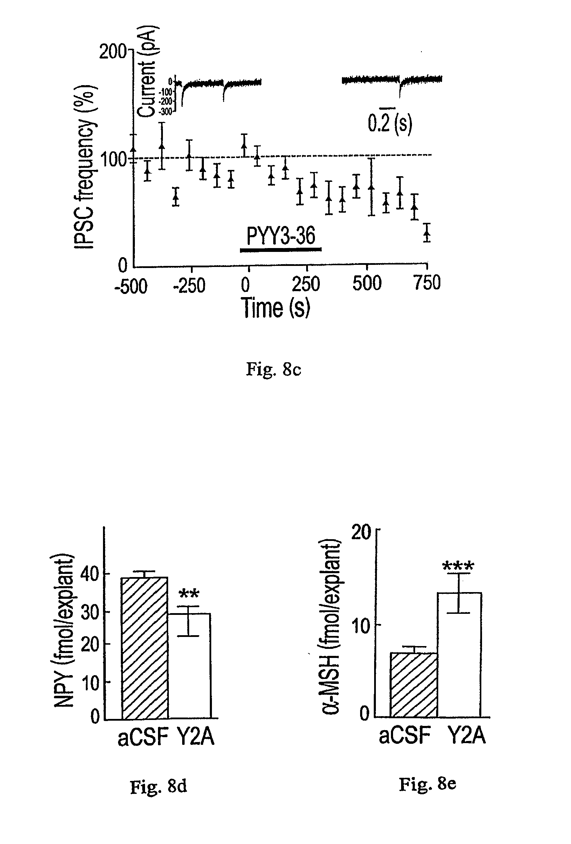

**Fig. 8** is a set of images relating to the electrophysiological and neuropeptide responses to PYY₃₋₃₆ and Y2A. Fig. 8a is a tracing showing the effect of PYY₃₋₃₆ (10 nM) on the frequency of action potentials in POMC neurons (whole-cell configuration recordings; n = 22) * p< 0.05. PYY₃₋₃₈ was administered at time D for 3 minutes; baseline, -3 to 0 minute; PYY₃₋₃₆, 2-5 minutes; and wash-out, 8-11 minutes. Inset shows a representative recording of membrane potential and action potential frequency. Fig. 8b is a graph of the effect if PYY₃₋₃₈ (10nM) on the frequency of action potentials in loose cell-attached patch recordings (n=8). Data from individual cells were normalized to the firing rate for the 200s before PYY₃₋₃₈ addition. Fig. 8c is a tracing and a graph of the effect of PYY₃₋₃₆ (50nM) on spontaneous IPSCs onto POMC neurons (n=13). Inset shows a representative recording of IPSCs before and after PYY₃₋₃₆ (50nM), respectively. Results in Fig. 8a-8c are expressed as mean V s.e.m. Fig. 8d and 8e are bar graphs showing NPY (Fig. 8d) and %-MSH (Fig. 8e) released from hypothalamic explants in response to Y2A. Hypothalamic slices were incubated with artificial CSF (aCSF), with or without 50nM Y2A, for 45 minutes. Results are expressed as mean V s.e.m. (n=40); ** = p<0.01; *** = p<0.001 compared to saline.

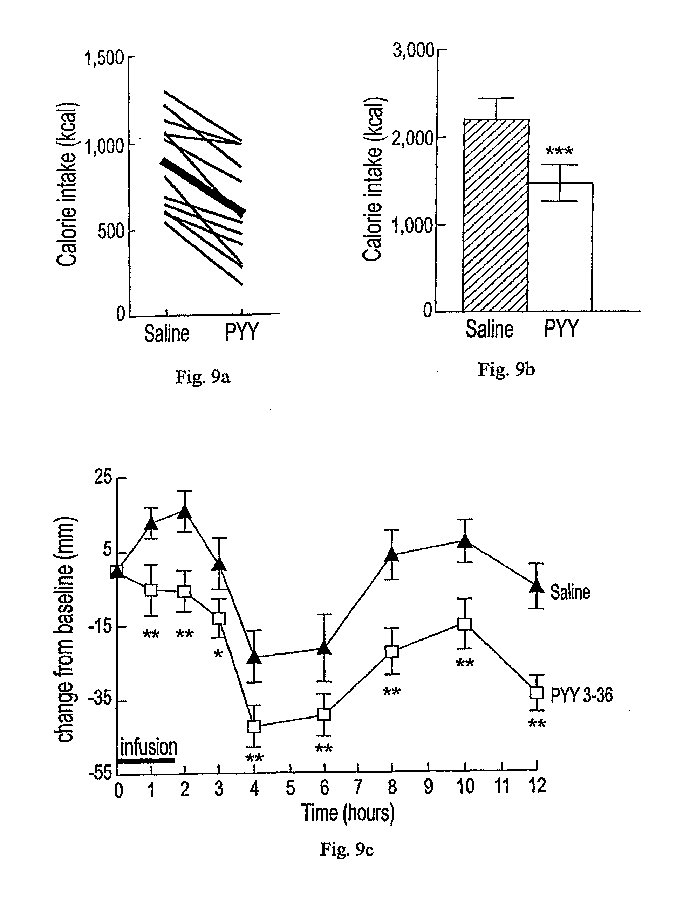

**Fig. 9** is a set of graphs showing the effect of PYY₃₋₃₆ infusion on appetite and food intake in human subjects. Fig. 9a is a graph of the calorie intake from a "free-choice" buffet meal 2 hours after infusion with saline or PYY₃₋₃₆. The thin lines indicate individual changes in calorie intake for each subject between saline and PYY₃₋₃₆ administration. The thick line represents mean change between the two infusions (n = 12). Fig. 9b is a graph of the 24-hour calorie intake following infusion with saline or PYY₃₋₃₆. Total calorie intake, as assessed by food diaries, is shown for the 24-hour period following either saline or PYY₃₋₃₆ infusion. Data is given as mean ± s.e.m. (n = 12), *** = p < 0.0001 compared to saline. Fig. 9c is a graph of the appetite score (relative scale). Visual analogue scores (Raben et al., Br. J. Nutr. 73, 517-30, 1995) show perceived hunger during and after infusions. The results are presented as change from baseline scores and are the mean ± s.e.m. for all 12 subjects.

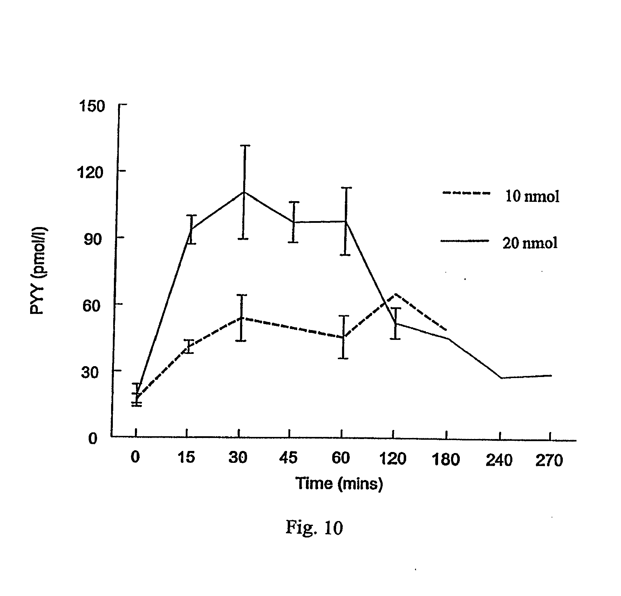

**Fig. 10** shows plasma PYY₃₋₃₆ levels following subcutaneous administration of 10 nmols PYY₃₋₃₆ (broken line) and 20 nmols PYY₃₋₃₆ (solid line).

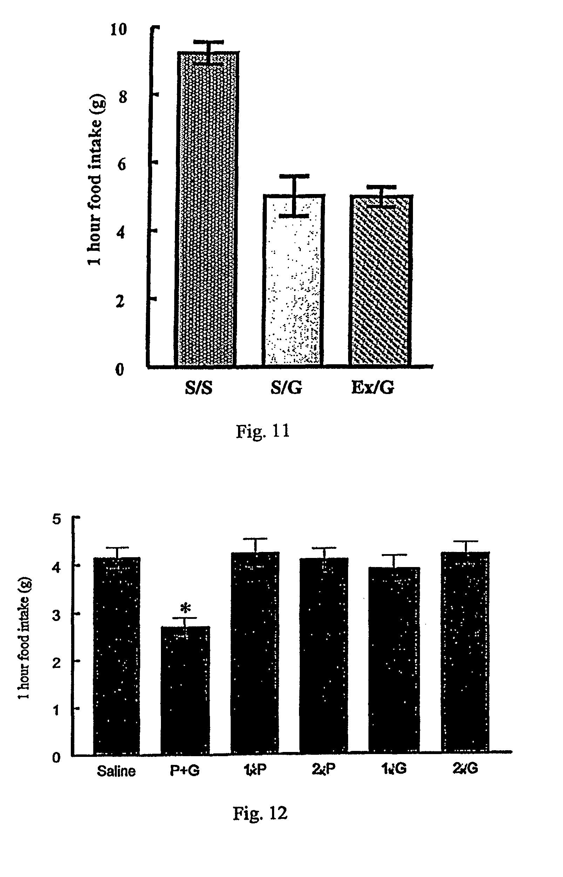

**Fig. 11** shows the effect on 1-hour food intake in non-fasted rats of IP administration of GLP-1 in the presence or absence of concomitant exendin 9-39 in the arcuate nucleus of the rat. S = Saline, G = GLP-1 (25 nmol/kg), and Ex = exendin 9-39 (20 nmoles/kg).
**Fig. 12** shows the effects of co-administration of PYY₃₋₃₆ and GLP-1 on 1-hour food intake in non-fasted rats injected (intraperitoneally) prior to the onset of the dark phase. IP GLP-1 (30µg/kg = 1xG or 60µg/kg = 2xG in 500 µl saline) or IP PYY₃₋₃₆ (3µg/kg = 1xP or 6µg/kg = 2xP in 500 µl saline). Co-administration (P+G group) is GLP-1 30µg/kg and PYY₃₋₃₆ 3µg/kg combined.

### SEQUENCE LISTING

The nucleic and amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand.

### DETAILED DESCRIPTION

### I. Abbreviations

α-**MSH:** alpha melanocortin stimulating hormone
**Arc:** arcuate nucleus
**EPSP:** excitatory postsynaptic potential
**GABA:** γaminobutyric acid
**GFP, EGFP:** green fluorescent protein
**GLP-1:** glucagons-like peptide-1
**ICV:** intracerebroventricular
**IP:** intraperitoneal
**IPSC:** inhibitory postsynaptic current
**kb:** kilobase
**kg:** kilogram
**MOP-R:** µ-opiod receptor
**MV:** millivolts
**NPY:** neuropeptide Y
**Oxm:** oxyntomodulin
**pmol:** picomole
**POMC:** proopiomelanocortin
**RIA:** radioimmunoassay
**RPA:** RNase protection assay
**s.e.m:** standard error of the mean
**TH:** tyrosine hydroxylase
µ**M:** micromolar
**V:** volts
**Y2A:** N-acetyl (Leu²⁸, Leu³¹) NPY (24-36)

### II. Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).
In order to facilitate review of the various embodiments of this disclosure, the following explanations of specific terms are provided:
**Action potential:** A rapidly propagated electrical message that speeds along an axon of a neuron and over the surface membrane of many muscle and glandular cells. In axons they are brief, travel at constant velocity, and maintain a constant amplitude. Like all electrical messages of the central nervous system, the action potential is a membrane potential change caused by the flow of ions through ion channels in the membrane. In one embodiment, an action potential is a regenerative wave of sodium permeability.
**Anorexia:** A lack or loss of the appetite for food. In one embodiment, anorexia is a result of "anorexia nervosa." This is an eating disorder primarily affecting females, usually with onset in adolescence, characterized by refusal to maintain a normal minimal body weight, intense fear of gaining weight or becoming obese, and a disturbance of body image resulting in a feeling of being fat or having fat in certain areas even when extremely emaciated, undue reliance on body weight or shape for self-evaluation, and amenorrhea. Associated features often include denial of the illness and resistance to psychotherapy, depressive symptoms, markedly decreased libido, and obsessions or peculiar behavior regarding food, such as hoarding. The disorder is divided into two subtypes, a restricting type, in which weight loss is achieved primarily through diet or exercise, and a binge-eating/purging type, in which binge eating or purging behavior also occur regularly.
**Antagonist:** A substance that tends to nullify the action of another, as an agent that binds to a cell receptor without eliciting a biological response, blocking binding of substances that could elicit such responses.
**Appetite:** A natural desire, or longing for food. In one embodiment, appetite is measured by a survey to assess the desire for food. Increased appetite generally leads to increased feeding behavior.
**Appetite Suppressants:** Compounds that decrease the desire for food. Commercially available appetite suppressants include, but are not limited to, amfepramone (diethylpropion), phentermine, mazindol and phenylpropanolamine fenfluramine, dexfenfluramine, and fluoxetine.
**Binding:** A specific interaction between two molecules, such that the two molecules interact. Binding can be specific and selective, so that one molecule is bound preferentially when compared to another molecule. In one embodiment, specific binding is identified by a disassociation constant (K_{d}).
**Body Mass Index (BMI):** A mathematical formula for measuring body mass, also sometimes called Quetelet's Index. BMI is calculated by dividing weight (in kg) by height² (in meters²). The current standards for both men and women accepted as "normal" are a BMI of 20-24.9 kg/m². In one embodiment, a BMI of greater than 25 kg/m² can be used to identify an obese subject. Grade I obesity corresponds to a BMI of 25-29.9 kg/m². Grade II obesity corresponds to a BMI of 30-40 kg/m²; and Grade III obesity corresponds to a BMI greater than 40 kg/m² (Jequier, Am. JClin. Nutr. 45:1035-47, 1987). Ideal body weight will vary among species and individuals based on height, body build, bone structure, and sex.
c-fos: The cellular homologue of the viral v-fos oncogene found in FBJ (Finkel-Biskis-Jinkins) and FBR murine osteosarcoma viruses (MSV). The human fos gene maps to chromosome 14q21-q31. Human fos has been identified as TIS-28.

C-fos is thought to have an important role in signal transduction, cell proliferation, and differentiation. It is a nuclear protein which, in combination with other transcription factors (for example, jun) acts as a trans-activating regulator of gene expression. C-fos is an immediate early response gene, which are believed to play a key role in the early response of cells to growth factors. C-fos is involved also in the control of cell growth and differentiation of embryonic hematopoietic cells and neuronal cells. The human c-fos coding amino acid and nucleic sequences are known (e.g., see Verma et al., Cold Spring Harb. Symp. Quant. Biol. 51, 949, 1986; GenBank Accession Nos. K00650 and M16287, and is available on the internet).
**Caloric intake or calorie intake:** The number of calories (energy) consumed by an individual.
**Calorie:** A unit of measurement in food. A standard calorie is defined as 4.184 absolute joules, or the amount of energy it takes to raise the temperature of one gram of water from 15 to 16° C (or1/100th the amount of energy needed to raise the temperature of one gram of water at one atmosphere pressure from 0° C to 100° C), food calories are actually equal to 1,000 standard calories (1 food calorie = 1 kilocalorie).
**Conservative variation:** The replacement of an amino acid residue by another, biologically similar residue. Examples of conservative variations include the substitution of one hydrophobic residue such as isoleucine, valine, leucine or methionine for another, or the substitution of one polar residue for another, such as the substitution of arginine for lysine, glutamic for aspartic acid, or glutamine for asparagine, and the like. The term "conservative variation" also includes the use of a substituted amino acid in place of an unsubstituted parent amino acid provided that antibodies raised to the substituted polypeptide also immunoreact with the unsubstituted polypeptide.

Non-limiting examples of conservative amino acid substitutions include those listed below:

| Original Residue | Conservative Substitutions |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

**Depolarization:** An increase in the membrane potential of a cell. Certain stimuli reduce the charge across the plasma membrane. These can be electrical stimuli (which open voltage-gated channels), mechanical stimuli (which activate mechanically-gated channels) or certain neurotransmitters (which open ligand-gated channels). In each case, the facilitated diffusion of sodium into the cell increases the resting potential at that spot on the cell creating an excitatory postsynaptic potential (EPSP). Depolarizations can also be generated by decreasing the frequency of inhibitory postsynaptic currents (IPSCs), these are due to inhibitory neurotransmitters facilitating the influx of chloride ions into the cell, creating an IPSC. If the potential is increased to the threshold voltage (about -50 mV in mammalian neurons), an action potential is generated in the cell.
**Diabetes:** A failure of cells to transport endogenous glucose across their membranes either because of an endogenous deficiency of insulin and/or a defect in insulin sensitivity. Diabetes is a chronic syndrome of impaired carbohydrate, protein, and fat metabolism owing to insufficient secretion of insulin or to target tissue insulin resistance. It occurs in two major forms: insulin-dependent diabetes mellitus (IDDM, type I) and non-insulin dependent diabetes mellitus (NIDDM, type II) which differ in etiology, pathology, genetics, age of onset, and treatment.

The two major forms of diabetes are both characterized by an inability to deliver insulin in an amount and with the precise timing that is needed for control of glucose homeostasis. Diabetes type I, or insulin dependent diabetes mellitus (IDDM) is caused by the destruction of β cells, which results in insufficient levels of endogenous insulin. Diabetes type II, or non-insulin dependent diabetes, results from a defect in both the body's sensitivity to insulin, and a relative deficiency in insulin production.
**Exendin:** A 39-amino acid peptide isolated from the salivary glands of the Gila monster (*Heloderma suspectum*) (Eng J et al J Biol Chem 267:7402-7405, 1992), see SEQ ID NO:341. Exendin is an example of an agonist at the GLP-1 receptor. Molecules derived from exendin-4 and that also have GLP-1 agonist activity are further examples of GLP-1 agonists.
**Food intake:** The amount of food consumed by an individual. Food intake can be measured by volume or by weight. In one embodiment, food intake is the total amount of food consumed by an individual. In another embodiment, food intake is the amount of proteins, fat, carbohydrates, cholesterol, vitamins, minerals, or any other food component, of the individual. "Protein intake" refers to the amount of protein consumed by an individual. Similarly, "fat intake," "carbohydrate intake," "cholesterol intake," "vitamin intake," and "mineral intake" refer to the amount of proteins, fat, carbohydrates, cholesterol, vitamins, or minerals consumed by an individual.
**Glucagon-like peptide-1:** Peptides produced by processing of preproglucogon, which is a 160 amino acid polypeptide, in the central nervous system (CNS) and the intestine. GLP-1 (1-37) is the initial product. GLP-1 (1-37) is amidated by post-translational processing to yield GLP-1 (1-36) NH, or is enzymatically processed to give GLP-1 (7-37) (SEQ ID NO: 338). GLP-1 (7-37) can be amidated to give GLP-1 (7-36) amide (SEQ ID NO: 339), which is the most biologically active form. GLP-1 is released into the circulation in response to nutrient intake. Intestinal cells secrete GLP-1 (7-37) and GLP-1 (7-36) amide in a ratio of 1 to 5.

GLP-1 receptors are found in the brainstem, arcuate nucleus and paraventricular nucleus. Physiological actions of GLP-1 in man include stimulation of insulin release, suppression of gastric acid secretion and slowing of gastric emptying.

A GLP-1 agonist is a peptide, small molecule, or chemical compound that preferentially binds to the GLP-1 receptor and stimulate sthe same biological activity as GLP-1. In one embodiment, an agonist for the GLP-1 receptor binds to the receptor with an equal or greater affinity than a GLP-1 peptide. In another embodiment, an agonist selectively binds the GLP-1 receptor, as compared to binding to another receptor.

One of skill in the art can readily determine the dissociation constant (K_{d}) value of a given compound. This value is dependent on the selectivity of the compound tested. For example, a compound with a K_{d} which is less than 10 nM is generally considered an excellent drug candidate. However, a compound that has a lower affinity, but is selective for the particular receptor, can also be a good drug candidate. In one specific, non-limiting example, an assay, such as a competition assay, is used to determine if a compound of interest is a GLP-1 receptor agonist.

GLP-1 agonists include GLP-1 related peptides and peptides that result from natural or synthetic enzymatic or chemical processing of a GLP-1 peptide or a related peptide.
**Hyperpolarization:** A decrease in the membrane potential of a cell. Inhibitory neurotransmitters inhibit the transmission of nerve impulses via hyperpolarization. This hyperpolarization is called an inhibitory postsynaptic potential (IPSP). Although the threshold voltage of the cell is unchanged, a hyperpolarized cell requires a stronger excitatory stimulus to reach threshold.
**Inhibitory Postsynaptic Current:** A current that inhibits an electrophysiological parameter of a postsynaptic cell. The potential of a postsynaptic cell can be analyzed to determine an effect on a presynaptic cell. In one embodiment, the postsynaptic cell is held in voltage clamp mode, and postsynaptic currents are recorded. If necessary, antagonists of other classes of current can be added. In one specific, non-limiting example, to record GABAergic IPSCs, blockers of excitatory channels or receptors can be added. The instantaneous frequency over time is then determined.

In one embodiment, IPSCs give a measure of the frequency of GABA release from an NPY neuron. Thus, as NPY neurons release GABA onto POMC neurons, measurement of IPSC frequency is a gauge of the inhibitory tone that POMC neurons are receiving, and can be used to assess the effect of an agonist of PYY.
**Membrane potential:** The electrical potential of the interior of the cell with respect to the environment, such as an external bath solution. One of skill in the art can readily assess the membrane potential of a cell, such as by using conventional whole cell techniques. Activation of a cell is associated with less negative membrane potentials (for example shifts from about -50 mV to about -40 mV). These changes in potential increase the likelihood of action potentials, and thus lead to an increase in the rate of action potentials.

The rate of action potentials can be assessed using many approaches, such as using conventional whole cell access, or using, for example, perforated-patch whole-cell and cell-attached configurations. In each event the absolute voltage or current is not assessed, rather the frequency of rapid deflections characteristic of action potentials is assessed, as a function of time (therefore this frequency is an instantaneous frequency, reported in "bins"). This time component can be related to the time at which a compound, such as a PYY agonist, is applied to the bath to analyze the effect of the compound, such as the PYY agonist, on action potential firing rate.
**Neuropeptide Y (NPY):** A 36-amino acid peptide that is a neuropeptide identified in the mammalian brain. NPY is believed to be an important regulator in both the central and peripheral nervous systems and influences a diverse range of physiological parameters, including effects on psychomotor activity, food intake, central endocrine secretion, and vasoactivity in the cardiovascular system. High concentrations of NPY are found in the sympathetic nerves supplying the coronary, cerebral, and renal vasculature and have contributed to vasoconstriction. NPY binding sites have been identified in a variety of tissues, including spleen, intestinal membranes, brain, aortic smooth muscle, kidney, testis, and placenta. In addition, binding sites have been reported in a number of rat and human cell lines.

Neuropeptide Y (NPY) receptor has structure/activity relationships within the pancreatic polypeptide family. This family includes NPY, which is synthesized primarily in neurons; peptide YY (PYY), which is synthesized primarily by endocrine cells in the gut; and pancreatic polypeptide (PP), which is synthesized primarily by endocrine cells in the pancreas. These 36 amino acid peptides have a compact helical structure involving an amino acid structure, termed a "PP-fold" in the middle of the peptide.

NPY binds to several receptors, including the Y1, Y2, Y3, Y4 (PP), Y5, Y6, and Y7 receptors. These receptors are recognized based on binding affinities, pharmacology, and sequence (if known). Most, if not all of these receptors are G protein coupled receptors. The Y1 receptor is generally considered to be postsynaptic and mediates many of the known actions of neuropeptide Y in the periphery. Originally, this receptor was described as having poor affinity for C-terminal fragments of neuropeptide Y, such as the 13-36 fragment, but interacts with the full length neuropeptide Y and peptide YY with equal affinity (e.g., see PCT publication WO 93/09227).

Pharmacologically, the Y2 receptor is distinguished from Y1 by exhibiting affinity for C-terminal fragments of neuropeptide Y. The Y2 receptor is most often differentiated by the affinity of neuropeptide Y(13-36), although the 3-36 fragment of neuropeptide Y and peptide YY provides improved affinity and selectivity (see Dumont et al., *Society for Neuroscience Abstracts* 19:726, 1993). Signal transmission through both the Y1 and the Y2 receptors are coupled to the inhibition of adenylate cyclase. Binding to the Y-2 receptor was also found to reduce the intracellular levels of calcium in the synapse by selective inhibition of N-type calcium channels. In addition, the Y-2 receptor, like the Y1 receptors, exhibits differential coupling to second messengers (see U.S. Patent No. 6,355,478). Y2 receptors are found in a variety of brain regions, including the hippocampus, substantia nigra-lateralis, thalamus, hypothalamus, and brainstem. The human, murine, monkey and rat Y2 receptors have been cloned (e.g., see U.S. Patent No. 6,420,352 and U.S. Patent No. 6,355,478).

A Y2 receptor agonist is a peptide, small molecule, or chemical compound that preferentially binds to the Y2 receptor and stimulates intracellular signaling. In one embodiment, an agonist for the Y2 receptor binds to the receptor with an equal or greater affinity than NPY. In another embodiment, an agonist selectively binds the Y2 receptor, as compared to binding to another receptor.

One of skill in the art can readily determine the dissociation constant (K_{d}) value of a given compound. This value is dependent on the selectivity of the compound tested. For example, a compound with a K_{d} which is less than 10 nM is generally considered an excellent drug candidate. However, a compound that has a lower affinity, but is selective for the particular receptor, can also be a good drug candidate. In one specific, non-limiting example, an assay, such as a competition assay, is used to determine if a compound of interest is a Y2 receptor agonist. Assays useful for evaluating neuropeptide Y receptor antagonists are also well known in the art (see U.S. Patent No. 5,284,839, and Walker et al., Journal of Neurosciences 8:2438-2446, 1988).
**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual.
**Obesity:** A condition in which excess body fat may put a person at health risk (see Barlow and Dietz, Pediatrics 102:E29, 1998; National Institutes of Health, National Heart, Lung, and Blood Institute (NHLBI), Obes. Res. 6 (suppl. 2):51S-209S, 1998). Excess body fat is a result of an imbalance of energy intake and energy expenditure. In one embodiment, the Body Mass Index (BMI) is used to assess obesity. In one embodiment, a BMI of 25.0 kg/m² to 29.9 kg/m² is overweight, while a BMI of 30 kg/m² is obese.
In another embodiment, waist circumference is used to assess obesity. In this embodiment, in men a waist circumference of 102 cm or more is considered obese, while in women a waist circumference of 89 cm or more is considered obese. Strong evidence shows that obesity affects both the morbidity and mortality of individuals. For example, an obese individual is at increased risk for heart disease, non-insulin dependent (type 2) diabetes, hypertension, stroke, cancer (e.g. endometrial, breast, prostate, and colon cancer), dyslipidemia, gall bladder disease, sleep apnea, reduced fertility, and osteoarthritis, amongst others (see Lyznicki et al., Am. Fam. Phys. 63:2185, 2001).
**Overweight:** An individual who weighs more than their ideal body weight. An overweight individual can be obese, but is not necessarily obese. In one embodiment, an overweight individual is any individual who desires to decrease their weight. In another embodiment, an overweight individual is an individual with a BMI of 25.0 kg/m² to 29.9 kg/m²
**Oxyntomodulin:** A further peptide produced by post-translational processing of preproglucagon in the CNS and intestine, see SEQ ID NO: 340.. Agonists of oxyntomodulin may be identified as described above for agonists of GLP-1 and of NPY. include oxyntomodulins of other species and also modified sequences, for example, sequences in which The term OXM used in this text also covers any analogue of the above OXM sequence, wherein the histidine residue at position 1 is maintained or replaced by an aromatic moiety carrying a positive charge or a derivative thereof, preferably wherein the moiety is an amino acid, more preferably wherein it is a histidine derivative, while 1, 2, 3, 4, 5, 6, 7, 8, 9, 10, 11, 12, 13, 14, 15, 16, 17, 18, 19, 20, 21 or 22 of the other amino acids in the above OXM sequence can be independently replaced by any other independently chosen amino acid, with the exception of histidine in position 1.

Any one or more (to 22) other alpha-amino acid residue in the sequence can be independently replaced by any other one alpha-amino acid residue. Preferably, any amino acid residue other than histidine is replaced with a conservative replacement as well known in the art i.e. replacing an amino acid with one of a similar chemical type such as replacing one hydrophobic amino acid with another.

As discussed above, 1 to 22 of the amino acids can be replaced. In addition to the replacement option above, this may be by a non-essential or modified or isomeric form of an amino acid. For example, 1 to 22 amino acids can be replaced by an isomeric form (for example a D-amino acid), or a modified amino acid, for example a nor-amino acid (such as norleucine or norvaline) or a non-essential amino acid (such as taurine). Furthermore, 1 to 22 amino acids may be replaced by a corresponding or different amino acid linked via its side chain (for example gamma-linked glutamic acid). For each of the replacements discussed above, the histidine residue at position 1 is unaltered or defined above.

In addition, 1, 2, 3, 4 or 5 of the amino acid residues can be removed from the OXM sequence with the exception of histidine at the 1 position (or as defined above). The deleted residues may be any 2, 3, 4 or 5 contiguous residues or entirely separate residues.

The C-terminus of the OXM sequence may be modified to add further amino acid residues or other moieties.
**Pancreatic Polypeptide:** A 36 amino acid peptide produced by the pancreas that is has homology to PYY and NPY.
**Peripheral Administration:** Administration outside of the central nervous system. Peripheral administration does not include direct administration to the brain. Peripheral administration includes, but is not limited to intravascular, intramuscular, subcutaneous, inhalation, oral, rectal, transdermal or intra-nasal administration
**Polypeptide:** A polymer in which the monomers are amino acid residues which are joined together through amide bonds. When the amino acids are alpha-amino acids, either the L-optical isomer or the D-optical isomer can be used, the L-isomers being preferred. The terms "polypeptide" or "protein" as used herein are intended to encompass any amino acid sequence and include modified sequences such as glycoproteins. The term "polypeptide" is specifically intended to cover naturally occurring proteins, as well as those which are recombinantly or synthetically produced. The term "polypeptide fragment" refers to a portion of a polypeptide, for example such a fragment which exhibits at least one useful sequence in binding a receptor. The term "functional fragments of a polypeptide" refers to all fragments of a polypeptide that retain an activity of the polypeptide. Biologically functional peptides can also include fusion proteins, in which the peptide of interest has been fused to another peptide that does not decrease its desired activity.
**PYY:** A peptide YY polypeptide obtained or derived from any species. Thus, PYY includes the human full length polypeptide (as set forth in SEQ ID NO: 1) and species variations of PYY, including e.g. murine, hamster, chicken, bovine, rat, and dog PYY (SEQ ID NOS: 5-12). In one embodiment, PYY agonists do not include NPY. PYY also includes PYY₃₋₃₆. A "PYY agonist" is any compound which binds to a receptor that specifically binds PYY, and elicits an effect of PYY. In one embodiment, a PYY agonist is a compound that affects food intake, caloric intake, or appetite, and/or which binds specifically in a Y receptor assay or competes for binding with PYY, such as in a competitive binding assay with labeled PYY. PYY agonists include, but are not limited to, compounds that bind to the Y2 receptor.
**Substantially purified:** A polypeptide which is substantially free of other proteins, lipids, carbohydrates or other materials with which it is naturally associated. For example, the polypeptide may be at least 50%, 80% or 90% free of other proteins, lipids, carbohydrates or other materials with which it is naturally associated.
**Therapeutically effective amount:** A dose sufficient to prevent advancement, or to cause regression of a disorder, or which is capable of relieving a sign or symptom of a disorder, or which is capable of achieving a desired result. In several embodiments, a therapeutically effect of PYY or an agonist thereof is an amount sufficient to inhibit or halt weight gain, or an amount sufficient to decrease appetite, or an amount sufficient to reduce caloric intake or food intake or increase energy expenditure.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes."

### Altering Food Intake, Appetite, Caloric Intake and Energy Expenditure

Peripheral administration of PYY, or an agonist of PYY, and GLP-1 or an agonist thereof, may result in a decrease in the amount, either the total weight or the total volume of food. Or, administration of PYY, or an agonist thereof, and GLP-1 or an agonist thereof, may result in a decrease of the intake of a food component, such as a decrease in the ingestion of lipids, carbohydrates, cholesterol, or proteins. A preferred compound, PYY₃₋₃₆ can be administered. Or, total caloric intake is reduced by peripheral administration of a therapeutically effective amount of PYY and GLP-1 or an agonist thereof. Or, the caloric intake from the ingestion of a specific food component, such as the ingestion of lipids, carbohydrates, cholesterol, or proteins, is reduced.

The invention finds utility in a method for reducing appetite by administering a therapeutically effective amount of PYY or an agonist thereof and GLP-1 or an agonist thereof. Appetite can be measured by any means known to one of skill in the art. For example, decreased appetite can be assessed by a psychological assessment. Administration of PYY and the other agent(s) results in a change in perceived hunger, satiety, and/or fullness. Hunger can be assessed by any means known to one of skill in the art, for example using psychological assays, such as by an assessment of hunger feelings and sensory perception using a questionnaire, such as a Visual Analog Score (VAS) questionnaire (see the Examples section). In one specific example, hunger is assessed by answering questions relating to desire for food, drink, prospective food consumption, nausea, and perceptions relating to smell or taste.

Peripherally administering a therapeutically effective amount of PYY or an agonist thereof and GLP-1 or an agonist thereof, to the subject, may alter energy expenditure. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat. Manipulations of the arcuate circuitry described in this application, that alter food intake coordinately and reciprocally alter energy expenditure, may be involved. Energy expenditure is a result of cellular metabolism, protein synthesis, metabolic rate, and calorie utilization. Thus, peripheral administration of PYY and administration of GLP-1 or an agonist thereof, may result in increased energy expenditure, and decreased efficiency of calorie utilization.

Obesity is currently a poorly treatable, chronic, essentially intractable metabolic disorder. A therapeutic drug useful in weight reduction of obese persons could have a profound beneficial effect on their health. Disorders wherein obesity is a risk factor include cardiovascular disease, (including, but not limited to, hypertension, atherosclerosis, congestive heart failure, and dyslipidemia), stroke, gallbladder disease, osteoarthritis, sleep apnea, reproductive disorders such as, but not limited to, polycystic ovarian syndrome, cancers (e.g., breast, prostate, colon, endometrial, kidney, and esophagus cancer), varicose veins, acnthosis nigricans, eczema, exercise intolerance, insulin resistance, hypertension hypercholesterolemia, cholithiasis, osteoarthritis, orthopedic injury, insulin resistance (such as, but not limited to, type 2 diabetes and syndrome X) and tromboembolic disease (see Kopelman, Nature 404:635-43; Rissanen et al., British Med. J. 301, 835, 1990).

Other associated disorders also include depression, anxiety, panic attacks, migraine headaches, PMS, chronic pain states, fibromyalgia, insomnia, impulsivity, obsessive compulsive disorder, and myoclonus. Obesity is a recognized risk factor for increased incidence of complications of general anesthesia. (See e. g., Kopelman, Nature 404:635-43, 2000). It reduces life span and carries a serious risk of co-morbidities listed above.

Other diseases or disorders associated with obesity are birth defects (maternal obesity associated with increased incidence of neural tube defects), carpal tunnel syndrome (CTS), chronic venous insufficiency (CVI), daytime sleepiness, deep vein thrombosis (DVT), end stage renal disease (ESRD), gout, heat disorders, impaired immune response, impaired respiratory function, infertility, liver disease, lower back pain, obstetric and gynecologic complications, pancreatititis, as well as abdominal hernias, acanthosis nigricans, endocrine abnormalities, chronic hypoxia and hypercapnia, dermatological effects, elephantitis, gastroesophageal reflux, heel spurs, lower extremity edema, mammegaly (causing considerable problems such as bra strap pain, skin damage, cervical pain, chronic odors and infections in the skin folds under the breasts, etc.), large anterior abdominal wall masses (abdominal panniculitis with frequent panniculitis, impeding walking, causing frequent infections, odors, clothing difficulties, low back pain), musculoskeletal disease, pseudo tumor cerebri (or benign intracranial hypertension), and sliding hiatil hernia.

Disorders associated with increased caloric intake, insulin resistance, or glucose intolerance include obesity, any form of diabetes mellitus (e.g., type 1, 2 or gestational diabetes), eating disorders, insulin-resistance syndromes, and Alzheimer's disease.

A suitable administration format may be best determined by the subject or by a medical practitioner. Pharmaceutical compositions that include PYY, or an agonist thereof, will preferably be formulated in unit dosage form, suitable for individual administration of precise dosages. An effective amount of PYY or an agonist thereof can be administered in a single dose, or in multiple doses, for example daily, during a course of treatment. PYY may be administered whenever appetite suppression, decreased food intake, or decreased caloric intake is desired. Alternatively, PYY or an analog thereof may be administered slightly prior to whenever this effect is desired, such as, but not limited to about 10 minutes, about 15 minutes, about 30 minutes, about 60 minutes, about 90 minutes, or about 120 minutes, prior to the time the effect is desired. Or, a time release formulation may be utilized.

A therapeutically effective amount of PYY or an agonist thereof may be administered as a single pulse dose, as a bolus dose, or as pulse doses administered over time. Thus, in pulse doses, a bolus administration of PYY is provided, followed by a time period wherein no PYY is administered to the subject, followed by a second bolus administration. For example, pulse doses of PYY may be administered during the course of a day, during the course of a week, or during the course of a month.

The therapeutically effective amount of PYY or an agonist thereof is a molar equivalent amount of a PYY₃₋₃₆ dose of no more than 2.2nmol per kg body weight of the subject. It will be dependent on the molecule utilized, the subject being treated, the severity and type of the affliction, and the manner of administration. For example, a therapeutically effective amount of PYY or an agonist thereof can vary from about 0.01 µg per kilogram (kg) body weight to about 1 g per kg body weight, such as about 1 µg to about 5 mg per kg body weight, or about 5µg to about 1 mg per kg body weight. In another embodiment, PYY or an agonist thereof is administered to a subject at 0.5 to 135 picomole (pmol) per kg body weight, or about 72 pmol per kg body weight. In one specific, non-limiting example about 5 to about 50 nmol is administered as a subcutaneous injection, such as about 2 to about 20 nmol, or about 10 nmol is administered as a subcutaneous injection. The exact dose is readily determined by one of skill in the art based on the potency of the specific compound (such as the PYY polypeptide, or agonist) utilized, the age, weight, sex and physiological condition of the subject. The dose of an agonist can be a molar equivalent of the therapeutically effective dose of PYY or PYY ₃₋₃₆. In all cases, the above doses are subject to a maximum dosage of a molar equivalent amount of a PYY₃₋₃₆ dose of 2.2nmol per kg body weight.

The compositions or pharmaceutical compositions can be administered by any route, including intravenous, intraperitoneal, subcutaneous, sublingual, transdermal, intramuscular, oral, topical, transmucosal, or by pulmonary inhalation. Compositions useful in the disclosure may conveniently be provided in the form of formulations suitable for parenteral (including intravenous, intramuscular and subcutaneous), nasal or oral administration. The term "parenteral" as used herein refers to modes of administration which include intravenous, intramuscular, intraperitoneal, intrasternal, subcutaneous and intraarticular injection and infusion. PYY, including PYY₃₋₃₆, an agonist of PYY, can be administered subcutaneously. It is well known in the art that subcutaneous injections can be easily self-administered.

A suitable administration format may best be determined by a medical practitioner for each patient individually. Various pharmaceutically acceptable carriers and their formulation are described in standard formulation treatises, e.g., Remington's Pharmaceutical Sciences by E. W. Martin. See also Wang, Y. J. and Hanson, M. A., Journal of Parenteral Science and Technology, Technical Report No. 10, Supp. 42:2S, 1988.

PYY and PYY agonists useful in the methods of this disclosure can be provided as parenteral compositions, e.g., for injection or infusion. Preferably, they are suspended in an aqueous carrier, for example, in an isotonic buffer solution at a pH of about 3.0 to about 8.0, preferably at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers. A form of repository or "depot" slow release preparation may be used so that therapeutically effective amounts of the preparation are delivered into the bloodstream over many hours or days following transdermal injection or delivery.

Since the PYY and agonists are amphoteric, they may be utilized as free bases, as acid addition salts or as metal salts. The salts must, of course, be pharmaceutically acceptable, and these will include metal salts, particularly alkali and alkaline earth metal salts, e.g., potassium or sodium salts. A wide variety of pharmaceutically acceptable acid addition salts are available. Such products are readily prepared by procedures well known to those skilled in the art.

For parenteral administration, in one embodiment, PYY and PYY agonists can be formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. "Pharmaceutically acceptable carrier" refers to a non-toxic solid, semisolid or liquid filler, diluent, encapsulating material or formulation auxiliary of any type. For example, the formulation preferably does not include oxidizing agents and other compounds that are known to be deleterious to PYY and PYY agonists.

Generally, the formulations are prepared by contacting the PYY or PYY agonist uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

PPY and PYY agonists are also suitably administered by sustained-release systems. Suitable examples of sustained-release PYY and PYY agonists include suitable polymeric materials (such as, for example, semi-permeable polymer matrices in the form of shaped articles, e.g., films, or mirocapsules), suitable hydrophobic materials (for example as an emulsion in an acceptable oil) or ion exchange resins, and sparingly soluble derivatives (such as, for example, a sparingly soluble salt). Sustained-release PPY and PYY agonist compositions may be administered orally, rectally, parenterally, intracistemally, intravaginally, intraperitoneally, topically (as by powders, ointments, gels, drops or transdermal patch), bucally, or as an oral or nasal spray.

Sustained release matrices include polylactides (U.S. Patent No. 3,773,919, EP 58,481), copolymers of L-glutamic acid and gamma-ethyl-L-glutamate (Sidman et al., Biopolymers 22:547-556, 1983, poly(2-hydroxyethyl methacrylate)); (Langer et al., J. Biomed. Mater. Res. 15:167-277, 1981; Langer, Chem. Tech. 12:98-105, 1982, ethylene vinyl acetate (Langer et al., *Id*.) or poly-D-(-)-3-hydroxybutyric acid (EP 133,988).

Sustained-release PPY and PYY agonists include liposomally PPY and PYY agonists (see generally, Langer, Science 249:1527-1533, 1990; Treat et al., in Liposomes in the Therapy of Infectious Disease and Cancer, Lopez-Berestein and Fidler (eds.), Liss, New York, pp. 317-327 and 353-365, 1989). Liposomes containing PPY peptide and peptide analogs are prepared by methods known per se: DE 3,218,121; Epstein et al., Proc. Natl. Acad. Sci. U.S.A. 82:3688-3692, 1985; Hwang et al., Proc. Natl. Acad. Sci. U.S.A. 77:4030-4034, 1980; EP 52,322; EP 36,676; EP 88,046; EP 143,949; EP 142,641; Japanese Patent Application No. 83-118008; U.S. Patent No. 4,485,045, U.S. Patent No. 4,544,545; and EP 102,324. Ordinarily, the liposomes are of the small (about 200-800 Angstroms) unilamellar type in which the lipid content is greater than about 30 mole percent cholesterol, the selected proportion being adjusted for the optimal performance.

Preparations for administration can be suitably formulated to give controlled release of PYY and PYY agonists. For example, the pharmaceutical compositions may be in the form of particles comprising a biodegradable polymer and/or a polysaccharide jellifying and/or bioadhesive polymer, an amphiphilic polymer, an agent modifying the interface properties of the particles and a pharmacologically active substance. These compositions exhibit certain biocompatibility features which allow a controlled release of the active substance. See U.S. Patent No. 5,700,486.

In yet an additional embodiment, PPY and PYY agonists are delivered by way of a pump (see Langer, *supra*; Sefton, CRC Crit. Ref. Biomed. Eng. 14:201, 1987; Buchwald et al., Surgery 88:507, 1980; Saudek et al., N. Engl. J. Med. 321:574, 1989) or by continuous subcutaneous infusions, for example, using a mini-pump. An intravenous bag solution may also be employed. The key factor in selecting an appropriate dose is the result obtained, as measured by decreases in total body weight or ratio of fat to lean mass, or by other criteria for measuring control or prevention of obesity or prevention of obesity-related conditions, as are deemed appropriate by the practitioner. Other controlled release systems are discussed in the review by Langer (Science 249:1527-1533, 1990).

In another aspect of the disclosure, PPY and PYY agonists are delivered by way of an implanted pump, described, for example, in U.S. Patent No. 6,436,091; U.S. Patent No. 5,939,380; U.S. Patent No. 5,993,414.

Implantable drug infusion devices are used to provide patients with a constant and long term dosage or infusion of a drug or any other therapeutic agent. Essentially such device may be categorized as either active or passive.

Active drug or programmable infusion devices feature a pump or a metering system to deliver the drug into the patient's system. An example of such an active drug infusion device currently available is the Medtronic SynchroMed™ programmable pump. Such pumps typically include a drug reservoir, a peristaltic pump to pump out the drug from the reservoir, and a catheter port to transport the pumped out drug from the reservoir via the pump to a patient's anatomy. Such devices also typically include a battery to power the pump as well as an electronic module to control the flow rate of the pump. The Medtronic SynchroMed™ pump further includes an antenna to permit the remote programming of the pump. Passive drug infusion devices, in contrast, do not feature a pump, but rather rely upon a pressurized drug reservoir to deliver the drug. Thus such devices tend to be both smaller as well as cheaper as compared to active devices. An example of such a device includes the Medtronic IsoMed™. This device delivers the drug into the patient through the force provided by a pressurized reservoir applied across a flow control unit.

The implanted pump can be completely implanted under the skin of a patient, thereby negating the need for a percutaneous catheter. These implanted pumps can provide the patient with PYY or a PYY agonist at a constant or a programmed delivery rate, e.g., to give pulsed doses at or around meal time. Constant rate or programmable rate pumps are based on either phase-change or peristaltic technology. When a constant, unchanging delivery rate is required, a constant-rate pump is well suited for long-term implanted drug delivery. If changes to the infusion rate are expected, a programmable pump may be used in place of the constant rate pump system. Osmotic pumps may be much smaller than other constant rate or programmable pumps, because their infusion rate can be very low. An example of such a pump is described listed in U.S. Patent No. 5,728,396.

For oral administration, the pharmaceutical compositions can take the form of, for example, tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets can be coated by methods well known in the art. Liquid preparations for oral administration can take the form of, for example, solutions, syrups or suspensions, or they can be presented as a dry product for constitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations can also contain buffer salts, flavoring, coloring and sweetening agents as appropriate.

For administration by inhalation, the compounds for use according to the present disclosure are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of e.g., gelatin for use in an inhaler or insufflator can be formulated containing a powder mix of the compound and a suitable powder base such as lactose or starch.

The compounds can also be formulated in rectal compositions such as suppositories or retention enemas, e.g., containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

Pharmaceutical compositions that comprise a PYY, or an agonist thereof, as described herein as an active ingredient will normally be formulated with an appropriate solid or liquid carrier, depending upon the particular mode of administration chosen. The pharmaceutically acceptable carriers and excipients useful in this disclosure are conventional. For instance, parenteral formulations usually comprise injectable fluids that are pharmaceutically and physiologically acceptable fluid vehicles such as water, physiological saline, other balanced salt solutions, aqueous dextrose, glycerol or the like. Excipients that can be included are, for instance, other proteins, such as human serum albumin or plasma preparations. If desired, the pharmaceutical composition to be administered may also contain minor amounts of non-toxic auxiliary substances, such as wetting or emulsifying agents, preservatives, and pH buffering agents and the like, for example sodium acetate or sorbitan monolaurate. Other medicinal and pharmaceutical agents, for instance other appetite suppressants, or protease inhibitors, also may be included. Actual methods of preparing such dosage forms are known, or will be apparent, to those skilled in the art.

The dosage form of the pharmaceutical composition will be determined by the mode of administration chosen. For instance, in addition to injectable fluids, inhalation, suppository, and oral formulations can be employed. The pharmaceutical compositions can be produced of conventional mixing, granulating, confectioning, dissolving or lyophilizing processes.

Oral formulations may be liquid (e.g., syrups, solutions or suspensions), or solid (e.g., powders, pills, tablets, or capsules). For example, pharmaceutical compositions for oral use can be obtained by combining the active ingredient with one or more solid carriers, optionally granulating a resulting mixture, and, if desired, processing the mixture or granules, if appropriate with the addition of additional excipients, to form tablets or dragee cores.

Suitable carriers include fillers, such as sugars, for example lactose, saccharose, mannitol or sorbitol, cellulose preparations and/or calcium phosphates, for example tricalcium phosphate or calcium hydrogen phosphate, also binders, such as starches, for example corn, wheat, rice or potato starch, methylcellulose, hydroxypropylmethylcellulose, sodium carboxymethylcellulose and/or polyvinylpyffolidone, and/or, if desired, disintegrators, such as the above-mentioned starches, also carboxymethyl starch, cross-linked polyvinylpyrrolidone, alginic acid or a salt thereof, such as sodium alginate. Additional excipients include flow conditioners and lubricants, for example silicic acid, talc, stearic acid or salts thereof, such as magnesium or calcium stearate, and/or polyethylene glycol, or derivatives thereof.

For parenteral administration compositions include suitable aqueous solutions of an active ingredient in water-soluble form, for example in the form of a water-soluble salt, or aqueous injection suspensions that contain viscosity-altering substances, for example sodium carboxymethylcellulose, sorbitol and/or dextran, and, if desired, stabilizers. The active ingredient, optionally together with excipients, can also be in the form of a lyophilisate and can be made into a solution prior to parenteral administration by the addition of suitable solvents. Solutions such as those that are used, for example, for parenteral administration can also be used as infusion solutions.

For inhalation, PYY or an agonist thereof is administered as an aerosol or a dispersion in a carrier. In one specific, non-limiting example, PYY or an agonist thereof is administered as an aerosol from a conventional valve, such as, but not limited to, a metered dose valve, through an aerosol adapter also known as an actuator. A suitable fluid carrier can be also included in the formulation, such as, but not limited to, air, a hydrocarbon, such as n-butane, propane, isopentane, amongst others, or a propellant, such as, but not limited to a fluorocarbon. Optionally, a stabilizer is also included, and/or porous particles for deep lung delivery are included (e.g., see U.S. Patent No. 6,447,743).

Compounds with poor solubility in aqueous systems require formulation by using solubilizing agents such as ionic surfactants, cholates, polyethylene glycol (PEG), ethanol, or other agents which may have undesirable effects when used for inhalation. In addition, a treatment requiring successful delivery into alveoli of the lower pulmonary region may preclude from the formulation the use of certain irritants such as chlorofluorocarbons and should involve a minimum number of required doses. Alternatively, to avoid such limitations, liposomes or hydrophobic particles can be used. In one embodiment, an inhalation formulation for a sustained release includes using aerosol droplet particles approximately 1-2.1 µm in size, or of less than 1 µm in size. Small particle aerosol liposomes and liposome-drug combinations for medical use have been previously described (e.g., see EP 87309854.5).

According to the invention, a therapeutically effective amount of PYY or an agonist thereof is administered with a therapeutically effective amount of GLP-1 or an agonist thereof.

The PYY or agonist thereof and the GLP-1 or an agonist thereof may be administered simultaneously or substantially simultaneously, or sequentially, in any order. The PYY or agonist thereof and the GLP-1 or an agonist thereof may be administered in a single pharmaceutical composition or in separate compositions, and they may be administered by the same route or my different routes.

If the PYY and the GLP-1 or an agonist thereof are to be administered in a single pharmaceutical composition, that composition may be any of those described above for PYY or an agonist thereof. The composition may enable simultaneous or substantially simultaneous administration of the PYY or agonist thereof and the GLP-1 or an agonist thereof. If desired, the PYY or agonist thereof and the GLP-1 or an agonist thereof may be compartmentalized in the composition, for example, in different layers of a tablet, or in different granules in a capsule. If desired, such compartmentalization may be designed to give different release properties to the two components to enable delivery of the PYY or agonist component and the GLP-1 or an agonist thereof at different times, for example, sequentially.

Alternatively, the PYY or agonist thereof and the GLP-1 or an agonist thereof may be formulated in separate pharmaceutical compositions, for example, any of the pharmaceutical compositions described above for PYY and agonists thereof. Such separate compositions may be administered simultaneously or substantially simultaneously, or they may be administered sequentially, in any order.

If administered separately, whether sequentially or simultaneously (or substantially simultaneously), the PYY or agonist thereof and the GLP-1 or an agonist thereof may be administered by the same route or by different routes. It is generally more convenient to administer all the active agents in a single composition. However, in some cases it may be necessary or appropriate to administer the active agents by different routes. For example, peptides are generally not stable on oral administration unless modified or formulated in a special way, so must generally be administered via a non-oral route. Some agonists, for example, GLP-1 agonists, are chemical compounds that are stable when administered orally.

When PYY or an agonist thereof and GLP-1 or an agonist thereof are used in the manufacture of a medicament for use in a treatment as described herein, the medicament may be a single pharmaceutical composition comprising both components, as described above, or may be a two-component medicament, one component being a pharmaceutical composition comprising PYY or an agonist thereof, the other component being a pharmaceutical composition comprising GLP-1 or an agonist thereof, see above. The medicament, whether a one component medicament or a two component medicament as described above, will generally be packaged with instructions relating to its use. Such instructions will refer to the timing, dose and route of administration of the component(s).

If desired, a further agent that influences food intake and/or appetite may also be administered. Such agents include naturally-occurring agents and agonists thereof, for example, amylin and amylin agonists, leptin and leptin agonosts, and oxyntomodulin.

If desired, one or more other agents, such as, but not limited to, an additional appetite suppressant, may also be administered. Specific, non-limiting example of an additional appetite suppressant include amfepramone (diethylpropion), phentermine, mazindol and phenylpropanolamine, fenfluramine, dexfenfluramine, and fluoxetine.

PYY or a PYY agonist, or GLP-1 or a GLP-1 agonist, or both, can be administered simultaneously with the additional agent(s), or the various substances may be administered sequentially, and in any order. Thus, in one embodiment, PYY or an agonist thereof and GLP-1 or an agonist thereof is formulated and administered with an additional agent, for example, oxyntomodulin or an agonist thereof, or an appetite suppressant as a single composition.

The present invention finds utility in a method of treating obesity. The method includes administering to an obese subject a therapeutically effective amount of PYY or a PYY agonist, and GLP-1 or an agonist thereof. The subject can be insulin resistant or glucose intolerant, or both. In addition to being obese, the subject can have diabetes mellitus.

The PYY or a PYY agonist and GLP-1 or GLP-1 agonist are administered peripherally, such as in a single or divided dose. Suitable single or divided doses include, but are not limited to, 1µg to about 5 mg or about 0.01 µg/kg to about 500 µg/kg of PYY or a PYY agonist per dose. The PYY agonist can have potency in at least one of food intake or gastric emptying greater than NPY. Suitable doses also include those that raise the concentration of PYY and/or the agonist thereof significantly above the basal concentration of PYY, such as, but not limited to, a dose that that mimic postparandial serum concentrations of PYY (or the agonist). Thus, PYY or an agonist thereof may be administered to achieve the level of to effect a reduction in calorie intake, food intake, or appetite equivalent to the reduction in calorie intake, food intake, or appetite, or to increase the energy expenditure, caused by the postprandial level of PYY₃₋₃₆. Specific, non-limiting examples of doses include, but are not limited doses that produce the effect demonstrated when the serum levels of PYY are from about 40 pM/litre to about 60 pM/litre, for example, from about 40 pM/litre to about 50 pM/litre, or from about 40 pM/litre to about 45 pM/litre, or to about 43 pM/litre.

For all methods disclosed herein, the dose of PYY or PYY₃₋₃₆ can be based on the physiological levels observed post-prandially. The normal circulating levels of PYY₃₋₃₆ are about 8 pmol/litre, typically rising to about 40 to 60 pmol/litre after a meal. Agonists of PYY can be used at analogous doses. A single dose may be administered per day, or divided doses can be used (see above).

As indicated above, PYY₃₋₃₆ has a prolonged and sustained action, continuing to act even after it has been cleared from the circulating blood, for example, for up to 12 and even up to 24 hours. Accordingly, PYY or an agonist thereof may be administered twice per day or even just once. If desired, more doses may be administered, for example, three or four per day. For example, it may be desired to administer PYY or an agonist thereof before a meal, for example, about half an hour before a meal. It may be desired to administer PYY or an agonist thereof before each meal. Alternately, it may be desired to administer as few doses as possible, in which case a regime or one or two doses per day may be used.

GLP-1 or an agonist may be administered according to the same regime as the PYY or agonist thereof, whether administered simultaneously or substantially simultaneously with the PYY or PYY agonist, or sequentially. Alternatively, the GLP-1 may be administered in a different regime, for example, with more doses of GLP-1 than of PYY, for example, the PYY or PYY agonist may be administered once or twice per day, and the GLP-1 or agonist thereof may be administered three or four times per day. For example, PYY or a PYY agonist may be administered in the morning and evening, for example, before breakfast and before the evening meal, and the GLP-1 agonist thereof may also be administered before the mid-day meal.

When administered peripherally, PYY, including PYY₃₋₃₆ has its effects at physiological levels. No side effects are observed when PYY ₃₋₃₆ is used at such levels. Without being bound by theory, PYY₃₋₃₆ does not affect Y2 receptors throughout the brain, which could cause side effects. It should be noted, without being limiting, that a further advantage of PYY₃₋₃₆ is that PYY₃₋₃₆ does not increase blood pressure. The effects of PYY₃₋₃₆ are as long lasting as 24 hours. Recipients claim a decrease in appetite over that period, and a reduction of food intake of about one third has been reported.

In one specific, non-limiting example, PYY₃₋₃₆ is administered in a dose of about 1 nmol or more, 2 nmol or more, or 5 nmol or more. In this example, the dose of PYY₃₋₃₆ is generally not more than 100 nmol, for example, the dose is 90 nmols or less, 80 nmols or less, 70 nmols or less, 60 nmols or less, 50 nmols or less, 40 nmols or less, 30 nmols or less, 20 nmols or less, 10 nmols. For example, a dosage range may comprise any combination of any of the specified lower dose limits with any of the specified upper dose limits. Thus, exemplar non-limiting dose ranges include a dose of PYY₃₋₃₆ may be within the range of form 1 to 100 n mols, from 1 to 90 mols, from 1 to 80 nmols. Exemplary, non-limiting dose ranges include, from 2 to 100 nmols, from 2 to 90 n mols, for example, from 2 to 80 nmols etc., from 5 nmols to 100 mols, from 5 nmols to 90 nmols, from 5 nmols to 80 nmols etc. By way of example, a dose of from about 5 to about 50 nmol may be administered such as, but not limited to, from about 2 to about 20 nmol, for example, about 10 nmol. In all cases, the above doses are subject to a maximum dosage of a molar equivalent amount of a PYY₃₋₃₆ dose of 2.2 nmol per kg body weight. The selected dose may be administered for example, by injection, for example, as a subcutaneous injection. A PYY agonist may be administered at the same dose.

In a further, non-limiting example, PYY₃₋₃₆ may be administered peripherally at a dose of 0.1 nmoles or more per kg body weight of the subject, for example, 0.2 nmoles. The maximum dosage is 2.2 nmoles per kg body weight.

For subcutaneous administration, a dose of PYY for example PYY₃₋₃₆ or a PYY agonist of 10 nmoles or more, for example, 20 nmoles or more, for example, 30 nmoles or more, for example, 40 nmoles or more may be used. It is generally preferable to use up to about 70 nmoles, for example, up to about 60 nnmoles. A dose of about 40nmoles may be used. In one embodiment, a dose of PYY or PYY₃₋₃₆ at 0.143 n moles (1/7^{th} of a mole) is administered per kilogram, to achieve a dose that is similar to the postprandial level of PYY.

If PYY itself or an agonist thereof is used, the dose is preferably a molar equivalent of a PYY₃₋₃₆ dose, or achieves the same effect as a PYY₃₋₃₆ dose, as described above. The doses can be calculated on the basis of a subject, such as a subject weighing from 70 to 75 kg. The exact dose is readily determined by one of skill in the art based on the potency of the specific compound (such as the PYY polypeptide, or agonist) utilized, and the age, weight, sex and physiological condition of the subject.

All the teachings given above in relation to PYY₃₋₃₆ also apply to PYY itself and to PYY agonists. For example, the teachings relating to routes of administration and dosage regimes.

The GLP-1 or an agonist thereof should be administered in an therapeutically effective dose, see the section relating to GLP-1 and agonists thereof below. GLP-1 or an agonist thereof may be used in amounts or doses in the ranges described above for PYY or a PYY agonist. The doses of the various agent may be independent of each other or, for example, equimolar doses may be used, for example, equimolar doses of GLP-1 or an agonist thereof and PYY or an agonist thereof.

As disclosed herein, a naturally occurring peptide, PYY or PYY₃₋₃₆ can be used to achieve a physiological effect. This results in minimal side effects and enables long term use, if necessary. The dose of PYY or PYY₃₋₃₆ can be based on the physiological levels observed post-prandially. The normal circulating levels of PYY₃₋₃₆ are about 8 pmol/litre, typically rising to about 40 to 60 pmol/litre after a meal. PYY (e.g., PYY₃₋₃₆) and agonists can be used at analogous doses.

The considerations set out above in relation to the administration regime also relate to administration using the subcutaneous route.

### PYY Agonists

A PYY agonist, of use in the methods of the present disclosure, is a molecule that binds to a receptor that specifically binds PYY, and elicits an effect of PYY. Assays for binding to PYY receptors, and eliciting a response in a cell with a PYY receptor, are known in the art. A specific assay for detecting a PYY agonist is also disclosed herein. Thus, in one embodiment, a PYY agonist binds to a NPY neuron in the arcuate nucleus, which results in an electrophysiological effect on an NPY neuron. As disclosed herein, NPY neurons synapse with POMC neurons. Thus, the electrophysiological effect on the NYP neuron can result in a further electrophysiological effect on a POMC neuron. In one specific, non-limiting example, an administration of PYY agonist results in hyperpolization of the membrane potential of a POMC neuron. In another specific, non-limiting example, administration of a PYY agonist results in an increase in IPSCs in a POMC neuron.

In another embodiment, PYY agonists do not include NPY. Suitable PYY agonists include molecules that bind NPY neurons, but do not cross the blood/brain barrier. The arcuate nucleus neurons upon which PYY exerts its effects are not protected by the blood/brain barrier, and thus are readily accessible to peripherally available molecules. In addition, other brain sites that express the Y2 receptor are protected by the blood/brain barrier. Without being bound by theory, agents able to bind to the arcuate Y2R, but that do not cross the blood/brain barrier following peripheral administration, are likely to be of use.

In one embodiment, a PYY agonist is a compound that affects food intake, caloric intake, or appetite, and/or which binds specifically in a Y receptor assay or competes for binding with PYY, such as in a competitive binding assay with labeled PYY. PYY agonists include, but are not limited to, compounds that bind to the Y2 receptor.

PYY and agonists useful in the methods disclosed herein include, but are not limited to, polypeptides comprising, or alternatively consisting of, the amino acid sequence for PPY and agonists thereof, e.g., mutants, fragments and/or variants thereof. Variants include deletions, insertions, inversions, repeats and substitutions (e.g., conservative substitutions and non-conservative substitutions; see, e.g., Tables 1 and 2, *intra*). More than one amino acid (e.g., 2, 3, 4, 5, 6, 7, 8, 9, 10, etc.) can be deleted or inserted or substituted with another amino acid. Typically conservative substitutions are the replacements, one for another, among the aliphatic amino acids Ala, Val, Leu and Ile; interchange of Ser and Thr containing hydroxy residues, interchange of the acidic residues Asp and Glu, interchange between the amide residues Asn and Gln, interchange of the basic residues Lys and Arg, interchange of the aromatic residues Phe and Tyr, and interchange of the small-sized amino acids Ala, Ser, Thr, Met and Gly. Guidance concerning how to make phenotypically silent amino acid substitutions is provided in Bowie et al., Science 247:1306-1310, 1990.

As another example, polypeptide fragments may contain a continuous series of deleted residues from the amino (N)- or the carboxyl (C)- terminus, or both (see, e.g., Tables 1 and 2, *infra*). Any number of amino acids, ranging from 1 to 24, can be deleted from the N-terminus, the C-terminus or both.

Furthermore, the agonist polypeptides may also include, but are not limited to, polypeptides comprising, or alternatively consisting of, internal deletions of the amino acid sequences for PPY and/or agonist thereof (see, e.g., Table 2, *infra*). Such deletions may comprise one or more amino acid residue deletions (e.g., one, two, three, four, five, six, seven, eight, nine, ten, etc.) and may begin at any amino acid position (e.g., two, three, four, five, six, seven, eight, nine, ten, etc.). In addition, the polypeptides of this disclosure may contain one or more such internal deletions. Such deletions are contemplated in PPY, NPY and PP.

Also contemplated are agonist peptides that are PPY, NPY and/or PP chimeras having high affinity and/or selectivity for the Y2 receptor. These chimeras may comprise amino acid substitutions of one or more amino acids (e.g., 1, 2, 3, 4, 5, 6, 7, 8, 9, 10, etc.) from PPY, NPY and/or PP, variants, mutants and/or deletions thereof, with one or more amino acids (e.g., 1, 2, 3, 4, 5, 6, 7, 8, 9, 10, etc.) from a second PPY, NPY, or PP, variants, mutations and/or deletions thereof. These substitutions may begin at any amino acid position (e.g., two, three, four, five, six, seven, eight, nine, ten, etc.).

Preferably, the peptide is selective for the Y2 receptor. That is, it binds with higher affinity to Y2 compared to other receptors, such as Y1, Y2, Y3, Y4, Y5 and Y6. In another embodiment, the peptide is selective for the Y2 and Y5 receptors over the Y1, Y3, Y4 and Y6 receptors.

Other polypeptide fragments are fragments comprising structural or functional domain of the polypeptides of this disclosure. Such fragments include amino acid residues that comprise a polyproline-type II helix (residues 1-8), beta-turn (residues 9-14), amphipathic alpha-helix (residues 15-32) and/or a C-terminal turn structure (residues 33-36). See, Kirby et al., J Med Chem 36:385-393, 1993.

In addition, this disclosure includes the use of a polypeptide or agonist comprising, or alternatively consisting of, the amino acid sequence for PPY, NPY and PP species variants (see Table 1, *infra*) and/or mutants, and fragments thereof.

Also contemplated are fusion proteins, whereby a PYY or PYY agonist will be fused to another protein or polypeptide (the fusion partner) using recombinant methods known in the art. Alternatively, such a fusion protein may be synthetically synthesized by any known method. Any known peptide or protein can be used as the fusion partner (e.g., serum albumin, carbonic anhydrase, glutathione-S-transferase or thioredoxin, etc.). Preferred fusion partners will not have an adverse biological activity *in vivo*. Such fusion proteins may be designed linking the carboxy-terminus of the fusion partner to the amino-terminus of the PYY or agonist peptide, or vice versa. Optionally, a cleavable linker region may be used linking the PYY or PYY agonist to the fusion partner, and may be cleaved *in vivo* thereby resulting in the release of an active form of PYY or a PYY agonist. Examples of such cleavage regions include, but are not limited to, the linker regions D-D-D-D-Y (SEQ ID NO: 330), G-P-R (SEQ ID NO: 331), A-G-G (SEQ ID NO: 332) and H-P-F-H-L (SEQ ID NO 333), which can be cleaved by enterokinase, thrombin, ubiquitin cleaving enzyme and renin, respectfully. See, e.g., U.S. Patent No. 6,410,707.

Also contemplated as useful PYY agonists are Y2 specific NPY peptide agonists as described in U.S. Patent No. 5,026,685; U.S. Patent No. 5,574,010; U.S. Patent No. 5,604,203; U.S. Patent No. 5,696,093; U.S. Patent No. 6,046,167. See below:
Preferred PPY agonists are described herein as follows.

**TABLE 1 - PYY: Variation Among Species**

| PEPTIDE YY | AA SEQUENCE |
|---|---|
| Human | YPIKPEAPGEDASPEELNRYYASLRHYLNLVTRQRY (SEQ ID NO: 1) |
| Rat | YPAKPEAPGEDASPEELSRYYASLRHYLNLVTRQRY (SEQ ID NO: 5) |
| Pig | YPAKPEAPGEDASPEELSRYYASLRHYLNLVTRQRY (SEQ ID NO: 6) |
| Guinea pig | YPSKPEAPGSDASPEELARYYASLRHYLNLVTRQRY (SEQ ID NO: 7) |
| Frog | YPPKPENPGEDASPEEMTKYLTALRHYINLVTRQRY (SEQ ID NO: 8) |
| Raja | YPPKPENPGDDAAPEELAKYYSALRHYINLITRQRY (SEQ ID NO: 9) |
| Dogfish | YPPKPENPGEDAPPEELAKYYSALRHYINLITRQRY (SEQ ID NO: 10) |
| Lampetra | FPPKPDNPGDNASPEQMARYKAAVRHYINLITRQRY (SEQ ID NO: 11) |
| Petromyzon | MPPKPDNPSPDASPEELSKYMLAVRNYINLITRQRY (SEQ ID NO: 12) |
| | |

| NEUROPEPTIDE Y | AA SEQUENCE |
|---|---|
| Human | YPSKPDNPGEDAPAEDMARYYSALRHYINLITRQRY (SEQ ID NO: 2) |
| Rat | YPSKPDNPGEDAPAEDMARYYSALRHYINLITRQRY (SEQ ID NO: 13) |
| Rabbit | YPSKPDNPGEDAPAEDMARYYSALRHYINLITRQRY (SEQ ID NO: 14) |
| Dog | YPSKPDNPGEDAPAEDMARYYSALRHYINLITRQRY (SEQ ID NO: 15) |
| Pig | YPSKPDNPGEDAPAEDLARYYSALRHYINLITRQRY (SEQ ID NO: 16) |
| Cow | YPSKPDNPGEDAPAEDLARYYSALRHYINLITRQRY (SEQ ID NO: 17) |
| Sheep | YPSKPDNPGDDAPAEDLARYYSALRHYINLITRQRY (SEQ ID NO: 18) |
| Guinea pig | YPSKPDNPGEDAPAEDMARYYSALRHYINLITRQRY (SEQ ID NO: 19) |
| Avian | YPSKPDSPGEDAPAEDMARYYSALRHYINLITRQRY (SEQ ID NO: 20) |
| Rana | YPSKPDNPGEDAPAEDMAKYYSALRHYINLITRQRY (SEQ ID NO: 21) |
| Goldfish | YPTKPDNPGEGAPAEELAKYYSALRHYINLITRQRY (SEQ ID NO: 22) |
| Dogfish | YPSKPDNPGEGAPAEDLAKYYSALRHYINLITRQRY (SEQ ID NO: 23) |
| Lampetra | PPNKPDSPGEDAPAEDLARYLSAVRHYINLITRQRY (SEQ ID NO: 24) |
| | |

| PANCREATIC POLYPEPTIDE | AA SEQUENCE |
|---|---|
| Human | ASLEPEYPGDNATPEQMAQYAAELRRYINMLTRPRY (SEQ ID NO: 3) |
| Sheep | APLEPVYPGDNATPEQMAQYAADLRRYINMLTRPRY (SEQ ID NO: 25) |
| Pig | APLEPVYPGDDATPEQMAQYAAELRRYINMLTRPRY (SEQ ID NO: 26) |
| Dog | APLEPVYPGDDATPEQMAQYAAELRRYINMLTRPRY (SEQ ID NO: 27) |
| Cat | APLEPVYPGDNATPEQMAQYAAELRRYINMLTRPRY (SEQ ID NO: 28) |
| Cow | APLEPEYPGDNATPEQMAQYAAELRRYINMLTRPRY (SEQ ID NO: 29) |
| Rat | APLEPMYPGDYATHEQRAQYETQLRRYINTLTRPRY (SEQ ID NO: 30) |
| Mouse | APLEPMYPGDYATPEQMAQYETQLRRYINTLTRPRY (SEQ ID NO: 31) |
| Guinea pig | APLEPVYPGDNATPEQQMAQYAAEMRRYINMLTRPRY (SEQ ID NO: 32) |
| Chicken | GPSQPTYPGDDAPVEDLIRFYNDLQQYLNVVTRHRY (SEQ ID NO: 33) |
| Alligator | TPLQPKYPGDGAPVEDLIQFYNDLQQYLNVVTRPRF (SEQ ID NO: 34) |
| Bullfrog | APSEPHHPGDQATPDQLAQYYSDLYQYITFITRPRF (SEQ ID NO: 35) |
| Ref: Beck-Sickinger, A.G., Jung, G., Biopolymers 37:123-142, 1995*.* | |

**TABLE 2 - PEPTIDE AGONIST OF PYY**

| PEPTIDE | SEQUENCE |
|---|---|
| **PPY(3-36)(human)** | |
| | IKPEAPGEDASPEELNRYYASLRHYLNLVTRQRY (SEQ ID NO: 334) |
| Ref: Eberlein et al., Peptides 10:797-803, 1989; Grandt et al., Peptides 15(5):815-20, 1994. | |
| | |

| Variations of PPY(3-36) | |
|---|---|
| N-Terminal Deletions of PYY, including but not limited to: PYY(26-36), PYY(25-36), PYY(24-36), PYY(23-36), PYY(22-36), PYY(21-36), PYY(20-36), PYY(19-36), PYY(18-36), PYY(17-36), PYY(16-36), PYY(15-36), PYY(14-36), PYY(13-36), PYY(12-36), PYY(11-36), PYY(10-36), PYY(9-36), PYY(8-36), PYY(7-36), PYY(6-36), PYY(5-36), PYY(4-36), PYY(3-36). | |
| Ref: See, e.g., Balasubramaniam et al., Pept Res 1(1):32-5, Sep-Oct 1998; Liu et al., J Gastrointest Surg 5(2):147-52, Mar-Apr 2001. | |
| | |

| PEPTIDE | SEQUENCE |
|---|---|
| NPY (human) | |
| | YPSKPDNPGEDAPAEDMARYYSALRHYINLITRQRY (SEQ ID NO: 2) |
| Ref: Tatemoto et al., Proc Natl Acad Sci U.S.A. 79:5485-9, 1982. | |

### Variations of NPY

N-Terminal Deletions of NPY, including but not limited to: NPY(26-36), NPY(25-36), NPY(24-36), NPY(23-36), NPY(22-36), NPY(21-36), NPY(20-36), NPY(19-36), NPY(18-36), NPY(17-36), NPY(16-36), NPY(15-36), NPY(14-36), NPY(13-36), NPY(12-36), NPY(11-36), NPY(10-36), NPY(9-36), NPY(8-36), NPY(7-36), NPY(6-36), NPY(5-36), NPY(4-36), NPY(3-36).
Ref: See e.g., Gehlert et al., Proc Soc Exp Biol Med 218:7-22, 1998; Sheikh et al., Am J Physiol 261:G701-15, Nov. 1991.

Internal Deletions, including but not limited to: (1-4)-Aca-(14-36)pNPY, (1-4)-Aca-(15-36)pNPY, (1-4)-Aca-(16-36)pNPY, (1-4)-Aca-(17-36)pNPY, (1-4)-Aca-(18-36)pNPY, (1-4)-(31-36)pNPY11, (1-4)-Aca-(31-36)pNPY, (4-1)-(31-36)pNPY, (4-1)-Aca-(31-36)pNPY, (4-1)_{D}-(31-36)pNPY, (4-1)_{D}-Aca-(31-36)pNPY. Ref: Fournier et al., Mol Pharmacol 45(1):93-101, Jan 1994.

Additional Internal Deletion Mutants, including but not limited to: des-AA¹⁰⁻¹⁷-NPY, des-AA¹⁰⁻¹⁷, Ac-[D-Lys⁹(ε-Ac-Ala)]NPY, des-AA¹⁰⁻¹⁷, Ac[D-Lys⁹(ε-Ac-Ala)]NPY, des-AA¹⁰⁻¹⁷[Ala^{7,21}]NPY, des-AA¹⁰⁻¹⁷[Cys^{7,21}]NPY, des-AA¹⁰-¹⁷[Glu⁷,Lys²¹]NPY, des-AA¹¹⁻¹⁷[D-Lys¹⁰(ε-Ac), Cys^{7,21}]NPY, des-AA¹⁰⁻¹⁷[D-Cys⁷, D-Lys(ε-Ac), Cys²¹]NPY,des-AA¹⁰⁻¹⁷[D-Cys⁷, Lys⁹(ε-Ac), Cys²¹]NPY, des-AA¹⁰-¹⁷[Cys^{7,21}, Pro³⁴]NPY, des-AA¹⁰⁻¹⁷[Asp⁷, Dpr²¹, Pro³⁴]NPY, des-AA¹⁰⁻¹⁷[Glu⁷, Lys²¹, Pro³⁴]NPY, des-AA¹⁰⁻¹⁷[Cys^{7,21}, Leu³¹, Pro³⁴]NPY, des-AA¹⁰⁻²⁰[Cys^{7,21}, Pro³⁴]NPY, des-AA¹⁰⁻¹⁷[Cys^{2,27}]NPY, des-AA¹⁰⁻¹⁷[Cys², D-Cys²⁷]NPY.
Ref: Kirby et al., J Med Chem 38:4579-86, 1995.

Cyclic agonist of NPY, including but not limited to: [Lys 25-Glu 29]NPY(Ac-25-36), [Glu 25-Lys 29]NPY(Ac-25-36), [Lys 26-Glu31]NPY(Ac-25-36), [Glu 27-Lys 31]NPY(Ac-25-36), [Lys28-Glu 32]NPY(Ac-25-36), [Lys27-Glu34]NPY(Ac-25-36).
Ref: Rist et al., Eur J Biochem 247:1019-1028, 1997.

D-amino acid substitutions: [D-Tyr¹]NPY, [D-Pro²]NPY, [D-Ser³]NPY, [D-Lys⁴]NPY, [D-Pro⁵]NPY, [D-Asp⁶]NPY, [D-Asn⁷]NPY, [D-Pro⁸]NPY, [D-Ala⁹]NPY, [D-Glu¹⁰]NPY, [D-Asp¹¹]NPY, [D-Ala¹²]NPY, [D-Pro¹³]NPY, [D-Ala¹⁴]NPY, [D-Glu¹⁵]NPY, [D-Asp¹⁶]NPY, [D-Leu¹⁷]NPY, [D-Ala¹⁸]NPY, [D-Arg¹⁹]NPY, [D-Tyr²⁰]NPY, [D-Tyr²¹]NPY, [D-Ser²²]NPY, [D-Ala²³]NPY, [D-Leu2⁴]NPY, [D-Arg²⁵]NPY, [D-His²⁶]NPY, [D-Tyr²⁷]NPY, [D-Ile²⁸]NPY, [D-Asn²⁹]NPY, [D-Leu³⁰]NPY, [D-Ile³¹]NPY, [D-Thr³²]NPY, [D-Arg³³]NPY, [D-Gln³⁴]NPY, [D-Arg³⁵]NPY, [D-Tyr³⁶]NPY, [D-Tyr¹, D-Pro²]NPY, [D-Ser³, D-Lys⁴]NPY, [D-Pro⁵, D-Asp⁶]NPY, [D-Asn⁷, D-Pro⁸]NPY, [D-Glu¹⁰, D-Asp¹¹]NPY, [D-Asp¹¹, D-Ala¹²]NPY, [D-Pro¹³, D-Ala¹⁴]NPY, [D-Glu¹⁵, D-Asp¹⁶]NPY, [D-Met¹⁷, D-Ala¹⁸]NPY, [D-Arg¹⁹, D-Tyr²⁰]NPY, [D-Tyr²¹, D-Ser²²]NPY, [D-Ala²³, D-Leu²⁴]NPY, [D-Arg²⁵, D-His²⁶]NPY, [D-Tyr²⁷, D-Ile²⁸]NPY, [D-Asn²⁹, D-Leu³⁰]NPY, [D-Ile³¹, D-Thr³²]NPY, [D-Arg³³, D-Gln³⁴]NPY, [D-Arg³⁵, D-Tyr³⁶]NPY.

Ref: Kirby et al., J Med Chem 36:3802-08, 1993; Grundemar et al., Regulatory Peptides 62:131-136, 1996.

### Other NPY Agonist and Analogs

| PEPTIDE | SEQUENCE |
|---|---|
| NPY(3-36) | |
| | SKPDNPGEDAPAEDMARYYSALRHYINLITRQRY (SEQ ID NO: 335) |
| Ref: Grandt et al., Regulatory Peptides 67(1):33-7, 1996. | |
| | |

| PEPTIDE | SEQUENCE |
|---|---|
| N-Acetyl NPY(24-36) | |
| | LRHYINLITRQRY (SEQ ID NO: 213) |
| Ref: Potter et al., Eur J Pharmacol 267(3):253-262, May 17, 1994. | |
| | |

| PEPTIDE | SEQUENCE |
|---|---|
| N-Acetyl [Leu²⁸, Leu³¹] NPY(24-36) | |
| | LRHYLNLLTRQRY (SEQ ID NO: 214) |
| Ref: Potter et al., Eur J Pharmacol 267(3):253-262, May 17, 1994. | |
| [Leu²⁸, Leu³¹] NPY(24-36) | |
| | LRHYLNLLTRQRY (SEQ ID NO: 215) |
| Ref: Potter et al., Eur J Pharmacol 267(3):253-262, May 17, 1994. | |
| | |

| PEPTIDE | SEQUENCE |
|---|---|
| [Leu¹⁷, Gln¹⁹, Ala²¹, Ala²², Glu²³, Leu²⁸, Leu³¹] NPY(13-36) | |
| | PAEDLAQYAAELRHYLNLLTRQRY (SEQ ID NO: 216) |
| Ref: Potter et al., Eur J Pharmacol 267(3):253-262, May 17, 1994. | |
| | |

| PEPTIDE | SEQUENCE |
|---|---|
| Cyclo S-S [Cys²⁰,Cys²⁴]pNPY | |
| | SKPDNPGEDAPAEDMARCYSACRHYINLITRQRY (SEQ ID NO: 315) |
| Ref: Soll et al., Eur J Biochem 268(10):2828-37, May 2001. | |
| | |

| PEPTIDE | SEQUENCE |
|---|---|
| Cyclo-(28/32)-Ac-[Lys²⁸-Glu³²]-(25-36)-pNPY | |
| | RHYLNLIGRQRY (SEQ ID NO: 316) |
| Ref: Cabrele et al., J Pept Sci 6(3):97-122, Mar 2000. | |
| | |

| PEPTIDE | SEQUENCE |
|---|---|
| Cyclo-(27/31)-Ac-[Glu²⁷-Lys³¹]-(25-36)-pNPY | |
| | RHGLNLLGRQRY (SEQ ID NO: 317) |
| Ref: Cabrele et al., J Pept Sci 6(3):97-122, Mar 2000. | |
| | |

| PEPTIDE | SEQUENCE |
|---|---|
| [Tyr³², Leu³⁴]NPY(27-36) | |
| | YINLIYRLRY (SEQ ID NO: 318) |
| Ref: Leban et al., JMed Chem 38:1150-57, 1995. | |
| | |

| PEPTIDE | SEQUENCE |
|---|---|
| [Tyr³², Leu³⁴]NPY(26-36) | |
| | HYINLIYRLRY (SEQ ID NO: 319) |
| Ref: Leban et al., JMed Chem 38:1150-57, 1995. | |
| [Tyr³², Leu³⁴]NPY(25-36) | |
| | RHYINLIYRLRY (SED ID NO: 320) |
| Ref: Leban et al., J Med Chem 38:1150-57, 1995. | |
| | |
| [Leu³¹]NPY(27-36) | |
| | YINLLYRQRY (SEQ ID NO: 321) |
| Ref: Leban et al., J Med Chem 38:1150-57, 1995. | |
| | |

| PEPTIDE | SEQUENCE |
|---|---|
| [Tyr³², Leu³⁴] (1-4)-Ahr-(27-36)NPY | |
| | YPSL-Aha-YINLIYRLRY (SED ID NO: 322) |
| Ref: Leban et al., J Med Chem 38:1150-57, 1995. | |
| | |

| PEPTIDE | SEQUENCE |
|---|---|
| [Tyr³², Leu³⁴]NPY(28-36) | |
| | INLIYRLRY (SEQ ID NO: 323) |
| Ref: Leban et al., J Med Chem 38:1150-57, 1995. | |
| | |

| PEPTIDE | SEQUENCE |
|---|---|
| PP (human) | |
| | ASLEPEYPGDNATPEQMAQYAAELRRYINMLTRPRY (SEQ ID NO: 3) |
| Ref: Kimmel et al., Endocrinology 83:1323-30, 1968. | |

### Variations of PP

N-Terminal Deletions including but not limited to: PP(26-36), PP(25-36), PP(24-36), PP(23-36), PP(22-36), PP(21-36), PP(20-36), PP(19-36), PP(18-36), PP(17-36), PP(16-36), PP(15-36), PP(14-36), PP(13-36), PP(12-36), PP(11-36), PP(10-36), PP(9-36), PP(8-36), PP(7-36), PP(6-36), PP(5-36), PP(4-36), PP(3-36).

**TABLE 3 - EXAMPLES OF CONSERVATIVE AMINO ACID SUBSTITUTIONS OF PYY**

| Single point mutations of PYY(25-36) | |
|---|---|
| PEPTIDE | SEQUENCE |
| [Lys²⁵]PPY(25-36) | KHYLNLVTRQRY (SEQ ID NO: 36) |
| [Thr²⁷]PPY(25-36) | RHTLNLVTRQRY (SEQ ID NO: 37) |
| [Phe²⁷]PPY(25-36) | RHFLNLVTRQRY (SEQ ID NO: 38) |
| [Ile²⁸]PYY (25-36) | RHYINLVTRQRY (SEQ ID NO: 39) |
| [Val²⁸]PYY (25-36) | RHYVNLVTRQRY (SEQ ID NO: 40) |
| [Gln²⁹]PYY (25-36) | RHYLQLVTRQRY (SEQ ID NO: 41) |
| [Ile³⁰]PYY (25-36) | RHYLNIVTRQRY (SEQ ID NO: 42) |
| [Val³⁰]PYY (25-36) | RHYLNVVTRQRY (SEQ ID NO: 43) |
| [Ile³¹]PYY (25-36) | RHYLNLITRQRY (SEQ ID NO: 44) |
| [Leu³¹]PYY (25-36) | RHYLNLLTRQRY (SEQ ID NO: 45) |
| [Ser³²]PYY (25-36) | RHYLNLVSRQRY (SEQ ID NO: 46) |
| [Lys³³]PYY (25-36) | RHYLNLVTKQRY (SEQ ID NO: 47) |
| [Asn³⁴]PYY (25-36) | RHYLNLVTRNRY (SEQ ID NO: 48) |
| [Lys³⁵]PYY (25-36) | RHYLNLVTRQKY (SEQ ID NO: 49) |
| [Thr³⁶]PYY (25-36) | RHYLNLVTRQRT (SEQ ID NO: 50) |
| [Phe³⁶]PYY (25-36) | RHYLNLVTRQRF (SEQ ID NO: 51) |
| | |

| Double point mutations | |
|---|---|
| PEPTIDE | SEQUENCE |
| [Lys²⁵, Thr²⁷]PPY(25-36) | KHTLNLVTRQRY (SEQ ID NO: 52) |
| [Lys²⁵, Phe²⁷]PPY(25-36) | KHFLNLVTRQRY (SEQ ID NO: 53) |
| [Lys²⁵, Ile²⁸]PPY(25-36) | KHYINLVTRQRY (SEQ ID NO: 54) |
| [Lys²⁵, Val²⁸]PPY(25-36) | KHYVNLVTRQRY (SEQ ID NO: 55) |
| [Lys²⁵, Gln²⁹]PPY(25-36) | KHYLQLVTRQRY (SEQ ID NO: 56) |
| [Lys²⁵, Ile³⁰]PPY(25-36) | KHYLNIVTRQRY (SEQ ID NO: 57) |
| [Lys²⁵, Val³⁰]PPY(25-36) | KHYLNVVTRQRY (SEQ ID NO: 58) |
| [Lys²⁵, Ile³¹]PPY(25-36) | KHYLNLITRQRY (SEQ ID NO: 59) |
| [Lys²⁵, Leu³¹]PPY(25-36) | KHYLNLLTRQRY (SEQ ID NO: 60) |
| [Lys²⁵, Ser³²]PPY(25-36) | KHYLNLVSRQRY (SEQ ID NO: 61) |
| [Lys²⁵, Lys³³]PPY(25-36) | KHYLNLVTKQRY (SEQ ID NO: 62) |
| [Lys²⁵, Asn³⁴]PPY(25-36) | KHYLNLVTRNRY (SEQ ID NO: 63) |
| [Lys²⁵, Lys³⁵]PPY(25-36) | KHYLNLVTRQKY (SEQ ID NO: 64) |
| [Lys²⁵, Thr³⁶]PPY(25-36) | KHYLNLVTRQRT (SEQ ID NO: 65) |
| [Lys²⁵, Phe³⁶]PPY(25-36) | KHYLNLVTRQRF (SEQ ID NO: 66) |
| [Thr²⁷, Ile²⁸]PPY(25-36) | RHTINLVTRQRY (SEQ ID NO: 67) |
| [Thr²⁷, Val²⁸]PPY(25-36) | RHTVNLVTRQRY (SEQ ID NO: 68) |
| [Thr²⁷, Gln²⁹]PPY(25-36) | RHTLQLVTRQRY (SEQ ID NO: 69) |
| [Thr²⁷, Ile³⁰]PPY(25-36) | RHTLNIVTRQRY (SEQ ID NO: 70) |
| [Thr²⁷, Val³⁰]PPY(25-36) | RHTLNVVTRQRY (SEQ ID NO: 71) |
| [Thr²⁴, Ile³¹]PPY(25-36) | RHTLNLITRQRY (SEQ ID NO: 72) |
| [Thr²⁷, Leu³¹]PPY(25-36) | RHTLNLLTRQRY (SEQ ID NO: 73) |
| [Thr²⁷, Ser³²]PPY(25-36) | RHTLNLVSRQRY (SEQ ID NO: 74) |
| [Thr²⁷, Lys³³]PPY(25-36) | RHTLNLVTKQRY (SEQ ID NO: 75) |
| [Thr²⁷, Asn³⁴]PPY(25-36) | RHTLNLVTRNRY (SEQ ID NO: 76) |
| [Thr²⁷, Lys³⁵]PPY(25-36) | RHTLNLVTRQKY (SEQ ID NO: 77) |
| [Thr²⁷, Thr³⁶]PPY(25-36) | RHTLNLVTRQRT (SEQ ID NO: 78) |
| [Thr²⁷, Phe³⁶]PPY(25-36) | RHTLNLVTRQRF (SEQ ID NO: 79) |
| [Phe²⁷, Ile²⁸]PPY(25-36) | RHFINLVTRQRY (SEQ ID NO: 80) |
| [Phe²⁷, Val²⁸]PPY(25-36) | RHFVNLVTRQRY (SEQ ID NO: 81) |
| [Phe²⁷, Gln²⁹]PPY(25-36) | RHFLQLVTRQRY (SEQ ID NO: 82) |
| [Phe²⁷, Ile³⁰]PPY(25-36) | RHFLNIVTRQRY (SEQ ID NO: 83) |
| [Phe²⁷, Val³⁰]PPY(25-36) | RHFLNVVTRQRY (SEQ ID NO: 84) |
| [Phe²⁷, Ile³¹]PPY(25-36) | RHFLNLITRQRY (SEQ ID NO: 85) |
| [Phe²⁷, Leu³¹]PPY(25-36) | RHFLNLLTRQRY (SEQ ID NO: 86) |
| [Phe²⁷, Ser³²]PPY(25-36) | RHFLNLVSRQRY (SEQ ID NO: 87) |
| [Phe²⁷, Lys³³]PPY(25-36) | RHFLNLVTKQRY (SEQ ID NO: 88) |
| [Phe²⁷, Asn³⁴]PPY(25-36) | RHFLNLVTRNRY (SEQ ID NO: 89) |
| [Phe²⁷, Lys³⁵]PPY(25-36) | RHFLNLVTRQKY (SEQ ID NO: 90) |
| [Phe²⁷, Thr³⁶]PPY(25-36) | RHFLNLVTRQRT (SEQ ID NO: 91) |
| [Phe²⁷, Phe³⁶]PPY(25-36) | RHFLNLVTRQRF (SEQ ID NO: 92) |
| [Gln²⁹, Ile³⁰]PYY (25-36) | RHYLQIVTRQRY (SEQ ID NO: 93) |
| [Gln²⁹, Val³⁰]PYY (25-36) | RHYLQVVTRQRY (SEQ ID NO: 94) |
| [Gln²⁹, Ile³¹]PYY (25-36) | RHYLQLITRQRY (SEQ ID NO: 95) |
| [Gln²⁹, Leu³¹]PYY (25-36) | RHYLQLLTRQRY (SEQ ID NO: 96) |
| [Gln²⁹, Ser³²]PYY (25-36) | RHYLQLVSRQRY (SEQ ID NO: 97) |
| [Gln²⁹, Leu³³]PYY (25-36) | RHYLQLVTKQRY (SEQ ID NO: 98) |
| [Gln²⁹, Asn³⁴]PYY (25-36) | RHYLQLVTRNRY (SEQ ID NO: 99) |
| [Gln²⁹, Leu³⁵]PYY (25-36) | RHYLQLVTRQKY (SEQ ID NO: 100) |
| [Gln²⁹, Thr³⁶]PYY (25-36) | RHYLQLVTRQRT (SEQ ID NO: 101) |
| [Gln²⁹, Phe³⁶]PYY (25-36) | RHYLQLVTRQRF (SEQ ID NO: 102) |
| [Ile³⁰, Ile³¹]PYY (25-36) | RHYLNIITRQRY (SEQ ID NO: 103) |
| [Ile³⁰, Leu³¹]PYY (25-36) | RHYLNILTRQRY (SEQ ID NO: 104) |
| [Ile³⁰, Ser³²]PYY (25-36) | RHYLNIVSRQRY (SEQ ID NO: 105) |
| [Ile³⁰, Lys³³]PYY (25-36) | RHYLNIVTKQRY (SEQ ID NO: 106) |
| [Ile³⁰, Asn³⁴]PYY (25-36) | RHYLNIVTRNRY (SEQ ID NO: 107) |
| [Ile³⁰, Lys³⁵]PYY (25-36) | RHYLNIVTRQKY (SEQ ID NO: 108) |
| [Ile³⁰, Thr³⁶]PYY (25-36) | RHYLNIVTRQRT (SEQ ID NO: 109) |
| [Ile³⁰, Phe³⁶]PYY (25-36) | RHYLNIVTRQRF (SEQ ID NO: 110) |
| [Val³⁰, Ile³¹]PYY (25-36) | RHYLNVITRQRY (SEQ ID NO: 111) |
| [Val³⁰, Leu³¹]PYY (25-36) | RHYLNVLTRQRY (SEQ ID NO: 112) |
| [Val³⁰, Ser³²]PYY (25-36) | RHYLNVVSRQRY (SEQ ID NO: 113) |
| [Val³⁰, Lys³³]PYY (25-36) | RHYLNVVTKQRY (SEQ ID NO: 114) |
| [Val³⁰, Asn³⁴]PYY (25-36) | RHYLNVVTRNRY (SEQ ID NO: 115) |
| [Val³⁰, Lys³⁵]PYY (25-36) | RHYLNVVTRQKY (SEQ ID NO: 116) |
| [Val³⁰, Thr³⁶]PYY (25-36) | RHYLNVVTRQRT (SEQ ID NO: 117) |
| [Val³⁰, Phe³⁶]PYY (25-36) | RHYLNVVTRQRF (SEQ ID NO: 118) |
| [Ile³¹, Ser³²]PYY (25-36) | RHYLNLISRQRY (SEQ ID NO: 119) |
| [Ile³¹, Lys³³]PYY (25-36) | RHYLNLITKQRY (SEQ ID NO: 120) |
| [Ile³¹, Asn³⁴]PYY (25-36) | RHYLNLITRNRY (SEQ ID NO: 121) |
| [Ile³¹, Lys³⁵]PYY (25-36) | RHYLNLITRQKY (SEQ ID NO: 122) |
| [Ile³¹, Thr³⁶]PYY (25-36) | RHYLNLITRQRT (SEQ ID NO: 123) |
| [Leu³¹, Phe³⁶]PYY (25-36) | RHYLNLITRQRF (SEQ ID NO: 124) |
| [Leu³¹, Ser³²]PYY (25-36) | RHYLNLLSRQRY (SEQ ID NO: 125) |
| [Val³¹, Lys³³]PYY (25-36) | RHYLNLLTKQRY (SEQ ID NO: 126) |
| [Leu³¹, Asn³⁴]PYY (25-36) | RHYLNLLTRNRY (SEQ ID NO: 127) |
| [Leu³¹, Lys³⁵]PYY (25-36) | RHYLNLLTRQKY (SEQ ID NO: 128) |
| [Leu³¹, Thr³⁶]PYY (25-36) | RHYLNLLTRQRT (SEQ ID NO: 129) |
| [Leu³¹, Phe³⁶]PYY (25-36) | RHYLNLLTRQRF (SEQ ID NO: 130) |
| [Ser³², Lys³³]PYY (25-36) | RHYLNLVSKQRY (SEQ ID NO: 131) |
| [Ser³², Asn³⁴]PYY (25-36) | RHYLNLVSRNRY (SEQ ID NO: 132) |
| [Ser³², Lys³⁵]PYY (25-36) | RHYLNLVSRQKY (SEQ ID NO: 133) |
| [Ser³², Thr³⁶]PYY (25-36) | RHYLNLVSRQRT (SEQ ID NO: 134) |
| [Ser³², Phe³⁶]PYY (25-36) | RHYLNLVSRQRY (SEQ ID NO: 135) |
| [Lys³³, Asn³⁴]PYY (25-36) | RHYLNLVTKNRY (SEQ ID NO: 136) |
| [Lys³³, Lys³⁵]PYY (25-36) | RHYLNLVTKQKY (SEQ ID NO: 137) |
| [Lys³³, Thr³⁶]PYY (25-36) | RHYLNLVTKQRT (SEQ ID NO: 138) |
| [Lys³³, Phe³⁶]PYY (25-36) | RHYLNLVTKQRF (SEQ ID NO: 139) |
| [Asn³⁴, Lys³⁵]PYY (25-36) | RHYLNLVTRNKY (SEQ ID NO: 140) |
| [Asn³⁴, Thr³⁶]PYY (25-36) | RHYLNLVTRNRT (SEQ ID NO: 141) |
| [Asn³⁴, Phe³⁶]PYY (25-36) | RHYLNLVTRNRF (SEQ ID NO: 142) |
| [Lys³⁵, Thr³⁶]PYY (25-36) | RHYLNLVTRQKT (SEQ ID NO: 143) |
| [Lys³⁵, Phe³⁶]PYY (25-36) | RHYLNLVTRQKF (SEQ ID NO: 144) |

| Point Mutations of PYY(24-36) | |
|---|---|
| PEPTIDE | SEQUENCE |
| PYY(24-36) | LRHYLNLVTRQRY (SEQ ID NO: 145) |
| [Ile²⁴]PYY(24-36) | IRHYLNLVTRQRY (SEQ ID NO: 146) |
| [Val²⁴]PYY(24-36) | VRHYLNLVTRQRY (SEQ ID NO: 147) |

Also included as PYY(24-36) mutations are polypeptide variations (amino acid sequence variations) resulting from the combination of any of these three mutants with any of the above listed mutants for PYY(25-36), e.g., [Lys²⁵]PPY(24-36) (Amino acid sequence=LKHYLNLVTRQRY (SEQ ID NO: 191)) would result from combining the mutations from SEQ ID NO: 36 with SEQ ID NO: 145.

### Point Mutations of PYY(23-36)

| PEPTIDE | SEQUENCE |
|---|---|
| PYY(23-36) | SLRHYLNLVTRQRY (SEQ ID NO: 148) |
| [Thr²³] PYY(23-36) | TLRHYLNLVTRQRY (SEQ ID NO: 149) |

Also included as PYY(23-36) mutations are polypeptide variations (amino acid sequence variations) resulting from the combination of either of these two mutants with any of the above listed mutants for PYY(25-36), and/or any of the above listed mutants for PYY(24-36), e.g., [Lys²⁵]PPY(23-36) (Amino acid sequence=SLKHYLNLVTRQRY (SEQ ID NO: 192)) would result from combining the mutations from SEQ ID NO: 36 with SEQ ID NO: 148.

### Point Mutations of PYY(22-36)

| PEPTIDE | SEQUENCE |
|---|---|
| PYY(22-36) | ASLRHYLNLVTRQRY (SEQ ID NO: 150) |
| [Ser²²)PYY(22-36) | SSLRHYLNLVTRQRY (SEQ ID NO: 151) |

Also included as PYY(22-36) mutations are polypeptide variations (amino acid sequence variations) resulting from the combination of either of these two mutants with any of the above listed mutants for PYY(25-36), and/or any of the above listed mutants for PYY(23-36), e.g., [Lys²⁵]PPY(22-36) (Amino acid sequence=ASLKHYLNLVTRQRY (SEQ ID NO: 193)) would result from combining the mutations from SEQ ID NO: 36 with SEQ ID NO: 150.

### Point Mutations of PYY(21-36)

| PEPTIDE | SEQUENCE |
|---|---|
| PYY(21-36) | YASLRHYLNLVTRQRY (SEQ ID NO: 152) |
| [Thr²¹]PYY(21-36) | TASLRHYLNLVTRQRY (SEQ ID NO: 153) |
| [Phe²¹]PYY(21-36) | FASLRHYLNLVTRQRY (SEQ ID NO: 154) |

Also included as PYY(21-36) mutations are polypeptide variations (amino acid sequence variations) resulting from the combination of any of these three mutants with any of the above listed mutants for PYY(25-36), and/or any of the above listed mutants for PYY(22-36), e.g., [Lys²⁵]PPY(21-36) (Amino acid sequence=YASLKHYLNLVTRQRY (SEQ ID NO: 194)) would result from combining the mutations from SEQ ID NO: 36 with SEQ ID NO: 152.

### Point Mutations of PYY(20-36)

| PEPTIDE | SEQUENCE |
|---|---|
| PYY(20-36) | YYASLRHYLNLVTRQRY (SEQ ID NO: 155) |
| [Thr²⁰]PYY(20-36) | TYASLRHYLNLVTRQRY (SEQ ID NO: 156) |
| [Phe²⁰]PYY(20-36) | FYASLRHYLNLVTRQRY (SEQ ID NO: 157) |

Also included as PYY(20-36) mutations are polypeptide variations (amino acid sequence variations) resulting from the combination of any of these three mutants with any of the above listed mutants for PYY(25-36), and/or any of the above listed mutants for PYY(21-36), e.g., [Lys²⁵]PPY(20-36) (Amino acid sequence=YYASLKHYLNLVTRQRY (SEQ ID NO: 195)) would result from combining the mutations from SEQ ID NO: 36 with SEQ ID NO: 155.

### Point Mutations of PYY(19-36)

| PEPTIDE | SEQUENCE |
|---|---|
| PYY(19-36) | RYYASLRHYLNLVTRQRY (SEQ ID NO: 158) |
| [Lys¹⁹]PYY(19-36) | KYYASLRHYLNLVTRQRY (SEQ ID NO: 159) |

Also included as PYY(19-36) mutations are polypeptide variations (amino acid sequence variations) resulting from the combination of either of these two mutants with any of the above listed mutants for PYY(25-36), and/or any of the above listed mutants for PYY(20-36), e.g., [Lys²⁵]PPY(19-36) (Amino acid sequence=RYYASLKHYLNLVTRQRY (SEQ ID NO: 196)) would result from combining the mutations from SEQ ID NO: 36 with SEQ ID NO: 158.

### Point Mutations of PYY(18-36)

| PEPTIDE | SEQUENCE |
|---|---|
| PYY(18-36) | NRYYASLRHYLNLVTRQRY (SEQ ID NO: 160) |
| [Gln¹⁸]PYY(18-36) | QRYYASLRHYLNLVTRQRY (SEQ ID NO: 161) |

Also included as PYY(18-36) mutations are polypeptide variations (amino acid sequence variations) resulting from the combination of either of these two mutants with any of the above listed mutants for PYY(25-36), and/or any of the above listed mutants for PYY(19-36), e.g., [Lys²⁵]PPY(18-36) (Amino acid sequence=NRYYASLKHYLNLVTRQRY (SEQ ID NO: 197)) would result from combining the mutations from SEQ ID NO: 36 with SEQ ID NO: 160.

### Point Mutations of PYY(17-36)

| PEPTIDE | SEQUENCE |
|---|---|
| PYY(17-36) | LNRYYASLRHYLNLVTRQRY (SEQ ID NO: 162) |
| [Ile¹⁷]PYY(17-36) | INRYYASLRHYLNLVTRQRY (SEQ ID NO: 163) |
| [Val¹⁷]PYY(17-36) | VNRYYASLRHYLNLVTRQRY (SEQ ID NO: 164) |

Also included as PYY(17-36) mutations are polypeptide variations (amino acid sequence variations) resulting from the combination of any of these three mutants with any of the above listed mutants for PYY(25-36), and/or any of the above listed mutants for PYY(18-36), e.g., [Lys²⁵]PPY(17-36) (Amino acid sequence=LNRYYASLKHYLNLVTRQRY (SEQ ID NO: 198)) would result from combining the mutations from SEQ ID NO: 36 with SEQ ID NO: 162.

### Point Mutations of PYY(16-36)

| PEPTIDE | SEQUENCE |
|---|---|
| PYY(16-36) | ELNRYYASLRHYLNLVTRQRY (SEQ ID NO: 165) |
| [Asp¹⁶]PYY(16-36) | DLNRYYASLRHYLNLVTRQRY (SEQ ID NO: 166) |

Also included as PYY(16-36) mutations are polypeptide variations (amino acid sequence variations) resulting from the combination of either of these two mutants with any of the above listed mutants for PYY(25-36), and/or any of the above listed mutants for PYY(17-36), e.g., [Lys²⁵]PPY(16-36) (Amino acid sequence=ELNRYYASLKHYLNLVTRQRY (SEQ ID NO: 199)) would result from combining the mutations from SEQ ID NO: 36 with SEQ ID NO: 165.

### Point Mutations of PYY(15-36)

| PEPTIDE | SEQUENCE |
|---|---|
| PYY(15-36) | EELNRYYASLRHYLNLVTRQRY (SEQ ID NO: 167) |
| [Asp¹⁵]PYY(15-36) | DELNRYYASLRHYLNLVTRQRY (SEQ ID NO: 168) |

Also included as PYY(15-36) mutations are polypeptide variations (amino acid sequence variations) resulting from the combination of either of these two mutants with any of the above listed mutants for PYY(25-36), and/or any of the above listed mutants for PYY(16-36), e.g., [Lys²⁵]PPY(15-36) (Amino acid sequence=EELNRYYASLKHYLNLVTRQRY (SEQ ID NO: 200)) would result from combining the mutations from SEQ ID NO: 36 with SEQ ID NO: 167.

### Point Mutations of PYY(14-36)

| PEPTIDE | SEQUENCE |
|---|---|
| PYY(14-36) | PEELNRYYASLRHYLNLVTRQRY (SEQ ID NO: 169) |

Also included as PYY(14-36) mutations are polypeptide variations (amino acid sequence variations) resulting from the combination of this PYY(14-36) mutant with any of the above listed mutants for PYY(25-36), and/or any of the above listed mutants for PYY(15-36), e.g., [Lys²⁵]PPY(23-36) (Amino acid sequence=PEELNRYYASLKHYLNLVTRQRY (SEQ ID NO: 201) would result from combining the mutations from SEQ ID NO: 36 with SEQ ID NO: 169.

### Point Mutations of PYY(13-36)

| PEPTIDE | SEQUENCE |
|---|---|
| PYY(13-36) | SPEELNRYYASLRHYLNLVTRQRY (SEQ ID NO: 170) |
| [Thr¹³]PYY(13-36) | TPEELNRYYASLRHYLNLVTRQRY (SEQ ID NO: 171) |

Also included as PYY(13-36) mutations are polypeptide variations (amino acid sequence variations) resulting from the combination of either of these two mutants with any of the above listed mutants for PYY(25-36), and/or any of the above listed mutants for PYY(14-36), e.g., [Lys²⁵]PPY(13-36) (Amino acid sequence=SEELNRYYASLKHYLNLVTRQRY (SEQ ID NO: 202)) would result from combining the mutations from SEQ ID NO: 36 with SEQ ID NO: 170.

### Point Mutations of PYY(12-36)

| PEPTIDE | SEQUENCE |
|---|---|
| PYY(12-36) | ASPEELNRYYASLRHYLNLVTRQRY (SEQ ID NO: 172) |
| [Ser¹²]PYY(12-36) | SSPEELNRYYASLRHYLNLVTRQRY (SEQ ID NO: 173) |

Also included as PYY(12-36) mutations are polypeptide variations (amino acid sequence variations) resulting from the combination of either of these two mutants with any of the above listed mutants for PYY(25-36), and/or any of the above listed mutants for PYY(13-36), e.g., [Lys²⁵]PPY(12-36) (Amino acid sequence=ASEELNRYYASLKHYLNLVTRQRY (SEQ ID NO: 203)) would result from combining the mutations from SEQ ID NO: 36 with SEQ ID NO: 172.

### Point Mutations of PYY(11-36)

| PEPTIDE | SEQUENCE |
|---|---|
| PYY(11-36) | DASPEELNRYYASLRHYLNLVTRQRY (SEQ ID NO: 174) |
| [Glu¹¹]PYY(11-36) | EASPEELNRYYASLRHYLNLVTRQRY (SEQ ID NO: 175) |

Also included as PYY(12-36) mutations are polypeptide variations (amino acid sequence variations) resulting from the combination of either of these two mutants with any of the above listed mutants for PYY(25-36), and/or any of the above listed mutants for PYY(12-36), e.g., [Lys²⁵]PPY(11-36) (Amino acid sequence=DASEELNRYYASLKHYLNLVTRQRY (SEQ ID NO: 204)) would result from combining the mutations from SEQ ID NO: 36 with SEQ ID NO: 174.

### Point Mutations of PYY(10-36)

| PEPTIDE | SEQUENCE |
|---|---|
| PYY(10-36) | EDASPEELNRYYASLRHYLNLVTRQRY (SEQ ID NO: 176) |
| [Asp¹⁰]PYY(10-36) | DDASPEELNRYYASLRHYLNLVTRQRY (SEQ ID NO: 177) |

Also included as PYY(10-36) mutations are polypeptide variations (amino acid sequence variations) resulting from the combination of either of these two mutants with any of the above listed mutants for PYY(25-36), and/or any of the above listed mutants for PYY(11-36), e.g., [Lys²⁵]PPY(10-36) (Amino acid sequence=EDASEELNRYYASLKHYLNLVTRQRY (SEQ ID NO: 205)) would result from combining the mutations from SEQ ID NO: 36 with SEQ ID NO: 176.

### Point Mutations of PYY(9-36)

| PEPTIDE | SEQUENCE |
|---|---|
| PYY(9-36) | GEDASPEELNRYYASLRHYLNLVTRQRY (SEQ ID NO: 178) |

Also included as PYY(9-36) mutations are polypeptide variations (amino acid sequence variations) resulting from the combination of this PPY(9-36) mutant with any of the above listed mutants for PYY(25-36), and/or any of the above listed mutants for PYY(10-36), e.g., [Lys²⁵]PPY(9-36) (Amino acid sequence=GEDASPEELNRYYASLKHYLNLVTRQRY (SEQ ID NO: 206)) would result from combining the mutations from SEQ ID NO: 36 with SEQ ID NO: 178.

### Potin Mutations of PYY(8-36)

| PEPTIDE | SEQUENCE |
|---|---|
| PYY(8-36) | PGEDASPEELNRYYASLRHYLNLVTRQRY (SEQ ID NO: 179) |

Also included as PYY(8-36) mutations are polypeptide variations (amino acid sequence variations) resulting from the combination of this PPY(8-36) mutant with any of the above listed mutants for PYY(25-36), and/or any of the above listed mutants for PYY(9-36), e.g., [Lys²⁵]PPY(8-36) (Amino acid sequence= SEQ ID NO: 207)) would result from combining the mutations from SEQ ID NO: 36 with SEQ ID NO: 179.

### Point Mutations of PYY(7-36)

| PEPTIDE | SEQUENCE |
|---|---|
| PYY(7-36) | APGEDASPEELNRYYASLRHYLNLVTRQRY (SEQ ID NO: 180) |
| [Ser⁹]PYY(7-36) | SPGEDASPEELNRYYASLRHYLNLVTRQRY (SEQ ID NO: 181) |

Also included as PYY(7-36) mutations are polypeptide variations (amino acid sequence variations) resulting from the combination of either of these two mutants with any of the above listed mutants for PYY(25-36), and/or any of the above listed mutants for PYY(8-36), e.g., [Lys²⁵]PPY(7-36) (Amino acid sequence=APGEDASEELNRYYASLKHYLNLVTRQRY (SEQ ID NO: 208)) would result from combining the mutations from SEQ ID NO: 36 with SEQ ID NO: 180.

### Point Mutations of PYY(6-36)

| PEPTIDE | SEQUENCE |
|---|---|
| PYY(6-36) | EAPGEDASPEELNRYYASLRHYLNLVTRQRY (SEQ ID NO: 182) |
| [Asp⁶]PYY(6-36) | DAPGEDASPEELNRYYASLRHYLNLVTRQRY (SEQ ID NO: 183) |

Also included as PYY(6-36) mutations are polypeptide variations (amino acid sequence variations) resulting from the combination of either of these two mutants with any of the above listed mutants for PYY(25-36), and/or any of the above listed mutants for PYY(7-36), e.g., [Lys²⁵]PPY(6-36) (Amino acid sequence=EAPGEDASEELNRYYASLKHYLNLVTRQRY (SEQ ID NO: 209)) would result from combining the mutations from SEQ ID NO: 36 with SEQ ID NO: 182.

### Point Mutations of PYY(5-36)

| PEPTIDE | SEQUENCE |
|---|---|
| PYY(5-36) | PEAPGEDASPEELNRYYASLRHYLNLVTRQRY (SEQ ID NO: 184) |

Also included as PYY(5-36) mutations are polypeptide variations (amino acid sequence variations) resulting from the combination of this PPY(5-36) mutant with any of the above listed mutants for PYY(25-36), and/or any of the above listed mutants for PYY(6-36), e.g., [Lys²⁵]PPY(5-36) (Amino acid sequence=PEAPGEDASPEELNRYYASLKHYLNLVTRQRY (SEQ ID NO: 210)) would result from combining the mutations from SEQ ID NO: 36 with SEQ ID NO: 184.

### Point Mutations of PYY(4-36)

| PEPTIDE | SEQUENCE |
|---|---|
| PYY(4-26) | KPEAPGEDASPEELNRYYASLRHYLNLVTRQRY (SEQ ID NO: 185) |
| [Arg⁴]PYY(4-36) | RPEAPGEDASPEELNRYYASLRHYLNLVTRQRY (SEQ ID NO: 186) |
| [Gln⁴]PYY(4-36) | QPEAPGEDASPEELNRYYASLRHYLNLVTRQRY (SEQ ID NO: 187) |
| [Asn⁴]PYY(4-36) | NPEAPGEDASPEELNRYYASLRHYLNLVTRQRY (SEQ ID NO: 188) |

Also included as PYY(4-36) mutations are polypeptide variations (amino acid sequence variations) resulting from the combination of any of these four mutants with any of the above listed mutants for PYY(25-36), and/or any of the above listed mutants for PYY(5-36), e.g., [Lys²⁵]PPY(4-36) (Amino acid sequence=KPEAPGEDASEELNRYYASLKHYLNLVTRQRY (SEQ ID NO: 211)) would result from combining the mutations from SEQ ID NO: 36 with SEQ ID NO: 185.

### Point Mutations of PYY(3-36)

| PEPTIDE | SEQUENCE |
|---|---|
| PYY(3-36) | IKPEAPGEDASPEELNRYYASLRHYLNLVTRQRY (SEQ ID NO: 334) |
| [Leu³]PYY(3-36) | LKPEAPGEDASPEELNRYYASLRHYLNLVTRQRY (SEQ ID NO: 189) |
| [Val³] PYY(3-36) | VKPEAPGEDASPEELNRYYASLRHYLNLVTRQRY (SEQ ID NO: 190) |

Also included as PYY(3-36) mutations are polypeptide variations (amino acid sequence variations) resulting from the combination of any of these three mutants with any of the above listed mutants for PYY(25-36), and/or any of the above listed mutants for PYY(4-36), e.g., [Lys²⁵]PPY(3-36) (Amino acid sequence=IKPEAPGEDASEELNRYYASLKHYLNLVTRQRY (SEQ ID NO: 212)) would result from combining the mutations from SEQ ID NO: 36 with SEQ ID NO: 1.

Also contemplated are PYY agonists (NPY analogs) having the formula:

X-Q-R₁₉ -R₂₀ -R₂₁ -R₂₂ -R₂₃ -Leu-R₂₅ -R₂₆ -R₂₇ -R₂₈ -R₂₉ -R₃₀ -R₃₁ -R₃₂ -Arg-R₃₄ -Arg-R₃₆ -Y

wherein X is H or C^{a} Me or N^{a} Me or desamino or an acyl group having 7 carbon atoms or less; Q is R₁₇ -R₁₈, R₁₈ or desQ; R₁₇ is Met, Arg, Nle, Nva, Leu, Ala or D-Ala; R₁₈ is Ala, Ser, Ile, D-Ala, D-Ser or D-Ile; R₁₉ is Arg, Lys or Gln; R₂₀ is Tyr or Phe; R₂₁ is Tyr, Glu, His or Ala; R₂₂ is Ser, Ala, Thr, Asn or Asp; R₂₃ is Ala, Asp, Glu, Gln, Asn or Ser; R₂₅ is Arg or Gln; R₂₆ is His, Arg or Gln; R₂₇ is Phe or Tyr; R₂₈ is Ile, Leu, Val or Arg; R₂₉ is Asn or Ile; R₃₀ is Leu, Met, Thr or Val; R₃₁ is Ile, Val or Leu; R₃₂ is Thr or Phe; R₃₄ is Gln, Pro or His; R₃₆ is Phe or Tyr; and Y is NH₂ or OH; provided that when Q is R₁₈, then at least one of R₂₇ and R₃₆ is Phe. Analogs of NPY have the following applications: potent postsynaptic treatment of hypertension and cardiogenic shock, the treatment of acute cardiovascular circulatory failure, and the elevation of intracellular calcium. See U.S. Patent No. 5,026,685.

Certain preferred NPY analogs have the formula: X-R₁₈-Arg-Tyr-Tyr-R₂₂-R₂₃-Leu-Arg-His-Tyr-R₂₈-Asn-Leu-R₃₁-Thr-Arg-Gln-Arg-Tyr-NH₂ (SEQ ID NO: 342), wherein X is H or C^{a} Me or N^{a} Me or desamino or an acyl group having 7 carbon atoms or less; R₁₈ is Ala or Ser; R₂₂ is Ser or Ala; R₂₃ is Ala or Ser; R₂₇ is Phe or Tyr; R₂₈ is Ile or Leu; R₃₁ is Ile or Val; and R₃₆ is Phe or Tyr; provided that at least one of R₂₇ and R₃₆ is Phe. See U.S. Patent No. 5,026,685.

Other contemplated NPY analogs have the formula:

X-R₁₇ -R₁₈ -Arg-Tyr-Tyr-Ser-Ala-Leu-Arg-His-R₂₇-Ile-Asn-Leu-Ile-Thr-Arg-Gln-Arg-R₃₆-NH₂, (SEQ ID NO: 343)

wherein R₁₇ is Arg or Leu and R₁₈ is Ser or Ala or Ile; and wherein X, R₂₇ and R₃₆ are as previously indicated.

Still other preferred NPY analogs have the formula: wherein X is desamino or C^{a} Me or N^{a} Me and wherein R₁₈, R₂₅, R₂₇ and R₃₆ are as previously indicated.

Examples of such NPY agonists include:
pNPY (17-36) having the formula:
The peptide hNPY (17-36) having the formula:
The peptide [Phe²⁷]-NPY (18-36) having the formula:
The peptide [Ac-D-Ala¹⁷]-NPY (17-36) having the formula:
The peptide NPY (19-36) having the formula:
The peptide [Nle¹⁷]-NPY (17-36) having the formula:
The peptide [D-Ser¹⁸]-NPY (18-36) having the formula:
The peptide [Ala¹⁷, His²¹]-NPY (17-36) having the formula:
The peptide [D-Ile¹⁸]-NPY (18-36) having the formula:
The peptide [Ac-Arg¹⁷]-NPY (17-36) having the formula:
The peptide [Gln¹⁹]-NPY (19-36) having the formula:
The peptide [Phe²⁰]-NpY (18-36) having the formula:
The peptide [C^{a} MeLeu¹⁷]-pNPY (17-36) having the formula:
The peptide [N^{a} MeLeu¹⁷]-pNPY (17-36) having the formula:
The peptide [desamino Ala¹⁸]-NpY (18-36) having the formula:
The peptide [For-Ala¹⁸, Glu²³, Arg²⁶]-NPY (18-36) having the formula:
The peptide [Nva¹⁷, Ala²¹, Leu²⁸]-NPY (17-36) having the formula:
The peptide [Thr²², Gln²³]-NPY (18-36) having the formula:
The peptide [desamino Leu¹⁷, Asn²³, Val³⁰]-NPY (17-36) having the formula:
The peptide [Asp²², Ser²³, Thr³⁰]-NPY (18-36) having the formula:
The peptide [Gln²⁵, Leu³¹, Pro³⁴]-NPY (18-36) having the formula:
The peptide [Gln² Phe³⁶]-NPY (17-36) having the formula:
The peptide [Phe³⁶]-pPYY (19-36) having the formula:
The peptide pPYY (18-36) having the formula:
The peptide [Ac-Ser¹⁸, Phe²⁷]-pPYY (I8-36) having the formula:
The peptide [Nle¹⁷, Asn²², Phe²⁷]-NPY (17-36) having the formula:
The peptide [D-Ala¹⁸, Glu²¹, His³⁴]-NPY (18-36) having the formula:
The peptide [Bz-Leu¹⁷, Pro³⁴, Phe³⁶]-pNPY (17-36) having the formula:
The peptide [Lys¹⁹, Phe²⁷, Val²⁸]-NpY (18-36) having the formula:
The peptide [D-Ala¹⁷, Val²⁸, Phe³²]-NPY (17-36) having the formula:
The peptide [C^{a} MeSer¹⁸, Met³⁰, Phe³⁶]-NPY (18-36) having the formula:
The peptide [Arg¹⁷, Ile¹⁸, Phe^{27,36}]-NPY (17-36) having the formula:
The peptide [Ser¹⁸, Phe²⁷]-pNPY (17-36) having the formula:
The peptide [N^{a} Melle¹⁸, Gln²⁵, Phe²⁷]-NPY (18-36) having the formula:
The peptide [D-Ser¹⁸, Phe³⁶]-NPY (18-36) having the formula:
The peptide [Asp²³, Arg²⁶]hNPY (17-36) having the formula:
The peptide [Glu²³, Ile²⁹]-NPY (18-36) having the formula:
The peptide [D-Ala¹⁷]-NPY(17-36)-OH having the formula:

   D-Ala-Ala-Arg-Tyr-Tyr-Ser-Ala-Leu-Arg-His-Tyr-Ile-Asn-Leu-Ile-Thr-Arg-Gln-Arg-Tyr-OH (SEQ ID NO: 254).

Other peptide YY agonists have the formula: 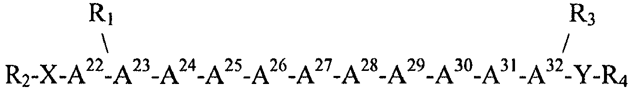 wherein:
X is a chain of 0-5 amino acids, inclusive, the N-terminal one of which is bonded to R₁ and R₂
Y is a chain of 0-4 amino acids, inclusive, the C-terminal one of which is bonded to R₃ and R₄
R₁ is H, C₁ -C₂ alkyl (e.g., methyl), C₆ -C₁₈ aryl (e.g., phenyl, napthaleneacetyl), C₁ --C₁₂ acyl (e.g., formyl, acetyl, and myristoyl), C₇ -C₁₈ aralkyl (e.g., benzyl), or C₇ -C₁₈ alkaryl (e.g., p-methylphenyl);
R₂ is H, C₁ -C₁₂ alkyl (e.g., methyl), C₆ -C₁₈ aryl (e.g., phenyl, naphthaleneacetyl), C₁ -C₁₂ acyl (e.g., formyl, acetyl, and myristoyl), C₇ -C₁₈ aralkyl (e.g., benzyl), or C₇ -C₁₈ alkaryl (e.g., p-methylphenyl);
A²² is an aromatic amino acid, Ala, Aib, Anb, N-Me-Ala, or is deleted;
A²³ is Ser, Thr, Ala, N-Me-Ser, N-Me-Thr, N-Me-Ala, or is deleted;
A²⁴ is Leu, lie, Vat, Trp, Gly, Aib, Anb, N-Me-Leu, or is deleted;
A²⁵ is Arg, Lys, homo-Arg, diethyl-homo-Arg, Lys-ε-NH-R (where R is H, a branched or straight chain C₁ -C₁₀ alkyl group, or an aryl group), Orn, or is deleted;
A²⁶ is His, Thr, 3-Me-His, 1-Me-His, β-pyrozolylalanine, N-Me-His, Arg, Lys, homo-Arg, diethyl-homo-Arg, Lys-ε-NH-R (where R is H, a branched or straight chain C₁ -C₁₀ alkyl group, or an aryl group), Orn, or is deleted;
A²⁷ is an aromatic amino acid other than Tyr;
A²⁸ is Leu, Ile, Vat, Trp, Aib, Aib, Anb, or N-Me-Leu;
A²⁹ is Asn, Ala, Gln, Gly, Trp, or N-Me-Asn;
A³⁰ is Leu, Ile, Val, Trp, Aib, Anb, or N-Me-Leu;
A³¹ is Vat, Ile, Trp, Aib, Anb, or N-Me-Val;
A³² is Thr, Ser, N-Me-Set, or N-Me-Thr;
R₃ is H, C₁ -C₁₂ alkyl (e.g., methyl), C₆ -C₁₈ aryl (e.g., phenyl, naphthaleneacetyl), C₁ -C₁₂ acyl (e.g., formyl, acetyl, and myristoyl), C₇ -C₁₈ aralkyl (e.g., benzyl), or C₇ -C₁₈ alkaryl (e.g., p-methylphenyl);
R₄ is H, C₁ -C₁₂ alkyl (e.g., methyl), C₆ -C₁₈ aryl (e.g., phenyl, naphthaleneacetyl), C₁ -C₁₂ acyl (e.g., formyl, acetyl, and myristoyl), C₇ -C₁₈ aralkyl (e.g., benzyl), or C₇ -C₁₈ alkaryl (e.g., p-methylphenyl), or a pharmaceutically acceptable salt thereof. See U.S. Patent No. 5,574,010.

Particularly preferred agonists of this formula include: N-α-Ala-Ser-Leu-Arg-His-Trp-Leu-Asn-Leu-Val-Thr-Arg-Gln-Arg-Tyr-NH₂ (SEQ ID NO: 255).

Other peptide YY agonists have the formula: 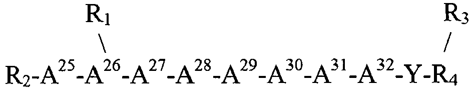
the N-terminal amino acid bonds to R₁ and R₂;
Y is a chain of 0-4 amino acids, inclusive the C-terminal one of which bonds to R3 and R4;
R₁ is H, C₁ -C₁₂ alkyl, C₆ -C₁₈ aryl, C₁ -C₁₂ acyl, C₇ -C₁₈ aralkyl, or C₇ -C₁₈ alkaryl;
R₂ is H, C₁ -C₁₂ alkyl, C₆ -C₁₈ aryl, C₁ -C₁₂ acyl, C₇ -C₁₈ aralkyl, or C₇ -C₁₈ alkaryl;
A²⁵ is Arg, Lys, homo-Arg, diethyl-homo-Arg, Lys-ε-NH-R (where R is H, a branched or straight chain C₁ -C₁₀ alkyl group, or an aryl group), Orn, or is deleted;
A²⁶ is Ala, His, Thr, 3-Me-His, 1-Me-His, β-pyrozolylalanine, N-Me-His, Arg, Lys, homo-Arg, diethyl-homo-Arg, Lys-ε-NH-R (where R is H, a branched or straight chain C₁ -C₁₀ alkyl group, or an aryl group), Orn or is deleted;
A²⁷ is an aromatic amino acid;
A²⁸ is Leu, Ile, Val, Trp, Aib, Anb, or N-Me-Leu;
A²⁹ is Asn, Ala, Gln, Gly, Trp, or N-Me-Asn;
A³⁰ is Leu, Ile, Val, Trp, Aib, Anb, or N-Me-Leu;
A³¹ is Val, Ile, Trp, Aib, Anb, or N-Me-Val;
A³² is Thr, Set, N-Me-Set, or N-Me-Thr or D-Trp;
R₃ is H, C₁-C₁₂ alkyl, C₆ -C₁₈ aryl, C₁ -C₁₂ acyl, C₇ -C₁₈ aralkyl, or C₇ -C₁₈ alkaryl; and
R₄ is H, C₁ -C₁₂ alkyl, C₆ -C₁₈ aryl, C₁ -C₁₂ acyl, C₇ -C₁₈ aralkyl, or C₇ -C₁₈ alkaryl, or a pharmaceutically acceptable salt thereof. Note that, unless indicated otherwise, for all peptide YY agonists described herein, each amino acid residue, e.g., Leu and A¹, represents the structure of NH--C(R)H--CO--, in which R is the side chain. Lines between amino acid residues represent peptide bonds which join the amino acids. Also, where the amino acid residue is optically active, it is the L-form configuration that is intended unless D-form is expressly designated.

Other PYY agonists have the formula: 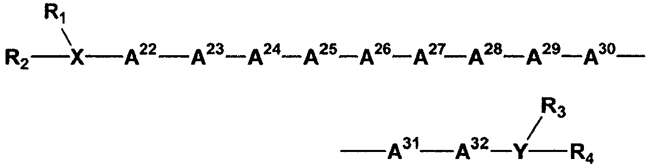 wherein:
X is a chain of 0-5 amino acids, inclusive, the N-terminal one of which is bonded to R₁ and R₂;
Y is a chain of 0-4 amino acids, inclusive, the C-terminal one of which is bonded to R₃ and R₄;
R₁ is H, C₁-C₁₂ alkyl (e.g. methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl);
R₂ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl);
A²² is an aromatic amino acid, Ala, Aib, Anb, N-Me-Ala, or is deleted;
A²³ is Ser, Thr, Ala, Aib, N-Me-Ser, N-Me-Thr, N Me-Ala, or is deleted;
A²⁴ is leu, Ile, Val, Trp, Gly, Nle, Nva, Aib, Anb, N-Me-Leu, or is deleted;
A²⁵ is Arg, Lys, homo-Arg, diethyl-homo-Arg, Lye-e-NH-R (where R is H, a branched or straight chain C₁-C₁₀ alkyl group, or an aryl group), Orn, or is deleted;
A²⁶ is Ala, His, Thr, 3-Me-His, 1-Me-His, β-pyrozolylalanine, N-Me-His, Arg, Lys, homo-Arg, diethyl-homo-Arg, Lys-ε-NH-R (where R is H, a branched or straight chain C₁-C₁₀ alkyl groups or an aryl group), Orn, or is deleted;
A²⁷ is an aromatic amino acid other than Tyr;
A²⁸ is Leu, Ile, Val, Trp, Nle, Nva, Aib, Anb, or N-Me-Leu;
A²⁹ is Asn, Ala, Gin, Gly, Trp, or N-Me-Asn;
A³⁰ is Leu, Ile, Val, Trp, Nle, Nva, Aib, Anb, or N-Me-Leu;
A³¹ is Val, Leu, Ile, Trp, Nle, Nva, Aib, Anb, or N-Me-Val;
A³² is Thr, Ser, N-Me-Ser, N-Me-Thr, or D-Trp;
R₃ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl); and
R₄ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl), or a pharmaceutically acceptable salt thereof.

In preferred embodiments, A²⁷ is Phe, Nal, Bip, Pcp, Tic, Trp, Bth, Thi, or Dip.

In preferred embodiments X is A¹⁷-A¹⁸-A¹⁹-A²⁰-A²¹ wherein
A¹⁷ is Cys, Leu, Ile, Val, Nle, Nva, Aib, Anb, or N-Me-Leu;
A¹⁸ is Cys, Ser, Thr, N-Me-Ser, or N-Me-Thr;
A¹⁹ is Arg, Lys, homo-Arg, diethyl-homo-Arg, Lys-ε-NH-R (where R is H, a branched or straight chain C₁-C₁₀ alkyl group, or C₆-C₁₈ aryl group), Cys, or Orn;
A²⁰ is an aromatic amino acid, or Cys; and
A²¹ is an aromatic amino acid, Cys, or a pharmaceutically acceptable salt thereof. In yet other preferred embodiments, Y is A³³-A³⁴-A³⁵-A³⁶ wherein
A³³ is Arg, Lys, homo-Arg, diethyl-homo-Arg, Lys-ε-NH-R (where R is H, a branched or straight chain C₁-C₁₀ alkyl group, or an aryl group), Cys, or Orn;
A³⁴ is Cys, Gln, Asn, Ala, Gly, N-Me-Cln, Aib, or Anb;
A³⁵ is Arg, Lys, homo-Arg, diethyl-homo-Arg, Lys-ε-NH-R (where R is H, a branched or straight chain C₁-C₁₀ alkyl group, or C₆-C₁₈ aryl group), Cys, or Orn; and
A³⁶ is an aromatic amino acid, Cys or a pharmaceutically acceptable salt thereof. See U.S. Patent No. 5,604,203.

Particular embodiments include compounds has the formula: N-α-Ac-Ala-Ser-Leu-Arg-His-Phe-Leu-Asn-Leu-Val-Thr-Arg-Gin-Arg-Tyr-NH₂ (SEQ. ID. NO: 325), H-Ala-Ser-Leu-Arg-His-Phe-Leu-Asn-Leu-Val-Thr-Arg-Gln-Arg-Tyr-NH₂ (SEQ. ID. NO: 326), N-α-Ac-Ala-Ser-Leu-Arg-Thr-Arg-Gin-Arg-Tyr-NH₂ (SEQ. ID. NO: 327), N-α-Ac-Ala-Ser-Leu-Arg-His-Thi-Leu-Asn-Leu-Val-Thr-Arg-Gin-Arg-Tyr-NH₂ (SEQ. ID. NO: 328), N-α-Ac-Tyr-Ser-Leu-Arg-His-Phe-Leu-Asn-Leu-Val-Thr-Arg-Gin-Arg-Tyr-NH₂ (SEQ. ID. NO: 329) or a pharmaceutically acceptable salt thereof.

Other PYY agonists have the formula: 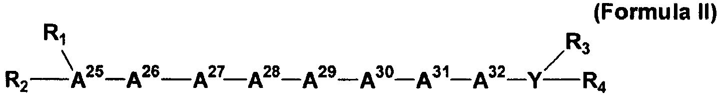
wherein the N-terminal amino acid is bounded to R₁ and R₂; Y is a chain of 0-4 amino acids, inclusive the C-terminal one of which is bonded to R₃ and R₄;
R₁ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl);
R₂ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl);
A²⁵ is Arg, Lys, homo-Arg, diethyl-homo-Arg, Lys-ε-NH-R (where R is H, a branched or straight chain C₁-C₁₀ alkyl group, or an aryl group), Orn, or is deleted;
A²⁶ is Ala, His, Thr, 3-Me-His, 1-Me-His, P-pyrozolylalanine, N-Me-His, Arg, Lys, homo-Arg, diethyl-homo-Arg, Lys-ε-NH-R (where R is H, a branched or straight chain C₁-C₁₀ alkyl groups or an aryl group), Orn, or is deleted;
A²⁷ is an aromatic amino acid;
A²⁸ is Leu, Ile, Val, Trp, Nle, Nva, Aib, Anb, or N-Me-Leu;
A²⁹ is Asn, Ala, Gin, Gly, Trp, or N-Me-Asn;
A³⁰ is Leu, Ile, Val, Trp, Nle, Nva, Aib, Anb, or N-Me-Leu;
A³¹ is Val, Ile, Trp, Nle, Nva, Aib, Anb, or N-Me-Val;
A³² is Thr, Ser, N-Me-Ser, N-Me-Thr, or D-Trp;
R₃ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl); and
R₄ is H, C₁-C₁₂ alkyl (e.g., methyl), C₆-C₁₈ aryl (e.g., phenyl, naphthaleneacetyl), C₁-C₁₂ acyl (e.g., formyl, acetyl, and myristoyl), C₇-C₁₈ aralkyl (e.g., benzyl), or C₇-C₁₈ alkaryl (e.g., p-methylphenyl), or a pharmaceutically acceptable salt thereof. See U.S. Patent No. 5,604,203.

In particular embodiments, A²⁷ is Phe, Nal, Bip, Pcp, Tic, Trp, Bth, Thi, or Dip.

In particular embodiments X is A³³-A³⁴-A³⁵-A³⁶ wherein
A³³ is Arg, Lys, homo-Arg, diethyl-homo-Arg, Lys-ε-NH-R (where R is H, a branched or straight chain C₁-C₁₀ alkyl group, or C₆-C₁₈ aryl group), Cys, or Orn;
A³⁴ is Gln, Asn, Ala, Gly, N-Me-Gin, Aib, Cys, or Anb;
A³⁵ is Arg, Lys, homo-Arg, diethyl-homo-Arg, Lys-ε-NH-R (where R is H, a branched or straight chain C₁-C₁₀ alkyl group, or C₆-C₁₈ aryl group), Cys, or Orn; and
A³⁶ is an aromatic amino acid, Cys, or a pharmaceutically acceptable salt thereof.

Preferably, the compound has the formula: N-α-Ac-Arg-His-Phe-Leu-Asn-Leu-Val-Thr-Arg-Gln-Arg-Tyr-NH₂ (SEQ. ID. NO: 324).

### Examplary PYY agonists include:

| YPAKEAPGEDASPEELSTYYASLR [im-DNP-His²⁶] | (SEQ ID NO: 256) |
|---|---|
| YLNLVTRZRY-NH₂ | |
| PYY(22-36) | |
| ASLRHYLNLVTRQRY-NH₂ | (SEQ ID NO: 257) |
| [Ala³²]PYY | |
| ASLRHYLNLV[Ala]RQRY-NH₂ | (SEQ ID NO: 258) |
| [Ala^{23,32}]PYY | |
| A[Ala]LRHYLNLV[Ala]RQRY-NN₂ | (SEQ ID NO: 259) |
| [Glu²⁸]PYY(22-36) | |
| ASLRHY[Glu]NLVTRQRY-NH₂ | (SEQ ID NO: 260) |
| N-α-Ac-PYY(22-36) | |
| N-α-Ac-ASLRHYLNLVTRORY-NH₂ | (SEQ ID NO: 261) |
| N-α-Ac[p.CL.Phe²⁶]PYY | |
| N-α-Ac-ASLR[p.CL.Phe²⁶]YLNLVTRQRY-NH₂ | (SEQ ID NO: 262) |
| N-α-Ac[Glu²⁸]PYY | |
| N-α-Ac-ASLRHY[Glu]NLVTRQRY-NH₂ | (SEQ ID NO: 263) |
| N-α-Ac[Phe²⁷]PYY | |
| N-α-Ac-ASLRH[Phe]ENLVTRQR[N-Me-Tyr]-NH₂ | (SEQ ID NO: 264) |
| N-α-Ac]8N-Me-Tyr³⁶]PYY | |
| N-α-Ac-ASLRHYENLVTR0R[N-Me-Tyr]-NH₂ | (SEQ ID NO: 265) |
| N-α-myristoyl-PYY(2214 36) | |
| N-α-myristoyl-ASLRHYLNLVTRQRY-NH₂ | (SEQ ID NO: 266) |
| N-α-naphthateneacetyl-PYY(22-36) | |
| N-α-naphthateneacetyl-ASLRHYLNLVTRQRY-NH₂ | (SEQ ID NO: 267) |
| N-α-Ac[Phe²⁷]PYY | |
| N-α-Ac-ASLRH[Phe]ENLVTR0R[N-Me-Tyr]-NH₂ | (SEQ ID NO: 268) |
| N-α-Ac-PYY (22-36) | |
| N-α-Ac-ASLRHYLNLVTRQRY-NH₂ | (SEQ ID NO: 269) |
| N-α-Ac-[Bth²⁷]PYY (22-36) | |
| N-α-Ac-ASLRH[Bth]LNLVTRQRY-NH₂ | (SEQ ID NO: 270) |
| N-α-Ac-[Bip²⁷]PYY (22-36) | (SEQ ID NO: 271) |
| N-α-Ac-ASLRH[Bth]LNLVTRQRY-NH₂ | (SEQ ID NO: 272) |
| N-α-Ac-[Nal²⁷]PYY (22-36) | |
| N-α-Ac-ASLRH[Nal]LNLVTRQRY-NH₂ | (SEQ ID NO: 273) |
| N-α-Ac-[Trp²⁷]PYY (22-36) | (SEQ ID NO: 274) |
| N-α-Ac-ASLRH[Trp]LNLVTRQRY-NH₂ | (SEQ ID NO: 275) |
| N-α-Ac-[Thi²⁷]PYY (22-36) | |
| N-α-Ac-ASLRN[Thi]LNLVTRQRY-NH₂ | (SEQ ID NO: 276) |
| N-α-Ac-[Tic²⁷]PYY (22-36) | |
| N-α-Ac-ASLRH[Tic]LNLVTRQRY-NH₂ | (SEQ ID NO: 277) |
| N-α-Ac-[Phe²⁷]PYY (25-36) | |
| N-α-Ac-H[Phe]LNLVTRQRY-NH₂ | (SEQ ID NO: 279) |
| N-α-Ac-[Phe²⁷]Thi²⁷]PYY (22-36) | |
| N-α-Ac-ASLRH[Phe]LNLVTRQR[Thi]-NH₂ | (SEQ ID NO: 280) |
| N-α-Ac-[Thz²⁶,Phe²⁷]PYY (22-36) | |
| N-α-Ac-ASLRH[Thz][Phe]LNLVTRQRY-NH₂ | (SEQ ID NO: 281) |
| N-α-Ac-[Phe²⁷]PYY (22-36) | |
| N-α-Ac-ASLRH[Thz][Phe]LNLVTRQRY-NH₂ | (SEQ ID NO: 282) |
| N-α-Ac-[Phe²⁷]PYY (22-36) | |
| N-α-Ac-[Phe]SLRN[Phe]LNLVTRQRY-NH₂. | (SEQ ID NO: 289) |
| N-α-Ac-[Tyr²²,Phe²⁷]PYY (22-36) | |
| N-α-Ac-[Tyr]SLRH[Phe]LNLVTRQRY-NH₂ | (SEQ ID NO: 290) |
| N-α-Ac-[Trp²⁸]PYY (22-36) | |
| N-α-Ac-ASLRHY[Trp]NLVTRQRY-NH₂ | (SEQ ID NO: 291) |
| N-α-Ac-[Trp²⁸]PYY (22-36) | |
| N-α-Ac-ASLRHYLN[Trp]VTRQRY-NH₂ | (SEQ ID NO: 292) |
| N-α-Ac-[Ala²⁶,Phe²⁷]PYY (22-36) | |
| N-α-Ac-ASLR[Ala][Phe]LNLVTRQRY-NH₂ | (SEQ ID NO: 293) |
| N-α-Ac-[Bth²⁷]PYY (22-36) | |
| N-α-Ac-ASLR[Bth]LNLVTRQRY-NH₂ | (SEQ ID NO: 294) |
| N-α-Ac-[Phe²⁷]PYY (22-36) | |
| N-α-Ac-ASLRH[Phe]LNLVTRQRY-NH₂ | (SEQ ID NO: 295) |
| N-α-Ac-[Phe^{27,36}]PYY (22-36) | |
| N-α-Ac-ASLRH[Phe]LNLVTRQR[Phe]-NH₂ | (SEQ ID NO: 296) |
| N-α-Ac-[Phe²⁷, D-Trp³²]PYY (22-36) | |
| N-α-Ac-ASLRH[Phe]LNLV[D-Trp]RQRY-NH₂ | (SEQ ID NO: 297) |

Other PYY agonists include neurophilic Y Y2 receptor specific peptides having the formula:

X1(-X2-X3-X4-X5-X6-X7-X8-X9-X10-X11-X12-X13-X14)ₙ-X15

wherein
X1 is NH, CH₃CO or one or two naturally occurring amino acids.
X2 is Leu, Ile or Val.
X3 is Arg, Lys or His.
X4 is His, Lys or Arg.
X5 is Tyr or Phe.
X6 is Leu, Ile or Val.
X7 is Asn or Gln.
X8 is Leu, Ile or Val.
X9 is Leu, Ile or Val.
X10 is Thr or Ser.
X11 is Arg, His or Lys.
X12 is Gln or Asn.
X13 is Arg, His or Lys.
X14 is Tyr or Phe.
X15 is COOH, NH₂ or one or two naturally occurring amino acids with the terminal amino acid being in the normal or carboxamide form; and
n is 1 to 5. See U.S. Patent No. 5,696,093.

Examplary agonists include:
CH₃CO-L-R-H-Y-L-N-L-L-T-R-Q-R-Y-NH₂ (SEQ ID NO: 298)
CH₃CO-L-R-H-Y-I-N-L-I-T-R-Q-R-Y-NH₂ (SEQ ID NO: 299)
NH₂-L-R-H-Y-L-N-L-L-T-R-Q-R-Y-NH₂ (SEQ ID NO: 300)
NH₂-L-R-H-Y-I-N-L-I-T-R-Q-R-Y-NH₂ (SEQ ID NO: 301)

Other PYY agonists have the formula:
N-α-R¹-[Nle^{24,28,30}, Trp²⁷, Nva³¹, ψ^{35/36}]PYY(22-36)-NH₂,
N-α-R¹-[Nle^{24,28}, Trp^{27,30}, Nva³¹, ψ^{35/36}]PYY(22-36)-NH₂,
N-α-R¹-[Nle^{24,28,30}, Phe²⁷, Nva³¹, ψ^{35/36}]PYY(22-36)-NH₂,
N-α-R¹-[Nle^{24,28}, Phe²⁷, Trp³⁰, Nva³¹, ψ^{35/36}]PYY(22-36)-NH₂,
N-α-R¹-[Trp³⁰, ψ^{35/36}]PYY(25-36)-NH₂,
N-α-R¹-[Trp³⁰]PYY(25-36)-NH₂,
N-α-R¹-[Nle^{24,28}, Trp³⁰, Nva³¹, ψ^{35/36}]PYY(22-36)-NH₂ and
N-α-R¹-[Nle²⁸, Trp³⁰, Nva³¹, ψ^{35/36}]PYY(22-36)-NH₂ or a pharmaceutically-acceptable salt thereof,
wherein R¹ is H, (C₁ -C₁₂)alkyl or (C₁ -C₁₂)acyl; and
ψ is a pseudopeptide bond selected from the group consisting of --CH₂ --NH- -, --CH₂ --S--, --CH₂ --CH₂ --, --CH₂ --O-- and --CH₂ --CO--. See U.S. Patent No. 6,046,162.

Particular compounds of the immediately foregoing group of compounds are where R¹ is acetyl and ψ is --CH₂ --NH--.

A particular group of compounds is selected from a group consisting of
N-α-Ac-[Nle^{24,28,30}, Trp²⁷, Nva³¹, ψ^{35/36}]PYY(22-36)-NH₂, (SEQ ID NO: 302)
N-α-Ac-[Nle^{24,28}, Trp^{27,30}, Nva³¹, ψ^{35/36}]PYY(22-36)-NH₂, (SEQ ID NO: 303)
N-α-Ac-[Nle^{24,28,30}, Phe²⁷, Nva³¹, ψ^{35/36}]PYY(22-36)-NH₂, (SEQ ID NO: 304)
N-α-Ac-[Nle^{24,28}, Phe²⁷, Trp³⁰, Nva³¹, ψ^{35/36}]PYY(22-36)-NH₂, (SEQ ID NO: 305)
N-α-Ac-[Trp³⁰, ψ^{35/36}]PYY(25-36)-NH₂, (SEQ ID NO: 306)
N-α-Ac-[Trp³⁰]PYY(25-36)-NH₂ (SEQ ID NO: 307) and
N-α-Ac-[Nle²⁸, Trp³⁰, Nva³¹, ψ^{35/36}]PYY(22-36)-NH₂, (SEQ ID NO: 308) or a pharmaceutically acceptable salt thereof.

Another particular compound has the formula N-α-Ac-[Nle^{24,28}, Trp³⁰, Nva.sup.³¹, ψ^{35/36}]PYY(22-36)-NH₂ (SEQ. ID. NO: 309) or a pharmaceutically acceptable salt thereof.

Another PYY agonist has the formula (A), 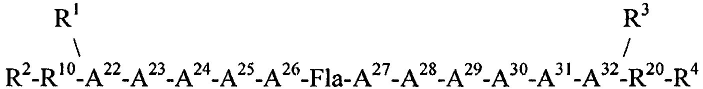 having one or two pseudopeptide bonds where each pseudopeptide bond is independently selected from the group consisting of --CH₂ --NH--, --CH₂ --S--,-CH₂ --CH₂ --, --CH₂ --O-- and -CH₂ --CO--; wherein:
R¹⁰ is a chain of 0-5 amino acids, inclusive, where the N-terminal amino acid is bonded to R¹ and R² by the side chain of the N-terminal amino acid or by the nitrogen of the amino group of the N-terminal amino acid;
R²⁰ is a chain of 0-4 amino acids, inclusive, where the C-terminal amino acid is bonded to R³ and R⁴ by the side chain of the C-terminal amino acid or by the carbon of the carboxyl group of the C-terminal amino acid;
R¹, R², R³ and R⁴ are each independently selected from the group consisting of H, (C₁ -C₁₂)alkyl, (C₆ -C₁₈)aryl, (C₁ -C₁₂)acyl, phenyl(C₁ -C₁₂)alkyl and ((C₁ - C₁₂)alkyl)₁₋₅ -phenyl;
A²² is an aromatic amino acid, Ala, Aib, Anb, N-Me-Ala or is deleted; A²³ is Ser, Thr, Ala, N-Me-Ser, N-Me-Thr, N-Me-Ala or is deleted;
A²⁴ is Leu, Ile, Nle, Val, Trp, Gly, Aib, Anb, N-Me-Leu or is deleted; A²⁵ is Arg, Lys, homo-Arg, diethyl-homo-Arg, Lys-p.epsilon.-NH-Z, Orn or is deleted;
A²⁶ is His, Thr, 3-Me-His, 1-Me-His, β-pyrazolylalanine, N-Me-His, Arg, Lys, homo-Arg, diethyl-homo-Arg, Lys-ε-NH-Z, Orn or is deleted;
A²⁸ is Leu, Ile, Nle, Val, Trp, Aib, Anb or N-Me-Leu;
A²⁹ is Asn, Ala, Gln, Gly, Trp or N-Me-Asn;
A³⁰ is Leu, Ile, Nle, Fla, Val, Trp, Aib, Anb or N-Me-Leu;
A³¹ is Val, Nva, Ile, Trp, Aib, Anb or N-Me-Val; and
A³² is Thr, Ser, N-Me-Ser or N-Me-Thr;
where Z for each occurrence is independently selected from the group consisting of H, (C₁ -C₁₀)alkyl and (C₆ -C₁₈)aryl; or a pharmaceutically acceptable salt thereof. See U.S. Patent No. 6,046,167.

A particular group of compounds of the immediately foregoing group of compounds is where R¹⁰ is A¹⁷ -A¹⁸ -A¹⁹ -A²⁰ -A²¹;
where A¹⁷ is Cys, Leu, Ile, Val, Nle, Nva, Aib, Anb or N-Me-Leu;
A¹⁸ is Cys, Ser, Thr, N-Me-Ser or N-Me-Thr;
A¹⁹ is Arg, Lys, homo-Arg, diethyl-homo-Arg, Lys-ε-NH-R.sup.5, Cys or Orn;
A²⁰ is an aromatic amino acid or Cys;
A²¹ is an aromatic amino acid or Cys;
R²⁰ is A³³ -A³⁴ -A³⁵ -A³⁶,
A³³ is Arg, Lys, homo-Arg, diethyl-homo-Arg, Lys-ε-NH-R⁵, Cys or Orn;
A³⁴ is Cys, Gin, Asn, Ala, Gly, N-Me-Gln, Aib or Anb;
A³⁵ is Arg, Lys, homo-Arg, diethyl-homo-Arg, Lys-ε-NH-R⁵, Cys or Orn; and
A³⁶ is an aromatic amino acid or Cys;
where R⁵ for each occurrence is independently selected from the group consisting of H₁ (C₁ -C₁₀)alkyl and (C₆ -C₁₈) aryl.

A particular group of compounds of the foregoing group of compounds are the compounds of the formula N-α-Ac-[Fla²⁷)]PYY(25-36)-NH₂ and N-α-Ac-[Fla²⁷ ]PYY(22-36)-NH₂ or a pharmaceutically acceptable salt thereof.

Another group of PYY agonist has the formula:
(I)

   (R¹ R²)-A¹-A²-A³-A⁴-A⁵-A⁶-A⁷-A⁸-A⁹-A¹⁰-R³⁰,
(II) 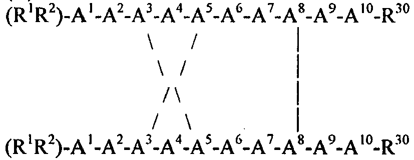
(III)

   (R¹ R²)-[A⁵-A⁶ -A⁷ -A⁸ -A⁹ -A¹⁰]ₘ R³⁰,
or a pharmaceutically acceptable salt thereof wherein
------represents an optional bond between the amino acids shown connected where each bond is independently selected from the group consisting of --S--Sonly when the amino acids connected are Cys-Cys, -CO-NH-, -CH₂ -NH- and 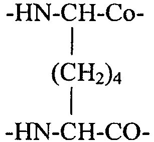 provided that when the optional bond is 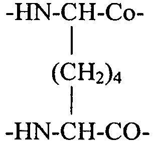 it replaces the two amino acids that the optional bond is attached to; q is 1-4; m is 1 to 4;
R³⁰ is OH or -O-R¹, provided that when A¹ to A⁷ are deleted then R³⁰ is also NH-R¹, where R³⁰ is attached to the carbon atom of the carboxyl of the C-terminal amino acid;
R¹ and R² for each occurrence are each independently selected from the group consisting of H, (C₁ -C₁₂)alkyl, (C₆ -C₁₈)aryl, (C₁ -C₁₂)acyl, phenyl(C₁ - C₁₂)alkyl and ((C₁ -C₁₂)alkyl)₁₋₅ -phenyl where R¹ and R² are attached to the nitrogen of the amine of the N-terminal amino acid;
A¹ is deleted or D- or L- of the following amino acids: Trp, Tyr, Fla, Bth, Nal, Tic, Tic-OH, Dip, Bip or optionally substituted Phe where the Phe is optionally substituted with one to five substituents selected from the group consisting of (C₁ - C₄)alkyl, halo, (C₁ -C₄)alkoxy, amino and nitro;
A² is deleted or D- or L- of the following amino acids: Ile, Val, Leu, Nle, Anb, Aib, Pro, Gln or Asn;
A³ is deleted or D- or L- of the following amino acids: Asn, Gln, Glu, Asp, Orn, Lys, Dpr or Cys;
A⁴ is deleted or D- or L- of the following amino acids: Ile, Val, Leu, Nle, Anb, Aib or Pro;
A⁵ is deleted or D- or L- of the following amino acids: Ile, Val, Leu, Nle, Anb, Aib, Pro, Glu, Asp, Orn, Lys, Dpr or Cys;
A⁶ is deleted or D- or L- of the following amino acids: Thr, Ser, Trp, Tyr, Fla, Bth, Nal, Tic, Tic-OH, Dip, Bip or optionally substituted Phe where the Phe is optionally substituted with one to five substituents selected from the group consisting of (C₁ -C₄)alkyl, halo, (C₁ -C₄)alkoxy, amino and nitro;
A⁷ is deleted or D- or L- of the following amino acids: Arg, Lys, homo-Arg, dialkyl-homo-Arg, Lys-ε-NH-R⁷ or Orn;
A⁸ is deleted or D- or L- of the following amino acids: Nva, Val, Ile, Leu, Nle, Anb, Aib, Pro, Gln, Asn, Glu, Asp, Orn, Lys, Dpr or Cys;
A⁹ is deleted or D- or L- of the following amino acids: Arg, Lys, homo-Arg, dialkyl-homo-Arg, Lys-ε-NH-R⁷ or Orn; and
A¹⁰ is deleted or D- or L- of the following amino acids: Tyr, Trp, Fla, Bth, Nal, Tic, Tic-OH, Dip, Bip, tyramine or optionally substituted Phe where the Phe is optionally substituted with one to five substituents selected from the group consisting of (C₁-C4)alkyl, halo, (C₁-C₄)alkoxy, amino and nitro, or the corresponding decarboxylated optionally substituted Phe;
where R⁷ for each occurrence is independently selected from the group consisting of H.sub.₁ (C₁-C₁₀)alkyl and (C₆-C₁₈) aryl, provided that not all of A₁ to A₁₀ are deleted at the same time. See U.S. Patent No. 6,046,167.

A particular group of compounds of the immediately foregoing group of compounds is
(SEQ ID NO: 310)
   H--Ile--Asn--Pro--Ile--Tyr--Arg--Leu--Arg--Tyr--OMe

(SEQ ID NO: 314)
   H-[Tyr-Arg-Leu-Arg-Tyr]₂ -Ome
or a pharmaceutically acceptable salt thereof.

PYY and PYY agonists may be produced by recombinant DNA technology by chemical synthesis, from natural sources, or by any combination thereof. PYY and PYY agonists may be modified by well known processes such as amidation, glycosylation, acylation (e.g. acetylation), sulfation, phosphylation, cyclization, lipidization and pegylation. Methods for lipidization with fatty acid derivatives of sulfhydryl-containing compounds are disclosed in U.S. Patent No. 5,936,092; U.S. Patent No. 6,093,692; and U.S. Patent No. 6,225,445. Fatty acid derivatives of sulfhydryl-containing PYY and PYY agonists comprising fatty acid-conjugated products with a disulfide linkage are employed for delivery of the PYY and PYY agonists to neuronal cells and tissues. This modification markedly increases the absorption of the compounds relative to the rate of absorption of the unconjugated compounds, as well as prolonging blood and tissue retention of the compounds. Moreover, the disulfide linkage in the conjugate is quite labile in the cells and thus facilitates intracellular release of the intact compounds from the fatty acid moieties.

Fatty acids, as constituents of phospholipids, make up the bulk of cell membranes. Due to their lipidic nature, fatty acids can easily partition into and interact with the cell membrane in a non-toxic way. Therefore, fatty acids represent potentially a useful carrier ligand for the delivery of proteins and peptides. Strategies that may use fatty acids in the delivery of proteins and peptides include the covalent modification of proteins and peptides and the use of fatty acid emulsions.

To prepare such conjugates, a sulfhydryl-containing PYY and PYY agonist is attached to a fatty acid derivative via a reversible, biodegradable disulfide bond. Such a conjugate is expected to bind to the apical side of a cell membrane, reach the basolateral membrane of the GI-epithelium as a result of membrane transport and turnover, and become released into interstitial fluid as the result of disulfide bond reduction.

Such lipidized PYY and PYY agonist compounds have the general formula 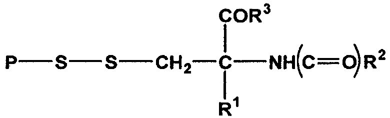 in which P is a residue derived from a PYY or PYY agonist; R¹ is hydrogen, lower alkyl or aryl; R² is a lipid-containing moiety and R³ is --OH, a lipid-containing moiety or an amino acid chain comprising one or 2 amino acids and terminating in -- CO₂H or -COR². See U.S. Patent No. 5,936,092. These conjugates are particularly useful for increasing the absorption and prolonging blood and tissue retention of PYY and PYY agonists.

Typical alkyl groups include C₁₋₆ alkyl groups including methyl, ethyl, propyl, isopropyl, butyl, isobutyl, sec-butyl, tert-butyl, pentyl, 2-pentyl, 3-pentyl, neopentyl, hexyl, 2-hexyl, 3-hexyl, 2-methyl-1-pentyl, 3-methyl-1-pentyl, 4-methyl-1-pentyl, and the like.

Preferred aryl groups are C₆₋₁₄ aryl groups and typically include phenyl, naphthyl, fluorenyl, phenanthryl, and anthracyl groups.

The term "lipid-containing moiety" refers to either a lipid group per se or a hydrocarbon-based group (in particular, one or more amino acids) comprising a lipid group. By the term "lipid group" is meant a hydrophobic substituent consisting of 4 to 26 carbon atoms, preferably 5 to 19 carbon atoms. Suitable lipid groups include, but are not limited to, the following: palmityl (C₁₅H₃₁,), oleyl (C₁₅H₂₉), stearyl (C₁₇H₃₅), cholate; and deoxycholate.

PCT Application No. WO 00/34236 describes drug-carrier conjugates and synthetic strategies for their production, as well as synthetic methods, intermediates, and final products useful for the uptake and release of biologically-active amino group containing compounds. Such lipidized PYY and PYY agonist compounds have general Formula I 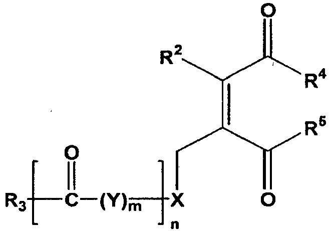
in which R² is selected from the group consisting of hydrogen, halo, alkyl, or aryl, wherein the alkyl or aryl groups are optionally substituted with one or more alkoxy, alkoxyalkyl, alkanoyl, nitro, cycloalkyl, alkenyl, alkynyl, alkanoyloxy, alkyl or halogen atoms;
R³ is a lipophilic group; one of R⁴ and R⁵ is a PYY or a PYY agonist and the other of R⁴ and R⁵ is OR⁶ where R⁶ is hydrogen, an alkali metal or a negative charge;
X is oxygen or sulfur;
Y is a bridging natural or unnatural amino acid; n is zero or 1; and m is an integer from zero to 10.

Typical alkyl groups include C₁₋₆ alkyl groups including methyl, ethyl, propyl, isopropyl, butyl, isobutyl, sec-butyl, tert-butyl, pentyl, 2-pentyl, 3-pentyl, neopentyl, hexyl, 2-hexyl, 3-hexyl, 2-methyl-l-pentyl, 3-methyl-1-pentyl, 4-methyl-1-pentyl, and the like.

Typical alkoxy groups include oxygen substituted by any of the alkyl groups mentioned above.

Typical alkoxyalkyl groups include any of the above alkyl groups substituted by an alkoxy group, such as methoxymethyl, ethoxymethyl, propoxymethyl, butoxymethyl, pentoxymethyl, hexoxymethyl, methoxyethyl, methoxypropyl, methoxybutyl, methoxypentyl, methoxyhexyl, and the like.

Preferred aryl groups are C₆₋₁₄ aryl groups and typically include phenyl, naphthyl, fluorenyl, phenanthryl, and anthracyl groups.

Typical alkoxy substituted aryl groups include the above aryl groups substituted by one or more of the above alkoxy groups, e.g., 3-methoxyphenyl, 2-ethoxyphenyl, and the like.

Typical alkyl substituted aryl groups include any of the above aryl groups substituted by any of the C₁₋₆ alkyl groups, including the group Ph(CH₂)n, where n is 1-6, for example, tolyl, o-, m-, and p-xylyl, ethylphenyl, 1-propylphenyl, 2-propylphenyl, 1-butylphenyl, 2-butylphenyl, t-butylphenyl, 1-pentylphenyl, 2-pentylphenyl, 3-pentylphenyl.

Typical alkenyl groups include C₂₋₆ alkenyl groups, e.g. ethenyl, 2-propenyl, isopropenyl, 2-butenyl, 3-butenyl, 4-pentenyl, 3-pentenyl, 2-pentenyl, 5-hexenyl, 4-hexenyl, 3-hexenyl, and 2-hexenyl groups.

Typical alkynyl groups include C₂₋₆ alkynyl groups e.g. enthynyl, 2-propenyl, 2-butynyl, 3-butynyl, 4-pentynyl, 3-pentynyl, 2-pentynyl, 5-hexynyl, 4hexynyl, 3-hexynyl, and 2-hexynyl groups.

Typical alkenyl or alkynyl substituted aryl groups include any of the above C₆₋₁₄ aryl groups substituted by any of the above C₂₋₆ alkenyl or C₂₋₆ alkynyl groups, e.g., ethenylphenyl, 1-propenylphenyl, 2-propenylphenyl, 1butenylphenyl, 2-butenylphenyl, 1-pentenylphenyl, 2-pentenylphenyl, 3-pentenylphenyl, 1-hexenylphenyl, 2-hexenylphenyl, 3-hexenylphenyl, ethynylphenyl, 1-propynylphenyl, 2-propynylphenyl, 1-butynylphenyl, 2-butynylphenyl, 1-pentynylphenyl, 2-pentynylphenyl, 3-pentynylphenyl, 1-hexynylphenyl, 2-hexynylphenyl, 3-hexynylphenyl groups.

Typical halo groups include fluorine, chlorine, bromine, and iodine.

Typical halo substituted alkyl groups include C₁₋₆ alkyl groups substituted by one or more fluorine, chlorine, bromine, or iodine atoms, e.g., fluoromethyl, difluoromethyl, trifluoromethyl, pentafluoroethyl, 1,1-difluoroethyl, and trichloromethyl groups.

Typical alkanoyl groups include C₁₋₅C(=O)- alkanoyl groups, e.g., acetyl, propionyl, butanoyl, pentanoyl, and hexanoyl groups, or by an arylalkanoyl group, e.g., a C₁₋₅C(=O)- alkanoyl group substituted by any of the above aryl groups.

Typical cycloalkyl groups include C₃₋₈ cycloalkyl groups including cyclopropyl, cyclobutyl, cyclopentyl, cyclohexyl, cycloheptyl and cyclooctyl groups.

The term "lipophilic group" as used herein refers to either a naturally occurring lipid per se, a hydrophobic branched or unbranched hydrocarbon comprising about 4 to about 26 carbon atoms, preferably about 5 to about 19 carbon atoms, a fatty acid or ester thereof, or a surfactant. Suitable lipophilic groups include, but are not limited to, long chain alkanoyl groups including: palmityl (C₁₅H₃₁), oleyl (C₁₅H₂₉), stearyl (C₁₇H₃₅), lauryl (C₁₁H23), cholyl, and myristyl (C₁₃H₂₇)

The term "natural or unnatural amino acid" as used herein refers to any of the 21 naturally occurring amino acids as well as D-form amino acids, blocked L-and D-form amino acids such as those blocked by amidation or acylation, substituted amino acids (e.g., those substituted with a sterically hindered alkyl group or a cycloalkyl group such as cyclopropyl or cyclobutyl) in which the substitution introduces a conformational restraint in the amino acid. The preferred naturally occurring amino acids for use in the present disclosure as amino acids or components of a peptide or protein are alanine, arginine, asparagine, aspartic acid, citrulline, cysteine, cystine, y-glutamic acid, glutamine, glycine, histidine, isoleucine, norleucine, leucine, lysine, methionine, ornithirie, phenylalanine, proline, hydroxyproline, serine, threonine, tryptophan, tyrosine, valine, γ -carboxyglutamate, or O-phosphoserine. The preferred non-naturally occurring amino acids for use in the present disclosure as amino acids or components of peptides or proteins are any of the β-amino acids, e.g., α-alanine, γ-amino butyric acid, γ-amino butyric acid, γ-(aminophenyl)butyric acid, α-amino isobutyric acid, ε-amino caproic acid, 7-amino heptanoic acid, amino benzoic acid, aminophenyl acetic acid, aminophenyl butyric acid, cysteine (ACM), methionine sulfone, phenylglycine, norvaline, ornithine, δ-ornithine, ρnitro-phenylalanine, 1,2,3,4-terahydroisoquinoline-3-carboxylic acid and thioproline.

Chemically modified derivatives of PYY and PYY agonists may provide additional advantages such as increased solubility, stability and circulating time of the polypeptide, or decreased immunogenicity (see U.S. Patent No. 4,179,337). Such modified derivatives include PYY and PYY agonists modified by pegylation. The terms "pegylated" and "pegylation" refer to the process of reacting a poly(alkylene glycol), preferably an activated poly(alkylene glycol), with a facilitator such as an amino acid, e.g. lysine, to form a covalent bond. Although "pegylation" is often carried out using poly(ethylene glycol) or derivatives thereof, such as methoxy poly(ethylene glycol), the term is not intended to be so limited here, but is intended to include any other useful poly(alkylene glycol), such as, for example poly(propylene glycol).

The chemical moieties for derivitization may also be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa.

As noted above, the polyethylene glycol may have a branched structure. Branched polyethylene glycols are described, for example, in U.S. Patent No. 5,643,575; Morpurgo et al., Appl. Biochem. Biotechnol. 56:59-72, 1996; Vorobjev et al., Nucleosides Nucleotides 18:2745-2750, 1999; and Caliceti et al., Bioconjug. Chem. 10:638-646, 1999.

The polyethylene glycol molecules (or other chemical moieties) should be attached to the polypeptides or proteins with consideration of effects on functional or antigenic domains of the polypeptides or proteins. There are a number of attachment methods available to those skilled in the art, e.g., EP 0 401 384 (coupling PEG to G-CSF), see also Malik et al., Exp. Hematol. 20:1028-1035, 1992 (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

As suggested above, polyethylene glycol may be attached to proteins and polypeptides via linkage to any of a number of amino acid residues. For example, polyethylene glycol can be linked to proteins and polypeptides via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) of the polypeptide or protein or to more than one type of amino acid residue (e.g., lysine, histidine, aspartic acid, glutamic acid, cysteine and combinations thereof) of the protein or polypeptide.

One may specifically desire proteins and polypeptides chemically modified at the N-terminus. Using polyethylene glycol as an illustration, one may select from a variety of polyethylene glycol molecules (by molecular weight, branching, etc.), the proportion of polyethylene glycol molecules to protein (or peptide) molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective proteins chemically modified at the N-terminus modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

As indicated above, pegylation of the proteins and polypeptides may be accomplished by any number of means. For example, polyethylene glycol may be attached to the protein or polypeptide either directly or by an intervening linker. Linkerless systems for attaching polyethylene glycol to proteins and polypeptides are described in Delgado et al., Crit. Rev. Thera. Drug Carrier Sys. 9:249-304, 1992; Francis et al., Intern. J. of Hematol. 68:1-18, 1998; U.S. Patent No. 4,002,531; U.S. Patent No. 5,349,052; WO 95/06058; and WO 98/32466.

One system for attaching polyethylene glycol directly to amino acid residues of proteins and polypeptides without an intervening linker employs tresylated MPEG, which is produced by the modification of monmethoxy polyethylene glycol (MPEG) using tresylchloride (ClSO₂CH₂CF₃). Upon reaction of the protein or polypeptide with tresylated MPEG, polyethylene glycol is directly attached to amine groups of the protein or polypeptide. Thus, the disclosure includes protein-polyethylene glycol conjugates produced by reacting proteins and polypeptides with a polyethylene glycol molecule having a 2,2,2-trifluoreothane sulphonyl group.

Polyethylene glycol can also be attached to proteins and polypeptides using a number of different intervening linkers. For example, U.S. Patent No. 5,612,460 discloses urethane linkers for connecting polyethylene glycol to proteins. Protein-polyethylene glycol conjugates wherein the polyethylene glycol is attached to the protein or polypeptide by a linker can also be produced by reaction of proteins or polypeptides with compounds such as MPEG-succinimidylsuccinate, MPEG activated with 1,1'-carbonyldiimidazole, MPEG-2,4,5-trichloropenylcarbonate, MPEG- p -nitrophenolcarbonate, and various MPEG-succinate derivatives. A number of additional polyethylene glycol derivatives and reaction chemistries for attaching polyethylene glycol to proteins and polypeptides are described in WO 98/32466.

The number of polyethylene glycol moieties attached to each protein or polypeptide (i.e., the degree of substitution) may also vary. For example, the pegylated proteins and polypeptides may be linked, on average, to 1, 2, 3, 4, 5, 6, 7, 8, 9, 10, 12, 15, 17, 20, or more polyethylene glycol molecules. Similarly, the average degree of substitution within ranges such as 1-3, 2-4, 3-5, 4-6, 5-7, 6-8, 7-9, 8-10, 9-11, 10-12, 11-13, 12-14, 13-15, 14-16, 15-17, 16-18, 17-19, or 18-20 polyethylene glycol moieties per protein or polypeptide molecule. Methods for determining the degree of substitution are discussed, for example, in Delgado et al., Crit. Rev. Thera. Drug Carrier Sys. 9:249-304, 1992.

The proteins and polypeptides containing substantially non-antigenic polymers, preferably poly(alkylene glycols) may be prepared, for example, as described in U.S. Patent No. 5,428,128; U.S. Patent No. 6,127,355; and U.S. Patent No. 5,880,131.

To effect covalent attachment of poly(ethylene glycol) (PEG) to a protein or polypeptide, the hydroxyl end groups of the PEG must first be converted into reactive functional groups. This process is frequently referred to as "activation" and the product is called "activated PEG." Methoxy poly(ethylene glycol) (mPEG), distally capped with a reactive functional group is often used. One such activated PEG is succinimidyl succinate derivative of PEG (SS-PEG). See also Abuchowski et al., Cancer Biochem. Biophys. 7:175-186, 1984; and U.S. Patent No. 5,122,614 which discloses poly(ethylene glycol)-N-succinimide carbonate and its preparation.

Alternative substantially non-antigenic polymers that may be employed in the practice of the present disclosure include materials such as dextran, polyvinyl pyrrolidones, polysaccharides, starches, polyvinyl alcohols, polyacrylamides, or other similar non-immunogenic polymers. Those of ordinary skill in the art will realize that the foregoing are merely illustrative and not intended to restrict the type of polymeric substances suitable for use herein.

In one aspect of the disclosure, the polymer is introduced into the peptide or protein molecule after being functionalized or activated for reaction and attachment to one or more amino acids. By activation, it is understood by those of ordinary skill in the art that the polymer is functionalized to include a desired reactive group. See, for example, U.S. Patent No. 4,179,337 and U.S. Patent No. 5,122,614. In this embodiment, the hydroxyl end groups of poly(alkylene glycols) are converted and activated into reactive functional groups.

In another aspect of the disclosure, the polymer is conjugated to a facilitator moiety prior to being introduced into the polypeptide or protein molecule. The facilitator moiety is preferably an amino acid such as lysine, however, non-amino acid moieties are also contemplated. Within the aspect, there are included multifunctionalized organic moieties such as alkyls or substituted alkyls. Such moieties can be prepared to have a nucleophilic functional group such as an amine and an electrophilic group such as an acid as well as a suitably functionalized region for conjugating with the desired polymer or polymers.

The facilitator moieties allow easier inclusion of a polymer into the peptide or protein molecule during synthesis. For example, poly(alkylene glycols) coupled to facilitator amino acids or amino acid residues in polypeptides or proteins by means of suitable coupling agents are illustrative. A useful review of a number of coupling agents known in the art appears in Dreborg et al., Critical Reviews in Therapeutic Drug Carrier Systems 6(4):315-165, 1990, see especially, pp. 317-320.

Pegylated PYY peptides and agonists can also be of the general formula 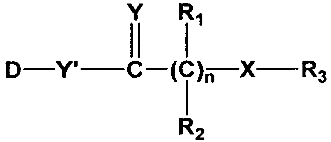 wherein:
D is a residue of a PYY peptide or agonist;
X is an electron withdrawing group;
Y and Y' are independently 0 or S;
(n) is zero (0) or a positive integer, preferably from 1 to about 12;
R₁ and R₂ are independently selected from the group consisting of H, C₁₋₆ alkyls, aryls, substituted aryls, aralkyls, heteroalkyls, substituted heteroalkyls, and substituted C₁₋₆ alkyls;
R₃ is a substantially non-antigenic polymer, C₁₋₁₂ straight or branched alkyl or substituted alkyl, C₅₋₈ cycloalkyl or substituted cycloalkyl, carboxyalkyl, carboalkoxy alkyl, dialkylaminoalkyl, phenylalkyl, phenylaryl or 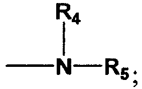 and
R₄ and R₅ are independently selected from the group consisting of H, C₁₋₆ alkyls, aryls, substituted aryls, aralkyls, heteroalkyls, substituted heteroalkyls and substituted C₁₋₆ alkyls or jointly form a cyclic C₅-C₇ ring. See U.S. Patent No. 6,127,355.

Typical alkyl groups include C₁₋₆ alkyl groups including methyl, ethyl, propyl, isopropyl, butyl, isobutyl, sec-butyl, tert-butyl, pentyl, 2-pentyl, 3-pentyl, neopentyl, hexyl, 2-hexyl, 3-hexyl, 2-methyl-l-pentyl, 3-methyl-1-pentyl, 4-methyl-1-pentyl, and the like.

Preferred aryl groups are C₆₋₁₄ aryl groups and typically include phenyl, naphthyl, fluorenyl, phenanthryl, and anthracyl groups.

Typical alkyl substituted aryl groups include any of the above aryl groups substituted by any of the C₁₋₆ alkyl groups, including the group Ph(CH₂)ₙ, where n is 1-6, for example, tolyl, o-, m-, and p-xylyl, ethylphenyl, 1-propylphenyl, 2-propylphenyl, 1-butylphenyl, 2-butylphenyl, t-butylphenyl, 1-pentylphenyl, 2-pentylphenyl, 3-pentylphenyl.

Typical cycloalkyl groups include C₃₋₈ cycloalkyl groups including cyclopropyl, cyclobutyl, cyclopentyl, cyclohexyl, cycloheptyl and cyclooctyl groups.

Typical electron withdrawing groups include O, NR₁, S, SO and SO₂, wherein R₁ is defined above.

### GLP-1 and Agonists Thereof

GLP-1 is produced from preproglucogon, which is a 160 amino acid polypeptide, in the central nervous system (CNS) and the intestine. It is released into the circulation in response to nutrient intake. Physiological actions of GLP-1 in man include stimulation of insulin release, suppression of gastric acid secretion and slowing of gastric emptying.

GLP-1 (1-37) (SEQ ID NO: 336) is the initial product of the processing of preproglucagon. GLP-1 (1-37) is amidated by post-translational processing to yield GLP-1 (1-36) NH (SEQ ID NO 337), or is enzymatically processed to give GLP-1 (7-37) (SEQ ID NO: 338). GLP-1 (7-37) can be amidated to give GLP-1 (7-36) amide (SEQ ID NO: 339). The sequences of human GLP-1 are given below:

A GLP-1 agonist is a peptide, small molecule, or chemical compound that preferentially binds to the GLP-1 receptor and stimulates the same biological activity as does GLP-1. In one embodiment, an agonist for the GLP-1 receptor binds to the receptor with an equal or greater affinity than GLP-1. In another embodiment, an agonist selectively binds the GLP-1 receptor, as compared to binding to another receptor. Exendin-4, which is a 39-amino acid peptide isolated from the salivary glands of the Gila monster (*Heloderma suspectum)* (Eng J et al J Biol Chem 267:7402-7405, 1992) is an example of an agonist at the GLP-1 receptor. Molecules derived from exendin-4 and that also have GLP-1 agonist activity are further examples of GLP-1 agonists. GLP-1 agonists include GLP-1 related peptides and peptides that result from natural or synthetic enzymatic or chemical processing of preproglucagon or of a GLP-1 peptide or a related peptide.

Any compound that is described as being a GLP-1 agonist may be used in the present invention, as may any compound that is tested for GLP-1 agonist activity, for example, as described above, and found to function as a GLP-1 agonist. A recombinant GLP-1 receptor suitable for use in screening is disclosed in WO93/19175. Many GLP-1 agonists are known and are described in the art. Examples of published patent specifications that disclose GLP-1 agonists are the following: WO2002/67918, WO2002/66479, WO2002/03978, WO2001/89554, WO2001/14386, WO2001/66135, WO2001/35988, WO2001/14368, WO2001/04156, WO2000/78333, WO2000/59887, WO2000/42026, EP 0955314, and WO99/43707. Examples of GLP-1 agonists are Arg34, Lys26(N-epsilon-(gamma-Glu(N-alpha-hexadecanoyl)))-GLP-1 (7-37), IP7-GLP-1 (7-37)OH.

GLP-1 or an agonist thereof may be administered according to the present invention peripherally at a dose of, for example, 0.1 nmoles or more per kg body weight of the subject, for example, 0.2 nmoles or more, for example, 0.4 nmoles or more, for example, 0.6 nmoles or more, for example, 0.8 nmoles or more, for example, 1.0 nmole or more, for example, 1.2 nmoles or more, for example, 1.4 nmoles or more, for example, 1.6 nmoles or more, for example, 1.8 nmoles or more, for example, 2.0 nmoles or more, for example, 2.2 nmoles or more, for example, 2.4 nmoles or more, for example, 2.6 nmoles or more, for example, 2.8 nmoles , for example, 3.0 nmoles or more, for example, up to 3.2 nmoles per kg body weight. The amount used may be up to 3.0 nmoles per kg body weight, for example, up to 2.8 nmoles, for example, up to 2.6 nmoles, for example, up to 2.4 nmoles, for example, up to 2.2 nmoles, for example, up to 2.0 nmoles, for example, up to 1.8 nmoles, for example, up to 1.4 nmoles, for example, up to 1.2 nmoles, for example, up to 1.0 nmoles, for example, up to 0.8 nmoles, for example, up to 0.6 nmoles, for example, up to 0.4 nmoles, for example, up to 0.2 nmoles per kg body weight. The dose is generally in the range of from 0.1 to 3.2 nmoles per kg body weight, for example, within any combination of upper and lower ranges given above.

We have shown that intracerebroventricular (ICV) administration of GLP-1 to rats potently inhibits food intake. This effect is blocked by the specific GLP-1 receptor antagonist, exendin 9-39. GLP-1 receptors are found in the brainstem, arcuate nucleus and PVN. Following ICV GLP-1 administration, c-fos is expressed in the arcuate nucleus and PVN. However, peripheral administration of GLP-1 in man and rat also inhibits food intake and results in c-fos expression in the brainstem of rats. As is disclosed herein, the effect on 1-hour food intake in non-fasted rats of IP administration of GLP-1 is not influenced by the presence of concomitant exendin 9-39, a GLP-1 receptor agonist, in the arcuate nucleus of the rat. This indicates that circulating GLP-1 acts via the brainstem rather than the arcuate nucleus.

These and other findings suggest that the main site for appetite inhibition by peripheral GLP-1 is the dorsal vagal complex, acting in part directly through the area postrema. However, the physiological importance of this in man is currently unclear.

As disclosed herein, when administered to humans, PYY was found to reduce appetite. When infused into humans at physiological post-prandial levels, PYY₃₋₃₆ significantly decreased appetite and reduced food intake by a third over 12 hours, and even by a third over 24 hours. Both the effect itself and the duration of the effect are surprising and unpredictable, as they occurred for many hours after the hormone had been cleared from the circulation. The effects, which are produced at physiological levels of the peptide, are strong indications that PYY acts in vivo to regulate feeding behavior.

As disclosed herein, peripheral administration of PYY₃₋₃₆ in the rat caused an increase of c-fos immunoreactivity in the arcuate nucleus of the hypothalamus and a decrease in hypothalamic neuropeptide Y (NPY) mRNA. Further, electrophysiological studies demonstrated that PYY₃₋₃₆ inhibits synaptic activity of the NPY nerve terminals and thus activates POMC neurons, which are known to receive inhibitory NPY synaptic inputs.

Without being bound by theory, these results demonstrate that the gut hormone PYY₃₋₃₆ can act via the neuropeptide Y Y2 receptor. This hypothesis is supported by the observation that when PYY₃₋₃₆ was administered to neuropeptide Y Y2 receptor null mice (Y2R gene knock out mice), no inhibition of feeding was observed. Administration of PYY₃₋₃₆ to wild type littermates of the Y2R null mice was fully effective in inhibiting feeding.

Thus, a novel gut-brain pathway that inhibits feeding after meals is described. Without being bound by theory, the natural pathway involves release of PYY from the gut, its conversion to PYY₃₋₃₆, which acts as an agonist on the neuropeptide Y Y2 receptor (NPY Y2 receptor) in the brain. The NPY Y2 receptor acts as a inhibitory pre-synaptic receptor reducing release of neuropeptide Y, which is a most potent stimulator of feeding, and also acting on the anorexigenic melanocortin systems, the result of the NPY Y2 receptor activity being to suppress appetite and decrease food intake. The action of PYY₃₋₃₆ may occur in the arcuate nucleus of the hypothalamus, but other areas may be also be involved.

The results obtained show that PYY₃₋₃₆, a gut hormone that circulates in the blood, inhibits appetite at physiological concentrations, and that the inhibitory effect is observed even for some hours after the hormone has been cleared from the blood. This effect has been observed in all species tested, i.e. in mouse, rat and human. The circulating gut hormone appears to act via hypothalamic circuits. The reduction of messenger RNA, necessary for the synthesis of brain appetite regulating hormones, in particular of hypothalamic NPY mRNA may be a possible mechanism for the long action of PYY₃₋₃₆.

When PYY₃₋₃₆ was co-administered parenterally, either IP or IV with GLP-1, a clear synergistic reduction in food intake was produced. This contrasts with the results obtained with oxyntomodulin, where there was no synergistic effect, only a sum of the individual effects. As indicated above, PYY₃₋₃₆ is considered to act via the arcuate nucleus. Oxynotmodulin is also considered to act via that region of the brain. GLP-1, however, is believed to act via the brainstem, as explained above. While not being limited to the following, it appears that, when agents that act via different areas of the brain and/or by different neurological routes are administered parenterally, they produce a synergistic affect, whereas when they act by the same area and/or route they do not.

These results indicate the importance in vivo of the interaction of gut hormones on the regulation of appetite and food intake. The present invention demonstrates that, surprisingly, it is possible to regulate appetite and food intake more effectively by informed choice of agents.

The disclosure is illustrated by the following non-limiting Examples.

### EXAMPLES

### Example 1

### Material and Methods

*Generation of POMC-EGFP mice:* The *EGFP* cassette contains its own Kozak consensus translation initiation site along with *SV40* polyadenylation signals downstream of the *EGFP* coding sequences directing proper processing of the 3' end of the EGFP mRNA. The *EGFP* cassette was introduced by standard techniques into the 5' untranslated region of exon 2 of a mouse *Pomc* genomic clone containing 13 kb of 5' and 2 kb of 3' flanking sequences (Young et al., J Neurosci 18, 6631-40, 1998). The transgene was microinjected into pronuclei of one-cell stage embryos of C57BL/6J mice (Jackson Laboratories) as described (Young et al., J Neurosci 18, 6631-40, 1998). One founder was generated and bred to wildtype C57BL/6J to produce N₁ hemizygous mice. In addition, N₂ and subsequent generations of mice homozygous for the transgene were also generated. The mice are fertile and have normal growth and development.

*Immunofluorescence and GFP co-localization:* Anesthetized mice were perfused transcardially with 4% paraformaldehyde and free-floating brain sections prepared with a vibratome. Sections were processed for immunofluorescence and colocalization of GFP fluorescence using standard techniques. Primary antisera and their final dilutions were rabbit anti-β-endorphin, 1:2500 v/v; rabbit anti-NPY, 1:25,000 v/v (Alanex Corp.); rabbit anti-ACTH, 1:2000 v/v; and mouse anti-TH, 1:1000 v/v (Incstar). After rinsing, sections were incubated with 10mg/ml biotinylated horse anti-mouse/rabbit IgG (Vector Laboratories) followed by Cy-3 conjugated streptavidin, 1:500 v/v (Jackson Immunoresearch Laboratories). Photomicrographs were taken on a Zeiss Axioscop using FITC and RITC filter sets (Chroma Technology Corp.).

*Electrophysiology (Example 2):* 200 µm thick coronal slices were cut from the ARC of four-week old male POMC-EGFP mice. Slices were maintained in (in mM) [NaCl, 126; KCl, 2.5; MgCl₂, 1.2; CaCl₂.2H₂O, 2.4; NaH₂PO₄.H₂O, 1.2; NaHCO₃, 21.4; Glucose, 11.1] (Krebs) at 35°C and saturated with 95% O₂ 5% CO₂ for 1 hour(hr) prior to recordings. Recordings were made in Krebs at 35° C. Slices were visualized on an Axioskop FS2 (Zeiss) through standard infra red optics and using epifluoresence through a FITC filter set (see Fig. 1c). Whole cell recordings were made from fluorescent neurons using an Axopatch 1D amplifier (Axon Instruments) and Clampex 7 (Axon Instruments). Resting membrane potentials were determined using an event detection protocol on a PowerLab system (AD Instruments, Mountain View, CA) to average expanded traces of the membrane potential. Drugs were applied to the bath over the times indicated. The resting membrane potential was stable for up to an hour in cells treated with Krebs alone. I-V relationships for the Met-Enk currents were established using a step protocol; (-60 mV holding potential, sequentially pulsed (40 ms) from -120 to -50 mV, cells were returned to -60 mV for 2 s between voltage steps). The protocol was repeated after Met Enk addition. The net current was the difference between the two I-V relationships. This protocol was repeated in Krebs with 6.5 mM K⁺. I-V relationships to identify the postsynaptic leptin current were performed similarly with slow voltage ramps (5 mV/ s from -100 to -20 mV) before and 10 minutes after the addition of leptin (100 nM). GABAergic IPSCs were recorded using a CsCl internal electrode solution (in mM) [CsCl, 140; Hepes, 10; MgCl₂, 5; Bapta, 1; (Mg)-ATP, 5; (Na)GTP, 0.3]. Both mini IPSCs and large amplitude (presumably multisynaptic) IPSCs were observed in the untreated slices. TTX (1 µM) abolished large IPSCs. Data were acquired before and after addition of drug for the times indicated on the figures at a -50 mV holding potential in 2 s. sweeps every 4 s. Mini postsynaptic currents were analyzed using Axograph 4 (Axon Instruments). IPSCs and excitatory postsynaptic currents (EPSCs) were distinguished on the basis of their decay constants; additionally picrotoxin (100 µM) blocked all IPSCs. POMC neurons receive a low EPSC tone and the frequency was not modulated by any of the treatments described here.

*Immunostainingfor light and electron microscopy:* Double immunocytochemistry for NPY and POMC using different color diaminobenzidine(DAB) chromogens was carried out on fixed mouse hypothalami according to published protocols (Horvath et al., Neuroscience 51, 391-9, 1992). For electron microscopy, preembedding immunostaining for β-endorphin was using an ABC Elite kit (Vector Laboratories) and a DAB reaction followed by post-embedding labeling of GABA and NPY using rabbit anti-GABA, 1:1000 v/v and gold conjugated (10 nm) goat anti-rabbit IgG or sheep anti-NPY and gold conjugated (25 nm) goat anti-sheep IgG. Finally, sections were contrasted with saturated uranyl acetate (10 minutes) and lead citrate (20-30 s) and examined using a Philips CM-10 electron microscope.

*Animals:* Male Wistar rats (200-250g), 7-8 weeks old (Charles River Laboratories, United Kingdom) were maintained under controlled temperature (21-23° C) and light conditions (lights on 07:00-19:00) with *ad libitum* access to water and food (RM1 diet; SDS Ltd., Witham, United Kingdom) except where stated. Arcuate and paraventricular nuclei cannulations and injections were performed as previously described (Glaum et al., Mol. Pharmacol. 50, 230-5, 1996; Lee et al., J. Physiol (Lond) 515, 439-52; 1999; Shiraishi et al., Nutrition 15, 576-9, 1999). Correct intranuclear cannula placement was confirmed histologically at the end of each study period (Glaum et al., Mol. Pharmacol 50, 230-5, 1996; Lee et al., J. Physiol (Lond) 515, 439-52, 1999; Shiraishi et al., Nutrition 15, 576-9, 1999). All animal procedures were approved under the British Home Office Animals (Scientific Procedures) Act, 1986. All injection studies on fasting animals were performed in the early light-phase (0800-0900). All dark-phase feeding studies injections were performed just prior to lights off.

Male *Pomc-EGFP* mice were studied at 5-6 weeks of age and were generated as described above. *Y2r*-null mice were generated using *Cre-lox* P mediated recombination, which results in the germline deletion of the entire coding region of the Y2 receptor. All *Y2r-null* mice were maintained on a mixed C57/B16-129SvJ background. Male mice aged 8-12 weeks and between 20-30 g bodyweight were kept under controlled temperature (21-23° C) and light conditions (lights on 06:00-18:00) with *ad libitum* access to water and food (Gordon's Speciality Stock feeds) except where stated. All studies were performed in the early light-phase (0700-0800).

*Intraperitoneal injections:* Rats were accustomed to IP injection by injections of 0.5 ml saline on the two days prior to study. For all studies, animals received an IP injection of PYY₃₋₃₆, GLP-1 or saline in 500 µl (for rats) or 100 µl (for mice).

*Electrophysiology:* Whole cell patch clamp recordings were made from POMC neurons in the hypothalamus of 180 µm thick coronal slices from *Pomc-*EGFP mice, as previously reported (Cowley et al., Nature 411, 480-484, 2001). "Loose cell-attached" recordings were made using extracellular buffer in the electrode solution, and maintaining seal resistance between 3-5Mohm throughout the recording. Firing rates were analysed using mini-analysis protocols (MiniAnalysis, Jaejin Software, NJ). Vehicle controls were used in this system, previously validated for the electrophysiological actions of neuropeptides (Cowley et al., Nature 411, 480-484, 2001). Data were analysed by ANOVA, Neuman-Keuls posthoc comparison, and Wilcoxon Signed Rank Test.

*Hypothalamic explants:* Male Wistar rats were killed by decapitation and the whole brain immediately removed, mounted with the ventral surface uppermost and placed in a vibrating microtome (Biorad, Microfield Scientific Ltd., Devon, UK). A 1.7 mm slice was taken from the base of the brain to include the PVN and the ARC and immediately transferred to 1ml of artificial CSF (aCSF) (Kim et al., J. Clin. Invest. 105, 1005-11, 2000) equilibrated with 95% O₂ and 5% CO₂ and maintained at 37° C. After an initial 2-hour equilibration period, with aCSF replaced every 60 minutes, the hypothalami were then incubated for 45 minutes in 600µl aCSF (basal period) before being exposed to the Y2A (50nM) in 600µl aCSF. Finally, the viability of the tissue was verified by a 45 minute exposure to 56 mM KCL; isotonicity was maintained by substituting K⁺ for Na⁺. At the end of each period, the aCSF was removed and frozen at -20° C until assayed for NPY and αMSH by radioimmunoassay.

*C-fos expression:* C-fos expression was measured in adult Wistar rats and Pomc-EGFP mice 2 hours after IP administration of saline or PYY₃₋₃₆ (5µg/100g) using standard immunohistochemical techniques (Hoffman et al., Front. Neuroendocrinol. 14, 173-213, 1993). Data were obtained from 3 rats and 5 mice in each group. For the *Pomc-EGFP* mice 5 anatomically matched arcuate nucleus sections (Franklin et al., The Mouse Brain in Stereotaxic Coordinates, Academic Press, San Diego, 1997) were counted from each animal, and images acquired using a Leica TSC confocal microscope (Grove et al., Neuroscience 100, 731-40, 2000).

*RNase protection assay (RPA):* Total RNA was extracted from hypothalami (Trizol, Gibco). RPAs were performed (RPAIII kit, Ambion) using 5µg RNA and probes specific for NPY, αMSH and β actin (internal standard). For each neuropeptide, the ratio of the optical density of the neuropeptide mRNA band to that of β actin was calculated. Neuropeptide mRNA expression levels are expressed relative to saline control (mean ± s.e.m. n = 4 per group). The statistical analysis used was ANOVA, with Bonferroni post hoc analysis.

*Plasma assays:* Human leptin was measured using a commercially available radioimmunoassay (RIA) (Linco Research, USA). All other plasma hormone levels were measured using established in-house RIAs (Tarling et al., Intensive Care Med. 23, 256-260, 1997). Glucose concentrations were measured using a YSI 2300STAT analyser (Yellow Springs Instruments Inc., Ohio, USA). Plasma paracetamol levels were measured using an enzymatic colorimetric assay (Olympus AU600 analyzer).

*Human Studies:* PYY₃₋₃₆ was purchased from Bachem (California, USA). The Limulus Amoebocyte Lysate assay test for pyrogen was negative and the peptide was sterile on culture. Ethical approval was obtained from the Local Research Ethics Committee (project registration 2001/6094) and the study was performed in accordance with the principles of the Declaration of Helsinki. Subjects gave informed written consent.

Each subject was studied on two occasions with at least 1 week between each study. Volunteers filled out a food diary for three days prior to each infusion, and for the following 24 hours. All subjects fasted and drank only water from 20:00 on the evening prior to each study. Subjects arrived at 08:30 on each study day, were cannulated and then allowed to relax for 30 minutes prior to the onset of the study protocol. Blood samples were collected every 30 minutes into heparinised tubes containing 5,000 Kallikrein Inhibitor Units (0.2 ml) of aprotinin (Bayer) and centrifuged. Plasma was separated and then stored at -70° C until analysis. Subjects were infused with either saline or 0.8 pmol.kg¹.min⁻¹ PYY₃₋₃₆ for 90 minutes (about 72 pmol total infusion), in a double blind randomized crossover design.

Two hours after the termination of the infusion, subjects were offered an excess free-choice buffet meal (Edwards et al., Am. J. Physiol. Endocrinol. Metab. 281, E155-E166, 2001), such that all appetites could be satisfied. Food and water were weighed pre- and postprandially and caloric intake calculated. Appetite ratings were made on 100 mm visual analogue scores (VAS) with the text expressing the most positive and the negative rating anchored at each end (Raben et al., Br. J. Nutr. 73, 517-30, 1995). VAS was used to assess hunger, satiety, fullness, prospective food consumption and nausea. Caloric intake following saline and PYY₃₋₃₆ were compared using a paired t test. The postprandial response curves were compared by ANOVA using repeated paired measures, with time and treatment as factors.

*Measurements of Energy Expenditure:* To determine the actions of PYY on energy expenditure the OXYMAX system is utilized with rodents following PYY injection into a treatment cohort. This system is also utilized with rodents following a saline injection (control cohort). The equipment measures O₂ consumption and CO₂ production; the efficiency with which the body produces CO₂ from O₂ gives a reliable index of caloric or metabolic efficiency. A similar system is used with human volunteers.

### Example 2

### Neural Network in the Arcuate Nucleus

A strain of transgenic mice was generated expressing green fluorescent protein (EGFP Clontech), under the transcriptional control of mouse *Pomc* genomic sequences that include a region located between -13 kb and -2 kb required for accurate neuronal expression (Young et al., J Neurosci 18, 6631-40, 1998) (Fig. 1a). Bright green fluorescence (509 nm) was seen in the two CNS regions where POMC is produced: the ARC and the nucleus of the solitary tract. Under ultraviolet (450-480 nm) excitation POMC neurons were clearly distinguished from adjacent, non-fluorescent neurons (Fig. 1b) visualized under infrared optics. Double immunofluorescence revealed >99% cellular co-localization of EGFP and POMC peptides within the ARC (Fig. 1c). There was close apposition of both tyrosine hydroxylase (TH)- and NPY-stained terminals on EGFP-expressing POMC neurons, but no evidence of co-localization of the TH or NPY immunoreactivity with EGFP. Total fluorescent cell counts performed on coronal hypothalamic sections revealed 3148 ± 62 (mean ± SEM: n=3) POMC-EGFP neurons distributed through the entire ARC (Franklin et al., The Mouse Brain in Stereotaxic Coordinates, Academic Press, San Diego, 1997) (Fig. 1d). POMC neurons in the mouse are located both medially and ventrally within the ARC, in contrast to a predominantly lateral position in the rat ARC.

POMC-EGFP neurons in hypothalamic slices had a resting membrane potential of -40 to -45 mV and exhibited frequent spontaneous action potentials. The non-selective opioid agonist met-enkephalin (Met-Enk: 30 µM; Sigma) caused a rapid (35- 40 s), reversible hyperpolarization (10-20 mV) of the membrane potential of POMC cells (n=10) and prevented spontaneous action potential generation (Fig. 2a). In normal (2.5 mM K⁺) Krebs buffer, the reversal-potential of the inwardly-rectifying opioid current was approximately -90mV, while in 6.5 mM K⁺ Krebs the reversal-potential was shifted to approximately -60 mV (n=3: Fig. 2b). The µ opioid receptor (MOP-R) antagonist CTAP (1 µM; Phoenix Pharmaceuticals) completely prevented the current induced by Met-Enk in POMC cells (n=3: Fig. 2c). These characteristics indicate the opioid current was due to activation of MOP-R and increased ion conductance through G protein coupled, inwardly-rectifying potassium channels (GIRK) (Kelly et al., Neuroendocrinology 52, 268-75, 1990). The similar opioid responses in EGFP-labeled POMC neurons to that of a guinea pig (Kelly et al., Neuroendocrinology 52, 268-75, 1990) or mouse (Slugg et al., Neuroendocrinology 72, 208-17, 2000). POMC cells, identified by post-recording immunohistochemistry, suggests that expression of the EGFP transgene does not compromise either expression of receptors nor their coupling to second messenger systems in POMC neurons.

Next, the direct effects of leptin on identified POMC cells in slice preparations were investigated. Leptin (0.1 - 100 nM) depolarized 72 of 77 POMC cells by 3-30 mV (Fig. 3a; mean ± SEM depolarization at 100 nM leptin = 9.7 ± 1.2 mV, n= 45) within 2-10 minutes, in a concentration responsive manner (Fig. 3b). There were two components to the depolarization and neither were fully reversible within 40 minutes. Firstly, the depolarization was due to a small inward current which reversed at approximately -20 mV (Fig. 3c), suggesting the involvement of a non-specific cation channel (Powis et al., Am J Physiol 274, R1468-72, 1998). Secondly, leptin treatment decreased the GABAergic tone onto POMC cells. GABAergic inhibitory postsynaptic currents (IPSCs) were observed in POMC cells and leptin (100 nM) decreased their frequency by 25% (Fig. 3d) in 5 out of 15 cells suggesting that it acted presynaptically to reduce GABA release (leptin had no effect on IPSCs in10 out of 15 POMC neurons). The effect on IPSC frequency occurred with a similar lag to the effect on membrane potential. Thus, leptin not only directly depolarizes POMC neurons but also acts at GABAergic nerve terminals to reduce the release of GABA onto POMC neurons, allowing them to adopt a more depolarized resting potential. The consistent depolarization of POMC cells by leptin was specific because leptin had no effect on 5 of 13 adjacent non-fluorescent cells tested (Fig. 3e), while it hyperpolarized 5 (Fig. 3f) and depolarized 3 other non-POMC neurons in the ARC. The electrophysiological effects of leptin reported here are consistent with leptin's biological actions; leptin rapidly causes release of α-MSH from rat hypothalami (Kim et al., J Clin Invest 105, 1005-11, 2000), presumably by activating POMC neurons.

Previous reports of neuronal hyperpolarization by leptin (Glaum et al., Mol Pharmacol 50, 230-5, 1996; Spanswick et al., Nature 390, 521-5, 1997), and the demonstrated co-localization of GABA and NPY (Horvath et al., Brain Res 756, 283-6, 1997) within subpopulations of ARC neurons, led us to speculate that leptin hyperpolarizes NPY/GABA cells that directly innervate POMC neurons, and thus reduces GABAergic drive onto POMC cells. Both the leptin and NPY Y2 receptors are expressed on NPY neurons in the ARC (Hakansson et al., J Neurosci 18, 559-72, 1998; Broberger et al., Neuroendocrinology 66, 393-408, 1997). Furthermore, activation of Y2 receptors inhibits NPY release from NPY neurons (King et al., J Neurochem 73, 641-6, 1999), and presumably would also diminish GABA release from NPY/GABA terminals. This is an alternative pharmacological approach, independent of leptin, to test the hypothesized innervation of POMC neurons by GABAergic NPY neurons. Indeed, NPY (100 nM; Bachem) decreased the frequency of GABAergic IPSCs by 55% within 3 minutes, in all 12 POMC cells tested (Fig. 4a). Both NPY and leptin still inhibited IPSCs in the presence of tetrodotoxin (TTX) (6 of 6 and 3 of 5 cells respectively), indicating that some of the inhibition of IPSCs was occurring through direct effects at presynaptic nerve terminals. POMC neurons express the NPY Y1 receptor (Broberger et al., Neuroendocrinology 66, 393-408, 1997) and NPY also hyperpolarized all POMC neurons tested, by an average of 9±6 mV (n=3).

Another pharmacological test to confirm the origin of GABAergic innervation on POMC neurons from NPY/GABA terminals was to test the effect of the recently characterized and highly selective MC3-R agonist D-Trp⁸-γMSH (Grieco et al., J Med Chem 43, 4998-5002, 2000) on local GABA release. D-Trp⁸-γMSH (7 nM) increased the frequency of GABAergic IPSCs (280 ± 90%) recorded from 3 of 4 POMC neurons (Fig. 4b). It had no effect on one cell. The positive effect of MC3-R activation, together with the negative effects of NPY and leptin, demonstrate the dynamic range of the NPY/GABA synapse onto POMC neurons and point to the important role of this synapse in modulating signal flow within the ARC. D-Trp⁸-γMSH (7 nM) also hyperpolarized (-5.5 ± 2.4 mV) 9 of 15 POMC neurons tested and decreased the frequency of action potentials (Fig 4c); the remaining cells showed no significant response to D-Trp⁸-γMSH. These effects could be due entirely to increased GABA release onto the POMC cells, or could be due to an additional postsynaptic action of D-Trp⁸-γMSH on POMC neurons, approximately half of which also express the MC3-R (Bagnol et al., J Neurosci (Online) 19, RC26, 1999). Thus, MC3-R acts in a similar autoreceptor manner to MOP-Rs on POMC neurons, diminishing POMC neuronal activity in response to elevated POMC peptides.

To further determine that the IPSCs in POMC neurons were due to local innervation by NPY/GABA cells, multi-label immunohistochemistry was performed using light and electron microscopy. Although independent NPY (Csiffary et al., Brain Res 506, 215-22, 1990) and GABA (Horvath et al., Neuroscience 51, 391-9, 1992) innervation of POMC cells has been reported, co-localization of NPY and GABA in nerve terminals forming synapses onto POMC cells has not been shown. Similar to the rat (Csiffary et al., Brain Res 506, 215-22, 1990), a dense innervation of POMC cells by NPY axon terminals was detected in the mouse (Fig. 4d). Electron microscopy confirmed the coexpression of NPY and GABA in axon terminals and revealed that these boutons established synapses on the perikarya of all 15 ARC POMC neurons analyzed (representative example, Fig. 4e).

A detailed model of regulation of this circuit shows dual mechanisms of leptin action in the ARC, interactions between NPY/GABA and POMC neurons, and autoregulatory feedback from opioid and melanocortin peptides as well as NPY (Fig. 4f). In this model, leptin directly depolarizes the POMC neurons and simultaneously hyperpolarizes the somata of NPY/GABA neurons, and diminishes release from NPY/GABA terminals. This diminished GABA release disinhibits the POMC neurons, and result in an activation of POMC neurons and an increased frequency of action potentials.

### Example 3

### Administration of PYY Inhibits Food Intake

The orexigenic NPY and the anorectic alpha melanocortin stimulating hormone (α-MSH) systems of the hypothalamic arcuate nucleus are involved in the central regulation of appetite (Schwartz et al., Nature 404, 661-671, 2000).
However the potential mechanisms signaling meal ingestion directly to these hypothalamic-feeding circuits are unclear. PYY₃₋₃₆ is a gut-derived hormone that is released postprandially in proportion to the calories ingested (Pedersen-Bjergaard et al., Scand. J. Clin. Lab. Invest. 56, 497-503, 1996). The effects of peripheral administration of PYY₃₋₃₆ on feeding were investigated.

An intraperitoneal injection (IP) of PYY₃₋₃₆ to freely feeding rats, prior to the onset of the dark-phase, significantly decreased subsequent food intake (Fig. 5a). A similar inhibition of feeding was seen following IP injection in rats fasted for 24 hours (Fig. 5b). A time course of the plasma PYY₃₋₃₆ levels achieved following IP injection of PYY₃₋₃₆ demonstrated a peak level at 15 minutes post injection, which was within the normal postprandial range (peak PYY₃₋₃₆ levels 15 minutes post IP injection of 0.3µg/100g = 99.3 ± 10.4 pmol/l vs. peak postprandial level = 112.1 ± 7.8 pmol/l, n = 8-10 per group), suggesting that physiological concentrations of PYY₃₋₃₆ inhibit feeding. PYY₃₋₃₆ did not affect gastric emptying (percentage of food ingested remaining in the stomach at 3 hours: PYY₃₋₃₆ = 36 ± 1.9 %, saline = 37.4 ± 1.0 % n = 12) (Barrachina et al., Am. J. Physiol. 272, R1007-11, 1997). PYY₃₋₃₆ administered IP twice daily for 7 days reduced cumulative food intake (7-day cumulative food intake: PYY₃₋₃₆ = 187.6 ± 2.7g vs. saline = 206.8 ± 2.3, n = 8 per group, P < 0.0001) and decreased body weight gain (Fig. 5d) (PYY₃₋₃₆ = 48.2 ± 1.3g vs. saline = 58.7 ± 1.9, n = 8 per group, P < 0.002).

### Example 4

### PYY Administration Affects c-fos Expression

To investigate whether this inhibition of food intake involved a hypothalamic pathway, c-fos expression was examined in the arcuate nucleus, an important center of feeding control (Schwartz et al., Nature 404, 661-671, 2000; Cowley et al., Nature 411, 480-484, 2001), following a single IP injection of PYY₃₋₃₆. There was a 2-fold increase in the number of cells positive for c-fos in the lateral arcuate of the rat (PYY₃₋₃₆ =168 ± 2, saline = 82.7 ± 5, n = 3, P < 0.0001). Likewise in *Pomc-EGFP*-transgenic mice (Cowley et al., Nature 411, 480-484, 2001) IP administration of PYY₃₋₃₆ resulted in a 1.8-fold increase in the number of arcuate cells positive for c-fos (Fig. 6b), compared with saline control animals (Fig. 6a) (PYY₃₋₃₆ = 250 ± 40, saline = 137 ± 15, n = 5, P < 0.05). IP PYY₃₋₃₆ caused a 2.6 fold increase in the proportion of POMC neurons that express c-fos (PYY₃₋₃₆ = 20.4 ± 2.9%, saline = 8 ± 1.4%, n = 5, P < 0.006) (Figs. 6c and d).

These observations suggested that PYY₃₋₃₆ may act via the arcuate nucleus. Thus, the actions of PYY₃₋₃₆, and its effects upon NPY and POMC circuits in the hypothalamus, were studied. In view of the sustained inhibition of food intake and the effects on weight gain following peripheral administration of PYY₃₋₃₆ both *Pomc* and *Npy* hypothalamic messenger RNA (mRNA) were measured using RNase protection assays. A significant decrease in *Npy* mRNA in response to PYY₃₋₃₆ was observed 6 hours post IP injection, compared with saline treated animals (saline = 17.3 ± 2.0, PYY₃₋₃₆ = 8.8 ± 1.0, relative optical density units, P < 0.02). A non-significant increase occurred in *Pomc* mRNA levels.

### Example 5

### Y2 receptors

PYY₃₋₃₆ shows a 70% amino acid sequence identity to NPY and acts through NPY receptors (Soderberg et al., J. Neurochem. 75, 908-18, 2000). The Y2R is a putative inhibitory presynaptic receptor and is highly expressed on the arcuate NPY neurons (Broberger et al., Neuroendocrinology 66, 393-408, 1997), though not on the neighboring POMC neurons. PYY₃₋₃₆ is a high affinity agonist at the Y2 receptor (Grandt et al., Regul. Pept. 51, 151-159, 1994). It was hypothesized that peripheral PYY₃₋₃₆ inhibits food intake via the Y2R in the arcuate nucleus, an area known to be directly accessible to circulating hormones (Kalra et al., Endocr. Rev. 20, 68-100, 1999).

To investigate this hypothesis, PYY₃₋₃₆ was injected directly into the arcuate nucleus (Kim et al., Diabetes 49, 177-82, 2000). In rats fasted for 24 hours, food intake was significantly decreased by doses as low as 100 fmol (Fig. 7a), resulting in a similar inhibition to that seen following IP administration. To establish whether these effects were via the Y2R, aY2R selective agonist was used (Potter et al., Eur. J. Pharmacol. 267, 253-262, 1994), N-acetyl (Leu ²⁸, Leu ³¹) NPY (24-36) [Y2A]. Its affinity was confirmed using receptor-binding studies (Small et al., Proc. Natl. Acad Sci. U.S.A. 94, 11686-91, 1997) on cell lines expressing the NPY Y1, Y2 and Y5 receptors (Y2 IC₅₀ = 1.3 ± 0.2 nM, Y1 IC₅₀ > 5000 nM, Y5 IC₅₀ > 5000 nM). Intra-arcuate nucleus injection of Y2A in rats previously fasted for 24 hours dose-dependently (100 fmol - 1 nmol) inhibited food intake (chow ingested 2 hours post-injection, 0.1 nmol Y2A = 6.2 ± 0.5 g, saline = 8.2 ± 0.6 g, n = 8 per group, P < 0.05).

To confirm the anatomical specificity of this effect Y2A (100 fmol - 1 nmol) was injected into the paraventricular nucleus (PVN) (Kim et al., J. Clin. Invest. 105, 1005-11, 2000) of rats fasted for 24 hours and found no alteration of food intake (2 hour post-injection saline = 8.3 ± 0.4 g, 0.1nmol Y2A = 8.0 ± 0.6 g, n = 8 per group). To further determine the role of the Y2R in the feeding inhibition caused by peripheral PYY₃₋₃₆, the effect of PYY₃₋₃₆ on *Y2r*-null mice and littermate controls was examined. PYY₃₋₃₆ inhibited daytime feeding in a dose responsive manner in fasted male wild-type mice but did not inhibit food intake in fasted male *Y2r*-null mice (Figs. 7b and 7c). Food intake measured in response to a fast demonstrated that male *Y2r*-null mice eat significantly more at 2, 4 and 24 hours compared with their littermate controls (24-hour cumulative food intake; *Y2r*-null mice = 7.1 ± 0.48g vs. wild-type = 5.3 ± 0.7g, n = 8 per group, P < 0.05).

The electrophysiological response of hypothalamic POMC neurons to administration of both PYY₃₋₃₆ and Y2A was examined. These neurons were identified using mice with targeted expression of green fluorescent protein in POMC neurons (Cowley et al., Nature 411, 480-484, 2001). PYY₃₋₃₆ disinhibited the POMC neurons, resulting in a significant depolarization of 19 of the 22 POMC neurons tested (Fig. 8a inset) (10.3 ± 2.1 mV depolarization, n = 22, P < 0.0003). A similar depolarization was seen with Y2A (8.7 ±1.8 mV depolarization, n = 9, P < 0.002). The depolarization caused by PYY₃₋₃₆ stimulated a significant increase in the frequency of action potentials in POMC neurons (Fig 8a) (93% increase over control, P < 0.05, n = 22). In the whole cell mode the effect of PYY₃₋₃₆ was sometimes reversed upon washout, but only after a long latency (30 minutes). A similar washout of leptin effects upon these neurons was observed.

To exclude effects of cellular rundown, or seal deterioration, the effects of PYY₃₋₃₆ in the "loose cell-attached" (or extracellular) configuration was examined. PYY₃₋₃₆ caused a reversible 5-fold increase in the frequency of action potentials in loose cell-attached recordings of POMC neurons (Fig. 8b). This increase in firing rate occurred with the same latency as PYY₃₋₃₆ reduced the frequency of inhibitory postsynaptic currents (IPSCs) onto all 13 POMC neurons tested (Fig. 8c) (51.9 ± 9.2 % reduction, n = 13, P < 0.0001), indicating a reduced frequency of GABA release onto POMC neurons. Interestingly, the firing rate of POMC neurons returned to basal, in spite of continued inhibition of IPSCs. A similar effect upon IPSC frequency was seen with Y2A (44.4 ± 9.3% reduction, n = 8, P < 0.004) suggesting this effect to be via Y2R. PYY₃₋₃₆ (25 nM) caused a hyperpolarization (5.2 ± 1.16 mV, P < 0.004, n = 5) of unidentified, but presumably NPY-containing, non-POMC, neurons in the arcuate nucleus. There is a tonic GABAergic inhibition of POMC neurons by NPY neurons (Cowley et al., Nature 411, 480-484, 2001) and these results suggest that PYY₃₋₃₆ acts by inhibiting NPY neurons, thus decreasing this GABAergic tone and consequentially disinhibiting POMC neurons. The effect of Y2A on peptide secretion was also examined using hypothalamic explants (Kim et al., J. Clin. Invest. 105, 1005-11, 2000). Y2A significantly decreased NPY release, with a concomitant increase in α-MSH release from hypothalamic explants (Figs. 8d and 4e). Taken together, these observations suggest that PYY₃₋₃₆ modulates both the NPY and melanocortin systems in the arcuate nucleus.

### Example 6

### Human Studies

Because of the importance of the melanocortin system in man (Barsh et al., Nature 404, 644-651, 2000) and the profound effects of PYY₃₋₃₆ on both feeding and weight change seen in rodents, the effects of PYY₃₋₃₆ on appetite and food intake were investigated in human subjects. Twelve healthy fasted, non-obese volunteers (six men and six women, mean age 26.7 ± 0.7 years, BMI = 24.6 ± 0.94 kg.m⁻²) were infused with PYY₃₋₃₆ (0.8 pmol.kg⁻¹.min⁻¹) or saline for 90 minutes in a double-blind placebo controlled crossover study.

PYY₃₋₃₆ plasma concentrations increased from mean basal concentration of 8.3 ± 1.0 pM to 43.5 ± 3 pM during the PYY₃₋₃₆ infusion and mimicked postprandial levels (Pedersen-Bjergaard et al., Scand. J. Clin. Lab. Invest. 56, 497-503, 1996; Adrian et al., Gastroenterology 89, 1070-1077, 1985). Post-infusion, PYY₃₋₃₆ concentrations returned to basal within 30 minutes. PYY₃₋₃₆ infusion resulted in a significant decrease in hunger scores (Raben et al., Br. J. Nutr. 73, 517-30, 1995) (Fig. 9c), but not in the scores for sleepiness or sickness. Calorie intake during a free-choice buffet meal (Tarling et al., Intensive Care Med. 23, 256-260, 1997) two hours after the termination of the infusion was reduced by over a third compared to saline (36 ± 7.4%, p < 0.0001) (Fig. 9a). There was no effect upon fluid intake and no difference in sensations of fullness or nausea reported by the volunteers. PYY₃₋₃₆ administration had no effect on gastric emptying, as estimated by the paracetamol absorption method (Edwards et al., Am. J. Physiol. Endocrinol. Metab. 281, E155-E166, 2001; Tarling et al., Intensive Care Med. 23, 256-260, 1997), or on plasma glucose, plasma leptin, GLP-1, or insulin. Analysis of the food diaries revealed a significant inhibition of food intake in the 12-hour period following the PYY₃₋₃₆ infusion (saline = 2205 ± 243 kcal, PYY₃₋₃₆ = 1474 ± 207 kcal). However, food intake during a 12 to 24 hour period between the two groups was virtually identical. Overall there was a 33% decrease in cumulative total calorie consumption in the 24-hour period following the PYY₃₋₃₆ infusion (Fig.9b). These findings demonstrate that infusion of PYY₃₋₃₆, matching postprandial levels, caused a marked inhibition of both appetite and food intake in man.

In an additional study, two groups of healthy subjects (n = 12 per group, 6 males and 6 females), one with increased Body Mass Index (BMI) (mean = 32.73 +/- 0.93 kg/m2) and another group with low BMI (mean = 20.49 +/- 2.05 kg/m2), were studied on two occasions with at least 1 week between each study. All subjects fasted and drank only water from 20:00 hours on the evening prior to each study. Subjects arrived at 08:30 on each study day, were cannulated and then allowed to relax for 30 minutes prior to the onset of the study protocol. Subjects were infused with either saline or 0.8 pmol.kg1.min-1 PYY₃₋₃₆ for 90 minutes, in a double blind randomized crossover design. Two hours after the termination of the infusion, subjects were offered an excess free-choice buffet meal, such that all appetites could be satisfied. Food and water were weighed pre- and postprandially and caloric intake calculated. Caloric intake following saline and PYY₃₋₃₆ were compared using a paired t test (p<0.001). The number of calories ingested following administration of PYY₃₋₃₆ differed significantly from the number of calories ingested following administration of saline for both the overweight group and the lean group. The overweight group showed a 28.8 +/- 4.3 % reduction and the lean group a 31.1 +/-4.4 % reduction. However, the reduction for the overweight group did not differ significantly from the reduction for the lean group. These findings demonstrate that infusion of PYY₃₋₃₆, matching postprandial levels, caused a marked inhibition of both appetite and food intake in both lean and overweight subjects.

Administration of PYY₃₋₃₆ at doses of 10 nmoles and 20 nmoles (in saline) subcutaneously to human subjects resulted in raised plasma levels of PYY₃₋₃₆ as shown in Fig. 10.

Without being bound by theory, cells within the arcuate nucleus could detect circulating peripheral satiety signals and relay these signals to other brain regions (Butler et al., Nature Neuroscience 4, 605-611, 2001). This is supported by the observation that leptin modifies the activity of both the POMC and NPY arcuate neurons (Cowley et al., Nature 411, 480-484, 2001). The results disclosed herein demonstrate, through a combination of electrophysiological and hypothalamic explant studies, that the gut hormone, PYY₃₋₃₆, can directly influence hypothalamic circuits, resulting in coordinate changes in POMC and NPY action. The results presented here demonstrate that NPY neurons in the ARC are not protected by the blood/brain barrier, and thus are accessible to circulating molecules. Furthermore, PYY₃₋₃₆ administered directly into this brain region reduces food intake.

The data disclosed herein demonstrates that postprandial levels of PYY₃₋₃₆ inhibit food intake in more than one mammalian species (e.g. rodents and human subjects) for up to 12 hours, thereby demonstrating a role in regulation of food intake. This role can be described as a long term role, such as over a period of several hours (e.g. at least two, three, four, eight, or twelve hours, or from about two to about fifteen hours). This is in contrast to previously characterized gut-derived 'short-term' satiety signals, e.g. cholecystokinin (Schwartz et al., Nature 404, 661-671, 2000; Moran, Nutrition 16, 858- 865, 2000), the effects of which are relatively short-lived (e.g., from about 1-4 hours).

The failure of PYY₃₋₃₆ to inhibit food intake in the *Y2r*-null mice provides evidence that PYY₃₋₃₆ reduces food intake via a Y2R dependent mechanism. The results disclosed herein suggest the existence of a novel gut-hypothalamic pathway in the regulation of feeding, involving postprandial PYY₃₋₃₆ acting at the arcuate Y2R. Thus, PYY, and analogs thereof, such as PYY₃₋₃₆ provide novel therapeutic agents for the treatment of obesity.

### Example 7

### Parenterally administered GLP-1

GLP-1 i.e. GLP-1 (7-36) amide (25 nmol/kg) or saline (control) was administered parenterally to non-fasted rats prior to the onset of the dark phase. Exendin 9-39 (20 nmoles/kg) or saline (control) injected into the arcuate nucleus of non-fasted rats. Food intake one hour later was measured.

The results as shown in Fig. 11 demonstrate that the effect on 1-hour food intake in non-fasted rats of IP administration of GLP-1 is not influenced by the presence of concomitant exendin 9-39, a GLP-1 receptor agonist, in the arcuate nucleus of the rat. This indicates that circulating GLP-1 acts via the brainstem rather than the arcuate nucleus. These and other findings suggest that the main site for appetite inhibition by peripheral GLP-1 is the dorsal vagal complex, acting in part directly through the area postrema.

### Example 8

### Co-administration of PYY₃₋₃₆ and GLP-1 in rats

PYY₃₋₃₆ and GLP-1 i.e. GLP-1 (7-36) amide were injected (intraperitoneally) into non-fasted rats prior to the onset of the dark phase. GLP-1 and PYY₃₋₃₆ were administered separately at the following doses: GLP-1 30µg/kg = 1xG or 60µg/kg = 2xG in 500 µl saline; PYY₃₋₃₆ (3µg/kg = 1xP or 6µg/kg = 2xP in 500 µl saline). PYY₃₋₃₆ and GLP-1 were co-administrated IP (P+G group) at doses of GLP-1 30µg/kg and PYY₃₋₃₆ 3µg/kg combined.

The results set out in Fig. 12 show that there is a clear synergistic effect on the reduction of food intake when PYY₃₋₃₆ and GLP-1 were co-administered (P+G). However, when the procedure was carried out substituting oxyntomodulin for GLP-1, the results obtained for reduction of food intake when PYY₃₋₃₆ and oxynotomdulin were co-administered were only the sum of the individual effects. No synergistic effect was observed.

### Example 9

### Co-administration of PYY₃-₃₆ and GLP-1 in humans

10 healthy volunteers (4 men and 6 women) having a mean age of 24.7 (+/-0.8) years and a mean BMI of 22.7 (+/- 0.6) kg/m² were recruited. The volunteers were asked to fast and to drink only water from 9 pm on the night preceding all study days. Each volunteer attended for four study days. The volunteers arrived at 9.30 am and the venflons were inserted. A rest period from 10.00 to 10.30 followed. Intrvenous infusion started at 10.30, and a buffet lunch was provided between 12.00 and 12.30. Infusion was terminated at 12.30, and the volunteers went home at 13.30.

The volunteers were given the following intra-venous infusions for 120 minutes (90 minutes pre-meal) in random order: Saline, GLP-1, PYY₃₋₃₆, PYY₃₋₃₆+GLP-1. The dose of both GLP-1 and PYY3-36 was 0.4pmol/kg/min. The study was double blinded. GLP-1 is i.e. GLP-1 (7-36) amide

The results of the food intake from the free buffet meal are given in the Table below

**Food intake from free buffet meal (Kcal)**

| | Saline | G | P | P+G |
|---|---|---|---|---|
| mean | 997 | 950 | 848 | 724 |
| Sem | 68.0 | 81.3 | 105.2 | 66.6 |
| p v saline | | 0.336318995 | 0.069375886 | 3.00605E-06 |
| p v P+G | | 0.003622523 | 0.044418337 | |
| % reduction from saline | | 5% | 15% | 27% |

using paired t tests for all statistical comparisons

These result demonstrate a synergistic effect when PYY3-36 and GLP-1 are co-administered.

The results obtained in Examples 4 and 5 above demonstrate that PYY₃₋₃₆ acts via the arcuate nucleus. Oxynotmodulin is also considered to act via that region of the brain. The results obtained in Example 8 above indicates that GLP-1 acts via the brainstem. It appears from the results obtained in this Example that, when agents such as PYY₃₋₃₆ and GLP-1 that act via different areas of the brain and/or by different neurological routes are administered parenterally, they produce a synergistic affect, whereas when they act by the same area and/or route such as PYY₃₋₃₆ and oxynotmodulin, they do not. The synergistic effect on food intake obtained when PYY and GLP-1 were co-administered to humans confirms the observation made in rats.

These results indicate the importance in vivo of the interaction of gut hormones on the regulation of appetite and food intake. The present invention demonstrates that, surprisingly, it is possible to regulate appetite and food intake more effectively by informed choice of agents.

## Claims

1. A Peptide YY (PYY) or an agonist thereof and a Glucagon-like peptide-1 (GLP-1) or an agonist thereof for use in treating obesity and/or diabetes in a human subject,
wherein the PYY or agonist thereof, and GLP-1 or agonist thereof are in a form suitable for peripheral administration, and
wherein the PYY or agonist thereof is for administration at a molar equivalent amount of a PYY₃₋₃₆ dose of no more than 2.2 nmol per kg body weight of said subject.

2. The PYY or agonist thereof and the GLP-1 or agonist thereof for use according to claim 1
wherein the PYY or agonist thereof and the GLP-1 or agonist thereof are formulated for administration simultaneously or sequentially.

3. The PYY or agonist thereof and the GLP-1 or agonist thereof for use according to any one of claims 1 to 2 wherein the PYY and the GLP-1 are formulated for administration via different routes.

4. The PYY or agonist thereof and the GLP-1 or agonist thereof for use according to any one of claims 1 to 3 wherein the subject has type II diabetes.

5. The PYY or agonist thereof and the GLP-1 or agonist thereof for use according to any one of claims 1 to 4 wherein peripheral administration comprises subcutaneous, intravenous, intramuscular, intranasal, transdermal or sublingual administration.

6. The PYY or agonist thereof and the GLP-1 or agonist thereof for use according to any one of claims 1 to 5 wherein the PYY or agonist thereof formulated for peripheral administration comprises a formulation for peripheral administration of about 45 to about 135 pmol per kilogram body weight of the subject, or of about 72 pmol per kilogram body weight of the subject.

7. The PYY or agonist thereof and the GLP-1 or agonist thereof for use according to any one of claims 1 to 5 wherein the PYY or agonist thereof formulated for peripheral administration comprises a formulation suitable for administration in a multitude of doses, wherein each dose in the multitude of doses comprises administration of about 0.5 to about 135 pmol per kilogram of body weight at least about 30 minutes prior to a meal.

8. The PYY or agonist thereof and the GLP-1 or agonist thereof for use according to any one of claims 1 to 7 wherein the PYY or the agonist thereof is formulated for administration in an amount sufficient to decrease calorie intake for a period of about 2 to 12 hours.

9. The PYY or agonist thereof and the GLP-1 or agonist thereof for use according to any one of claims 1 to 5 wherein the PYY or agonist thereof is formulated for peripheral administration at a dose of 0.1 nmoles per kg body weight of the subject, 0.2 nmoles per kg body weight of the subject, 0.4 nmoles per kg body weight of the subject, 0.6 nmoles per kg body weight of the subject, 0.8 nmoles per kg body weight of the subject, 1.0 nmole per kg body weight of the subject, 1.2 nmoles per kg body weight of the subject, 1.4 nmoles per kg body weight of the subject, 1.6 nmoles per kg body weight of the subject, 1.8 nmoles per kg body weight of the subject, 2.0 nmoles per kg body weight of the subject, 2.2 nmoles per kg body weight of the subject.

10. The PYY or agonist thereof and the GLP-1 or agonist thereof for use according to any one of claims 1 to 9 wherein the PYY or agonist thereof is modified by one or more of amidation, glycosylation, acylation, sulfation, phosphorylation, cyclization, lipidization, or pegylation.

11. The PYY or agonist thereof and the GLP-1 or agonist thereof for use according to any one of claims 1 to 10 wherein the GLP-1 or agonist thereof comprises the amino acid sequence of GLP-1 (7-36) (SEQ ID NO:339) or a variant thereof that binds a GLP-1 receptor.

12. The PYY or agonist thereof and the GLP-1 or agonist thereof for use according to any one of claims 1 to 11 wherein the PYY is PYY₃₋₃₆ or variant thereof.

13. The PYY or agonist thereof and the GLP-1 or agonist thereof for use according to any one of claims 1 to 12 wherein the GLP-1 agonist is extendin-4 or a derivative thereof that is a GLP-1 agonist.

14. The PYY or agonist thereof and the GLP-1 or agonist thereof for use according to any one of claims 1 to 13 suitable for administration in combination with amfepramone (diethylpropion), phentermine, mazindol, phenylpropanolamine, fenfluramine, dexfenfluramine, or fluoxetine.

15. The PYY or agonist thereof and the GLP-1 or agonist thereof for use according to any one of claims 1 to 14 wherein the PYY or agonist thereof is formulated for administration in an amount of from 1 to 100 nmols, from 2 to 90 nmols, from 5 to 80 nmols, or from 5 to 50 nmols.

16. A non-therapeutic method for:
a) decreasing calorie intake or food intake,
b) reducing weight gain, or inducing weight loss,
c) controlling any one or more of appetite, satiety and hunger,
d) maintaining desired body weight, a desired Body Mass Index, and/or a desired appearance and good health,
e) improving lipid profile,
in a human subject comprising the administration of Peptide YY (PYY) or an agonist thereof and a Glucagon-like peptide-1 (GLP-1) or an agonist thereof,
wherein the PYY or agonist thereof, and GLP-1 or agonist thereof are in a form suitable for peripheral administration,
wherein the PYY or agonist thereof is for administration at a molar equivalent amount of a PYY₃₋₃₆ dose of no more than 2.2 nmol per kg body weight of a subject, and
wherein the subject desires to lose weight.

17. The method of claim 16 wherein the PYY or agonist thereof and the GLP-1 or agonist thereof are formulated for administration simultaneously or sequentially.

18. The method of any one of claims 16 or 17 wherein the PYY and the GLP-1 are formulated for administration via different routes.

19. The method of any one of claims 16 to 18 wherein peripheral administration comprises subcutaneous, intravenous, intramuscular, intranasal, transdermal or sublingual administration.

20. The method of any one of claims 16 to 19 wherein the PYY or agonist thereof formulated for peripheral administration comprises a formulation for peripheral administration of about 45 to about 135 pmol per kilogram body weight of the subject, or of about 72 pmol per kilogram body weight of the subject.

21. The method of any one of claims 16 to 19 wherein the PYY or agonist thereof formulated for peripheral administration comprises a formulation suitable for administration in a multitude of doses, wherein each dose in the multitude of doses comprises administration of about 0.5 to about 135 pmol per kilogram of body weight at least about 30 minutes prior to a meal.

22. The method of any one of claims 16 to 21 wherein the PYY or the agonist thereof is formulated for administration in an amount sufficient to decrease calorie intake for a period of about 2 to 12 hours.

23. The method of any one of claims 16 to 19 wherein the PYY or agonist thereof is formulated for peripheral administration at a dose of 0.1 nmoles per kg body weight of the subject, 0.2 nmoles per kg body weight of the subject, 0.4 nmoles per kg body weight of the subject, 0.6 nmoles per kg body weight of the subject, 0.8 nmoles per kg body weight of the subject, 1.0 nmole per kg body weight of the subject, 1.2 nmoles per kg body weight of the subject, 1.4 nmoles per kg body weight of the subject, 1.6 nmoles per kg body weight of the subject, 1.8 nmoles per kg body weight of the subject, 2.0 nmoles per kg body weight of the subject, 2.2 nmoles per kg body weight of the subject.

24. The method of any one of claims 16 to 23 wherein the PYY or agonist thereof is modified by one or more of amidation, glycosylation, acylation, sulfation, phosphorylation, cyclization, lipidization, or pegylation.

25. The method of any one of claims 16 to 24 wherein the GLP-1 or agonist thereof comprises the amino acid sequence of GLP-1 (7-36) (SEQ ID NO:339) or a variant thereof that binds a GLP-1 receptor.

26. The method of any one of claims 16 to 25 wherein the PYY is PYY₃₋₃₆ or variant thereof.

27. The method of any one of claims 16 to 24 wherein the GLP-1 agonist is extendin-4 or a derivative thereof that is a GLP-1 agonist.

28. The method of any one of claims 16 to 27 suitable for administration in combination with amfepramone (diethylpropion), phentermine, mazindol, phenylpropanolamine, fenfluramine, dexfenfluramine, or fluoxetine.

29. The method of any one of claims 16 to 28 wherein the PYY or agonist thereof is formulated for administration in an amount of from 1 to 100 nmols, from 2 to 90 nmols, from 5 to 80 nmols, or from 5 to 50 nmols.

## Patentansprüche

1. Peptid YY (PYY) oder ein Agonist davon und ein Glukagon-artiges Peptid-1(GLP-1) oder ein Agonist davon zur Verwendung bei der Behandlung von Fettsucht und/oder Diabetes bei einem menschlichen Individuum,
wobei das PYY oder der Agonist davon und das GLP-1 oder der Agonist davon in einer Form sind, die zur peripheren Verabreichung geeignet ist, und
wobei das PYY oder der Agonist davon zur Verabreichung in einer Moläquivalentmenge einer PYY₃₋₃₆-Dosis von nicht mehr als 2,2 nmol pro kg Körpergewicht des Individuums ist.

2. PYY oder Agonist davon und GLP-1 oder Agonist davon zur Verwendung nach Anspruch 1, wobei das PYY oder der Agonist davon und das GLP-1 oder der Agonist davon zur gleichzeitigen oder aufeinanderfolgenden Verabreichung formuliert sind.

3. PYY oder Agonist davon und GLP-1 oder Agonist davon zur Verwendung nach einem der Ansprüche 1 bis 2, wobei das PYY und das GLP-1 zur Verabreichung auf unterschiedlichen Wegen formuliert sind.

4. PYY oder Agonist davon und GLP-1 oder Agonist davon zur Verwendung nach einem der Ansprüche 1 bis 3, wobei das Individuum Typ II - Diabetes hat.

5. PYY oder Agonist davon und GLP-1 oder Agonist davon zur Verwendung nach einem der Ansprüche 1 bis 4, wobei die periphere Verabreichung eine subkutane, intravenöse, intramuskuläre, intranasale, transdermale oder sublinguale Verabreichung umfasst.

6. PYY oder Agonist davon und GLP-1 oder Agonist davon zur Verwendung nach einem der Ansprüche 1 bis 5, wobei das PYY oder der Agonist davon, formuliert zur peripheren Verabreichung, eine Formulierung zur peripheren Verabreichung von etwa 45 bis etwa 135 pmol per kg Körpergewicht des Individuums oder von etwa 72 pmol pro kg Körpergewicht des Individuums aufweist.

7. PYY oder Agonist davon und GLP-1 oder Agonist davon zur Verwendung nach einem der Ansprüche 1 bis 5, wobei das PYY oder der Agonist davon, formuliert zur peripheren Verabreichung, eine Formulierung aufweist, die geeignet ist zur Verabreichung in einer Vielzahl von Dosen, wobei jede Dosis in der Vielzahl von Dosen eine Verabreichung von etwa 0,5 bis etwa 135 pmol pro kg Körpergewicht mindestens etwa 30 Minuten vor einer Mahlzeit umfasst.

8. PYY oder Agonist davon und GLP-1 oder Agonist davon zur Verwendung nach einem der Ansprüche 1 bis 7, wobei das PYY oder der Agonist davon formuliert ist zur Verabreichung in einer Menge, die ausreichend ist, um die Kalorienaufnahme für eine Dauer von etwa 2 bis 12 Stunden zu verringern.

9. PYY oder Agonist davon und GLP-1 oder Agonist davon zur Verwendung nach einem der Ansprüche 1 bis 5, wobei das PYY oder der Agonist davon formuliert ist zur peripheren Verabreichung in einer Dosis von 0,1 nmol pro kg Körpergewicht des Individuums, 0,2 nmol pro kg Körpergewicht des Individuums, 0,4 nmol pro kg Körpergewicht des Individuums, 0,6 nmol pro kg Körpergewicht des Individuums, 0,8 nmol pro kg Körpergewicht des Individuums, 1,0 nmol pro kg Körpergewicht des Individuums, 1,2 nmol pro kg Körpergewicht des Individuums, 1,4 nmol pro kg Körpergewicht des Individuums, 1,6 nmol pro kg Körpergewicht des Individuums, 1,8 nmol pro kg Körpergewicht des Individuums, 2,0 nmol pro kg Körpergewicht des Individuums, 2,2 nmol pro kg Körpergewicht des Individuums.

10. PYY oder Agonist davon und GLP-1 oder Agonist davon zur Verwendung nach einem der Ansprüche 1 bis 9, wobei das PYY oder der Agonist davon modifiziert ist durch eine oder mehrere der Maßnahmen Amidierung, Glykosylierung, Acylierung, Sulfatierung, Phosphorylierung, Zyklisierung, Lipidisierung oder Pegylierung.

11. PYY oder Agonist davon und GLP-1 oder Agonist davon zur Verwendung nach einem der Ansprüche 1 bis 10, wobei das GLP-1 oder der Agonist davon die Aminosäuresequenz GLP-1 (7 bis 36) (SEQ ID NO: 339) oder eine Variante davon, die einen GLP-1-Rezeptor bindet, aufweist.

12. PYY oder Agonist davon und GLP-1 oder Agonist davon zur Verwendung nach einem der Ansprüche 1 bis 11, wobei das PYY PYY₃₋₃₆ oder eine Variante davon ist.

13. PYY oder Agonist davon und GLP-1 oder Agonist davon zur Verwendung nach einem der Ansprüche 1 bis 12, wobei der GLP-1-Agonist Extendin-4 oder ein Derivat davon ist, das ein GLP-1-Agonist ist.

14. PYY oder Agonist davon und GLP-1 oder Agonist davon zur Verwendung nach einem der Ansprüche 1 bis 13, geeignet zur Verabreichung in Kombination mit Amfepramon (Diethylpropion), Phentermin, Mazindol, Fenylpropanolamin, Phenfluramin, Dexfinfluramin oder Floexetin.

15. PYY oder Agonist davon und GLP-1 oder Agonist davon zur Verwendung nach einem der Ansprüche 1 bis 14, wobei das PYY oder der Agonist davon formuliert ist zur Verabreichung in einer Menge von 1 bis 100 nmol, von 2 bis 90 nmol, von 5 bis 80 nmol oder von 5 bis 50 nmol.

16. Nicht-therapeutisches Verfahren zur:
a) Verringerung der Kalorienaufnahme oder der Nahrungsmittelaufnahme,
b) Reduzierung der Gewichtszunahme oder Einleitung eines Gewichtsverlusts
c) Kontrolle eines oder mehrerer der Gefühle Appetit, Sättigung und Hunger,
d) Beibehaltung eines gewünschten Körpergewichts, eines gewünschten Körpermasse-Indexes und/oder eines gewünschten Aussehens und guter Gesundheit,
e) Verbesserung des Lipidprofils,
bei einem menschlichen Individuum, aufweisend die Verabreichung von Peptid YY (PYY) oder eines Agonisten davon und eines Glukagon-artigen Peptids-1 (GPL-1) oder eines Agonisten davon,
wobei das PYY oder der Agonist davon und das GLP-1 oder der Agonist davon in einer Form sind, die zur peripheren Verabreichung geeignet ist,
wobei das PYY oder der Agonist davon zur Verabreichung in einer Moläquivalentmenge einer PYY₃₋₃₆-Dosis von nicht mehr als 2,2 nmol pro kg Körpergewicht eines Individuums ist, und wobei das Individuum Gewicht zu verlieren wünscht.

17. Verfahren nach Anspruch 16, wobei das PYY oder der Agonist davon und das GLP-1 oder der Agonist davon zur gleichzeitigen oder aufeinanderfolgenden Verabreichung formuliert sind.

18. Verfahren nach einem der Ansprüche 16 oder 17, wobei das PYY und das GLP-1 zur Verabreichung auf unterschiedlichen Wegen formuliert sind.

19. Verfahren nach einem der Ansprüche 16 bis 18, wobei die periphere Verabreichung eines subkutante, intravenöse, intramuskuläre, intranasale, transdermale oder sublinguale Verabreichung umfasst.

20. Verfahren nach einem der Ansprüche 16 bis 19, wobei das PYY oder der Agonist davon, formuliert zur peripheren Verabreichung, eine Formulierung zur peripheren Verabreichung von etwa 45 bis etwa 135 pmol pro kg Körpergewicht des Individuums, oder von etwa 72 pmol pro kg Körpergewicht des Individuums aufweist.

21. Verfahren nach einem der Ansprüche 16 bis 19, wobei das PYY oder der Agonist davon, formuliert zur peripheren Verabreichung, eine Formulierung aufweist, die geeignet ist zur Verabreichung in einer Vielzahl von Dosen, wobei jede Dosis in der Vielzahl von Dosen eine Verabreichung von etwa 0,5 bis etwa 135 pmol pro kg Körpergewicht mindestens etwa 30 Minuten vor einer Mahlzeit umfasst.

22. Verfahren nach einem der Ansprüche 16 bis 21, wobei das PYY oder der Agonist davon formuliert ist zur Verabreichung in einer Menge, die ausreichend ist, um die Kalorienaufnahme für eine Dauer von etwa 2 bis 12 Stunden zu verringern.

23. Verfahren nach einem der Ansprüche 16 bis 19, wobei das PYY oder der Agonist davon formuliert ist zur peripheren Verabreichung in einer Dosis von 0,1 nmol pro kg Körpergewicht des Individuums, 0,2 nmol pro kg Körpergewicht des Individuums, 0,4 nmol pro kg Körpergewicht des Individuums, 0,6 nmol pro kg Körpergewicht des Individuums, 0,8 nmol pro kg Körpergewicht des Individuums, 1,0 nmol pro kg Körpergewicht des Individuums, 1,2 nmol pro kg Körpergewicht des Individuums, 1,4 nmol pro kg Körpergewicht des Individuums, 1,6 nmol pro kg Körpergewicht des Individuums, 1,8 nmol pro kg Körpergewicht des Individuums, 2,0 nmol pro kg Körpergewicht des Individuums, 2,2 nmol pro kg Körpergewicht des Individuums.

24. Verfahren nach einem der Ansprüche 16 bis 23, wobei das PYY oder der Agonist davon modifiziert ist durch eine oder mehrere der Maßnahmen Amidierung, Glykosylierung, Acylierung, Sulfatierung, Phosphorylierung, Zyklisierung, Lipidisierung oder Pegylierung.

25. Verfahren nach einem der Ansprüche 16 bis 24, wobei das GLP-1 oder der Agonist davon die Aminosäuresequenz GLP-1 (7 bis 36) (SEQ ID NO: 339) oder eine Variante davon, die einen GLP-1-Rezeptor bindet, aufweist.

26. Verfahren nach einem der Ansprüche 16 bis 25, wobei das PYY PYY₃₋₃₆ oder eine Variante davon ist.

27. Verfahren nach einem der Ansprüche 16 bis 24, wobei der GLP-1-Agonist Extendin-4 oder ein Derivat davon ist, das ein GLP-1-Agonist ist.

28. Verfahren nach einem der Ansprüche 16 bis 27, das geeignet ist zur Verabreichung in Kombination mit Amfepramon (Diethylpropion), Phentermin, Mazindol, Fenylpropanolamin, Phenfluramin, Dexfinfluramin oder Floexetin.

29. Verfahren nach einem der Ansprüche 16 bis 28, wobei das PYY oder der Agonist davon formuliert ist zur Verabreichung in einer Menge von 1 bis 100 nmol, von 2 bis 90 nmol, von 5 bis 80 nmol oder von 5 bis 50 nmol.

## Revendications

1. Un peptide YY (PYY) ou un agoniste de celui-ci et un peptide apparenté au glucagon (GLP-1) ou un agoniste de celui-ci pour une utilisation pour le traitement de l'obésité et/ou des diabètes d'un sujet humain,
dans lequel le PYY ou l'agoniste de celui-ci et le GLP-1 ou l'agoniste de celui-ci sont sous une forme convenant à une administration périphérique, et
dans lequel le PYY ou l'agoniste de celui-ci sont utilisés pour une administration en une quantité molaire équivalente à une dose de PYY₃₋₃₆ non supérieure à 2,2 nmol par kg de poids corporel dudit sujet.

2. Le PYY ou un agoniste de celui-ci et le GLP-1 ou un agoniste de celui-ci pour une utilisation selon la revendication 1, dans lequel le PYY ou l'agoniste de celui-ci et le GLP-1 ou l'agoniste de celui-ci sont formulés pour une administration simultanée ou séquentielle.

3. Le PYY ou un agoniste de celui-ci et le GLP-1 ou un agoniste de celui-ci pour une utilisation selon les revendications 1 ou 2, dans lequel le PYY et le GLP-1 sont formulés pour une administration par différentes voies.

4. Le PYY ou un agoniste de celui-ci et le GLP-1 ou un agoniste de celui-ci pour une utilisation selon l'une quelconque des revendications 1 à 3, dans lequel le sujet est atteint de diabète de type II.

5. Le PYY ou un agoniste de celui-ci et le GLP-1 ou un agoniste de celui-ci pour une utilisation selon l'une quelconque des revendications 1 à 4, dans lequel l'administration périphérique comprend les administrations sous-cutanée, intraveineuse, intramusculaire, intranasale, transdermique ou sublinguale.

6. Le PYY ou un agoniste de celui-ci et le GLP-1 ou un agoniste de celui-ci pour une utilisation selon l'une quelconque des revendications 1 à 5, dans lequel le PYY ou un agoniste de celui-ci formulé pour une administration périphérique comprennent une formulation pour l'administration périphérique d'environ 45 à environ 135 pmol par kilogramme de poids corporel du sujet, ou d'environ 72 pmol par kilogramme de poids corporel du sujet.

7. Le PYY ou un agoniste de celui-ci et le GLP-1 ou un agoniste de celui-ci pour une utilisation selon l'une quelconque des revendications 1 à 5, dans lequel le PYY ou l'agoniste de celui-ci formulé pour une administration périphérique comprend une formulation convenant à pour une administration en une multitude de doses, dans lequel chaque dose de la multitude de doses comprend l'administration d'environ 0,5 à environ 135 pmol par kilogramme de poids corporel d'au moins environ 30 minutes avant un repas.

8. Le PYY ou un agoniste de celui-ci et le GLP-1 ou un agoniste de celui-ci pour une utilisation selon l'une quelconque des revendications 1 à 7, dans lequel le PYY ou l'agoniste de celui-ci est formulé pour une administration en une quantité suffisante pour diminuer l'apport calorique pour une période d'environ 2 à 12 heures.

9. Le PYY ou un agoniste de celui-ci et le GLP-1 ou un agoniste de celui-ci pour une utilisation selon l'une quelconque des revendications 1 à 5, dans lequel le PYY ou l'agoniste de celui-ci est formulé pour l'administration périphérique à une dose de
0,1 nmoles par kg de poids corporel du sujet,
0,2 nmoles par kg de poids corporel du sujet,
0,4 nmoles par kg de poids corporel du sujet,
0,6 nmoles par kg de poids corporel du sujet,
0,8 nmoles par kg de poids corporel du sujet,
1,0 nmole par kg de poids corporel du sujet,
1,2 nmoles par kg de poids corporel du sujet,
1,4 nmoles par kg de poids corporel du sujet,
1,6 nmoles par kg de poids corporel du sujet,
1,8 nmoles par kg de poids corporel du sujet,
2,0 nmoles par kg de poids corporel du sujet, ou
2,2 nmoles par kg de poids corporel du sujet.

10. Le PYY ou un agoniste de celui-ci et le GLP-1 ou un agoniste de celui-ci pour une utilisation selon l'une quelconque des revendications 1 à 9, dans lequel le PYY ou l'agoniste de celui-ci est modifié par une ou plusieurs amidation, glycosylation, acylation, sulfatation, phosphorylation, cyclisation, lipidisation ou pégylation.

11. Le PYY ou un agoniste de celui-ci et le GLP-1 ou un agoniste de celui-ci pour une utilisation selon l'une quelconque des revendications 1 à 10, dans lequel le GLP-1 ou l'agoniste de celui-ci comprend la séquence d'acides aminés de GLP-1 (7-36) (SEQ ID NO: 339) ou un variant de celui-ci qui est lie à un récepteur GLP-1.

12. Le PYY ou un agoniste de celui-ci et le GLP-1 ou un agoniste de celui-ci pour une utilisation selon l'une quelconque des revendications 1 à 11, dans lequel PYY est PYY₃₋₃₆ ou un variant de celui-ci.

13. Le PYY ou un agoniste de celui-ci et le GLP-1 ou un agoniste de celui-ci pour une utilisation selon l'une quelconque des revendications 1 à 12, dans lequel l'agoniste GLP-I est l'exendine-4 ou un de ses dérivés de celui-ci consistant en un agoniste du GLP-I.

14. Le PYY ou un agoniste de celui-ci et le GLP-1 ou un agoniste de celui-ci pour une utilisation selon l'une quelconque des revendications 1 à 13, convenant pour une administration en combinaison avec l'amfépramone (diéthylpropion), la phéntermine, le mazindol, la phenylpropanolamine, la fenfluramine, la dexfenfluramine ou la fluoxétine.

15. Le PYY ou un agoniste de celui-ci et le GLP-1 ou un agoniste de celui-ci pour une utilisation selon l'une quelconque des revendications 1 à 14, dans lequel le PYY ou un agoniste de celui-ci est formulé pour une administration en une quantité de 1 à 100 nmols, de 2 à 90 nmols, de 5 à 80 nmols, ou de 5 à 50 nmoles.

16. Un procédé non thérapeutique pour:
a) diminuer l'apport calorique ou la prise d'aliments,
b) réduire le gain de poids ou induire une perte de poids,
c) contrôler au moins l'une des conditions suivantes: l'appétit, la satiété et la faim,
d) maintenir le poids corporel désiré, l'indice de masse corporelle désiré, et/ou une apparence voulue et une bonne santé,
e) améliorer le profil lipidique
chez un sujet humain comprenant l'administration de peptide YY (PYY) ou d'un agoniste de celui-ci et du peptide-1 de type Glucagon (GLP-1) ou d'un agoniste de celui-ci,
dans lequel le PYY ou l'agoniste de celui-ci et GLP-1 ou l'agoniste de celui-ci sont sous une forme convenant à pour une administration périphérique,
dans lequel le PYY ou l'agoniste de celui-ci est destiné à une administration en une quantité molaire équivalente à une dose de PYY₃₋₃₆ de pas plus de 2,2 nmol par kg de poids corporel d'un sujet, et
dans lequel le sujet désire perdre du poids

17. Le procédé selon la revendication 16, dans lequel le PYY ou un agoniste de celui-ci et le GLP-1 ou un agoniste de celui-ci sont formulés pour une administration simultanée ou séquentielle.

18. Le procédé selon les revendications 16 ou 17, dans lequel le PYY et le GLP-I sont formulés pour une administration par différentes voies.

19. Le procédé selon l'une quelconque des revendications 16 à 18, dans lequel l'administration périphérique comprend les administrations sous-cutanée, intraveineuse, intramusculaire, intranasale, transdermique ou sublinguale.

20. Le procédé selon l'une quelconque des revendications 16 à 19, dans lequel le PYY ou l'agoniste de celui-ci, formulés pour une administration périphérique, comprennent une formulation pour administration périphérique d'environ 45 à environ 135 pmol par kilogramme de poids corporel du sujet ou d'environ 72 pmol par kilogramme de poids corporel du sujet.

21. Le procédé selon l'une quelconque des revendications 16 à 19, dans lequel le PYY ou l'agoniste de celui-ci, formulés pour une administration périphérique comprend une formulation convenant à pour une administration en une multitude de doses et en ce que chaque dose de la multitude de doses comprend l'administration d'environ 0,5 à environ 135 pmol par kilogramme de poids corporel d'au moins environ 30 minutes avant un repas.

22. Le procédé selon l'une quelconque des revendications 16 à 21, dans lequel le PYY ou l'agoniste de celui-ci est formulé pour une administration en une quantité suffisante pour diminuer l'apport calorique pour une période d'environ 2 à 12 heures.

23. Le procédé selon l'une quelconque des revendications 16 à 19, dans lequel le PYY ou l'agoniste de celui-ci est formulée pour l'administration périphérique en une dose de
0,1 nmoles par kg de poids corporel du sujet,
0,2 nmoles par kg de poids corporel du sujet,
0,4 nmoles par kg de poids corporel du sujet,
0,6 nmoles par kg de poids corporel du sujet,
0,8 nmoles par kg de poids corporel du sujet,
1,0 nmole par kg de poids corporel du sujet,
1,2 nmoles par kg de poids corporel du sujet,
1,4 nmoles par kg de poids corporel du sujet,
1,6 nmoles par kg de poids corporel du sujet,
1,8 nmoles par kg de poids corporel du sujet,
2,0 nmoles par kg de poids corporel du sujet, ou
2,2 nmoles par kg de poids corporel du sujet.

24. Le procédé selon l'une quelconque des revendications 16 à 23, dans lequel le PYY ou un agoniste de celui-ci est modifié par une ou plusieurs amidation, glycosylation, acylation, sulfatation, phosphorylation, cyclisation, lipidisation ou pégylation.

25. Le procédé selon l'une quelconque des revendications 16 à 24, dans lequel le GLP-1 ou l'agoniste de celui-ci comprend la séquence d'acides aminés de GLP-1 (7-36) (SEQ ID NO: 339) ou un variant de celui-ci qui est lie à un récepteur GLP-1.

26. Le procédé selon l'une quelconque des revendications 16 à 25, dans lequel PYY est PYY₃₋₃₆ ou un variant de celui-ci.

27. Le procédé selon l'une quelconque des revendications 16 à 24, dans lequel l'agoniste GLP-I est l'exendine-4 ou un de ses dérivés de celui-ci consistant en un agoniste du GLP-I.

28. Le procédé selon l'une quelconque des revendications 16 à 27, convenant pour une administration en combinaison avec l'amfépramone (diéthylpropion), la phéntermine, le mazindol, la phenylpropanolamine, la fenfluramine, la dexfenfluramine ou la fluoxétine.

29. Le procédé selon l'une quelconque des revendications 16 à 28, dans lequel le PYY ou l'agoniste de celui-ci est formulé pour une administration en une quantité de 1 à 100 nmols, de 2 à 90 nmols, de 5 à 80 nmols, ou de 5 à 50 nmoles.


### SEQUENCE LISTING

<110> Imperial College Innovations LTD
   oregon Health and Science University
<120> Modification of Feeding Behavior
<130> 9248 WO/JSvn
<150> GB 02 00507.2
   <151> 10-01-2002
<150> PCT/US02/31944
   <151> 24-09-2002
<160> 341
<170> PatentIn version 3.1
<210> 1
   <211> 36
   <212> PRT
   <213> Homo sapiens
<400> 1
   Tyr Pro Ile Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 2
   <211> 36
   <212> PRT
   <213> Homo sapiens
<400> 2
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35
<210> 3
   <211> 36
   <212> PRT
   <213> Homo sapiens
<400> 3
   Ala Ser Leu Glu Pro Glu Tyr Pro Gly Asp Asn Ala Thr Pro Glu Gln
   1               5                   10                  15
   Met Ala Gln Tyr Ala Ala Glu Leu Arg Arg Tyr Ile Asn Met Leu Thr
               20                  25                  30
   Arg Pro Arg Tyr
                   35
<210> 4
   <211> 3
   <212> PRT
   <213> Homo sapiens
<400> 4
<210> 5
   <211> 36
   <212> PRT
   <213> Rattus sp.
<400> 5
   Tyr Pro Ala Lys Pro Glu Ala Pro Gly Glu Asp Ala Ser Pro Glu Glu
   1               5                   10                  15
   Leu Ser Arg Tyr Tyr Ala Ser Leu Arg His Tyr Leu Asn Leu Val Thr
               20                  25                  30
   Arg Gln Arg Tyr
           35
<210> 6
   <211> 36
   <212> PRT
   <213> Sus sp.
<400> 6
   Tyr Pro Ala Lys Pro Glu Ala Pro Gly Glu Asp Ala Ser Pro Glu Glu
   1 5 10 15
   Leu Ser Arg Tyr Tyr Ala Ser Leu Arg His Tyr Leu Asn Leu Val Thr
   20 25 30
   Arg Gln Arg Tyr
   35
<210> 7
   <211> 36
   <212> PRT
   <213> Cavia porcellus
<400> 7
   Tyr Pro Ser Lys Pro Glu Ala Pro Gly Ser Asp Ala Ser Pro Glu Glu
   1 5 10 15
   Leu Ala Arg Tyr Tyr Ala Ser Leu Arg His Tyr Leu Asn Leu Val Thr
   20 25 30
   Arg Gln Arg Tyr
   35
<210> 8
   <211> 36
   <212> PRT
   <213> Rana sp.
<400> 8
   Tyr Pro Pro Lys Pro Glu Asn Pro Gly Glu Asp Ala Ser Pro Glu Glu
   1 5 10 15
   Met Thr Lys Tyr Leu Thr Ala Leu Arg His Tyr Ile Asn Leu Val Thr
   20 25 30
   Arg Gln Arg Tyr
   35
<210> 9
   <211> 36
   <212> PRT
   <213> Raja sp.
<400> 9
   Tyr Pro Pro Lys Pro Glu Asn Pro Gly Asp Asp Ala Ala Pro Glu Glu
   1 5 10 15
   Leu Ala Lys Tyr Tyr Ser Ala Leu Arg His Tyr Ile Asn Leu Ile Thr
   20 25 30
   Arg Gln Arg Tyr
   35
<210> 10
   <211> 36
   <212> PRT
   <213> Dogfish sp.
<400> 10
   Tyr Pro Pro Lys Pro Glu Asn Pro Gly Glu Asp Ala Pro Pro Glu Glu
   1               5                   10                  15
   Leu Ala Lys Tyr Tyr Ser Ala Leu Arg His Tyr Ile Asn Leu Ile Thr
               20                  25                  30
   Arg Gln Arg Tyr
           35
<210> 11
   <211> 36
   <212> PRT
   <213> Lampetra sp.
<400> 11
   Phe Pro Pro Lys Pro Asp Asn Pro Gly Asp Asn Ala Ser Pro Glu Gln
   1               5                   10                  15
   Met Ala Arg Tyr Lys Ala Ala Val Arg His Tyr Ile Asn Leu Ile Thr
               20                  25                  30
   Arg Gln Arg Tyr
           35
<210> 12
   <211> 36
   <212> PRT
   <213> Petromyzontidae gen. sp.
<400> 12
   Met Pro Pro Lys Pro Asp Asn Pro Ser Pro Asp Ala Ser Pro Glu Glu
   1               5                   10                  15
   Leu Ser Lys Tyr Met Leu Ala Val Arg Asn Tyr Ile Asn Leu Ile Thr
               20                  25                  30
   Arg Gln Arg Tyr
           35
<210> 13
   <211> 36
   <212> PRT
   <213> Rattus sp.
<400> 13
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35
<210> 14
   <211> 36
   <212> PRT
   <213> oryctolagus cuniculus
<400> 14
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35
<210> 15
   <211> 36
   <212> PRT
   <213> Canis familiaris
<400> 15
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35
<210> 16
   <211> 36
   <212> PRT
   <213> Sus sp.
<400> 16
   Tyr Pro Ser Lys Pro Asp Asn Pro Gly Glu Asp Ala Pro Ala Glu Asp
   1               5                   10                  15
   Leu Ala Arg Tyr Tyr Ser Ala Leu Arg His Tyr Ile Asn Leu Ile Thr
               20                  25                  30
   Arg Gln Arg Tyr
           35
<210> 17
   <211> 36
   <212> PRT
   <213> Bos taurus
<400> 17
   Tyr Pro Ser Lys Pro Asp Asn Pro Gly Glu Asp Ala Pro Ala Glu Asp
   1               5                   10                  15
   Leu Ala Arg Tyr Tyr Ser Ala Leu Arg His Tyr Ile Asn Leu Ile Thr
               20                  25                  30
   Arg Gln Arg Tyr
           35
<210> 18
   <211> 36
   <212> PRT
   <213> Ovis aries
<400> 18
   Tyr Pro Ser Lys Pro Asp Asn Pro Gly Asp Asp Ala Pro Ala Glu Asp
   1 5 10 15
   Leu Ala Arg Tyr Tyr Ser Ala Leu Arg His Tyr Ile Asn Leu Ile Thr
   20 25 30
   Arg Gln Arg Tyr
   35
<210> 19
   <211> 36
   <212> PRT
   <213> Cavia porcellus
<400> 19
   Tyr Pro Ser Lys Pro Asp Asn Pro Gly Glu Asp Ala Pro Ala Glu Asp
   1 5 10 15
   Met Ala Arg Tyr Tyr Ser Ala Leu Arg His Tyr Ile Asn Leu Ile Thr
   20 25 30
   Arg Gln Arg Tyr
   35
<210> 20
   <211> 36
   <212> PRT
   <213> Avian
<400> 20
   Tyr Pro Ser Lys Pro Asp Ser Pro Gly Glu Asp Ala Pro Ala Glu Asp
   1 5 10 15
   Met Ala Arg Tyr Tyr Ser Ala Leu Arg His Tyr Ile Asn Leu Ile Thr
   20 25 30
   Arg Gln Arg Tyr
   35
<210> 21
   <211> 36
   <212> PRT
   <213> Rana sp.
<400> 21
   Tyr Pro Ser Lys Pro Asp Asn Pro Gly Glu Asp Ala Pro Ala Glu Asp
   1               5                   10                  15
   Met Ala Lys Tyr Tyr Ser Ala Leu Arg His Tyr Ile Asn Leu Ile Thr
               20                  25                  30
   Arg Gln Arg Tyr
           35
<210> 22
   <211> 36
   <212> PRT
   <213> Carassius auratus
<400> 22
   Tyr Pro Thr Lys Pro Asp Asn Pro Gly Glu Gly Ala Pro Ala Glu Glu
   1               5                   10                  15
   Leu Ala Lys Tyr Tyr Ser Ala Leu Arg His Tyr Ile Asn Leu Ile Thr
               20                  25                  30
   Arg Gln Arg Tyr
           35
<210> 23
   <211> 36
   <212> PRT
   <213> Dogfish sp.
<400> 23
   Tyr Pro Ser Lys Pro Asp Asn Pro Gly Glu Gly Ala Pro Ala Glu Asp
   1               5                   10                  15
   Leu Ala Lys Tyr Tyr Ser Ala Leu Arg His Tyr Ile Asn Leu Ile Thr
               20                  25                  30
   Arg Gln Arg Tyr
           35
<210> 24
   <211> 36
   <212> PRT
   <213> Lampetra sp.
<400> 24
   Pro Pro Asn Lys Pro Asp Ser Pro Gly Glu Asp Ala Pro Ala Glu Asp
   1               5                   10                  15
   Leu Ala Arg Tyr Leu Ser Ala Val Arg His Tyr Ile Asn Leu Ile Thr
               20                  25                  30
   Arg Gln Arg Tyr
           35
<210> 25
   <211> 36
   <212> PRT
   <213> Ovis aries
<400> 25
   Ala Pro Leu Glu Pro Val Tyr Pro Gly Asp Asn Ala Thr Pro Glu Gln
   1               5                   10                  15
   Met Ala Gln Tyr Ala Ala Asp Leu Arg Arg Tyr Ile Asn Met Leu Thr
               20                  25                  30
   Arg Pro Arg Tyr
           35
<210> 26
   <211> 36
   <212> PRT
   <213> Sus sp.
<400> 26
   Ala Pro Leu Glu Pro Val Tyr Pro Gly Asp Asp Ala Thr Pro Glu Gln
   1               5                   10                  15
   Met Ala Gln Tyr Ala Ala Glu Leu Arg Arg Tyr Ile Asn Met Leu Thr
               20                  25                  30
   Arg Pro Arg Tyr
           35
<210> 27
   <211> 36
   <212> PRT
   <213> Canis familiaris
<400> 27
   Ala Pro Leu Glu Pro Val Tyr Pro Gly Asp Asp Ala Thr Pro Glu Gln
   1               5                   10                  15
   Met Ala Gln Tyr Ala Ala Glu Leu Arg Arg Tyr Ile Asn Met Leu Thr
               20                  25                  30
   Arg Pro Arg Tyr
           35
<210> 28
   <211> 36
   <212> PRT
   <213> Felis catus
<400> 28
   Ala Pro Leu Glu Pro Val Tyr Pro Gly Asp Asn Ala Thr Pro Glu Gln
   1               5                   10                  15
   Met Ala Gln Tyr Ala Ala Glu Leu Arg Arg Tyr Ile Asn Met Leu Thr
               20                  25                  30
   Arg Pro Arg Tyr
           35
<210> 29
   <211> 36
   <212> PRT
   <213> BOS taurus
<400> 29
   Ala Pro Leu Glu Pro Glu Tyr Pro Gly Asp Asn Ala Thr Pro Glu Gln
   1               5                   10                  15
   Met Ala Gln Tyr Ala Ala Glu Leu Arg Arg Tyr Ile Asn Met Leu Thr
               20                  25                  30
   Arg Pro Arg Tyr
           35
<210> 30
   <211> 36
   <212> PRT
   <213> Rattus sp.
<400> 30
   Ala Pro Leu Glu Pro Met Tyr Pro Gly Asp Tyr Ala Thr His Glu Gln
   1               5                   10                  15
   Arg Ala Gln Tyr Glu Thr Gln Leu Arg Arg Tyr Ile Asn Thr Leu Thr
               20                  25                  30
   Arg Pro Arg Tyr
           35
<210> 31
   <211> 36
   <212> PRT
   <213> Mus musculus
<400> 31
   Ala Pro Leu Glu Pro Met Tyr Pro Gly Asp Tyr Ala Thr Pro Glu Gln
   1               5                   10                  15
   Met Ala Gln Tyr Glu Thr Gln Leu Arg Arg Tyr Ile Asn Thr Leu Thr
               20                  25                  30
   Arg Pro Arg Tyr
           35
<210> 32
   <211> 37
   <212> PRT
   <213> cavia porcellus
<400> 32
   Ala Pro Leu Glu Pro Val Tyr Pro Gly Asp Asn Ala Thr Pro Glu Gln
   1               5                   10                  15
   Gln Met Ala Gln Tyr Ala Ala Glu Met Arg Arg Tyr Ile Asn Met Leu
   20 25 30
   Thr Arg Pro Arg Tyr
   35
<210> 33
   <211> 36
   <212> PRT
   <213> Gallus gallus
<400> 33
   Gly Pro Ser Gln Pro Thr Tyr Pro Gly Asp Asp Ala Pro Val Glu Asp
   1 5 10 15
   Leu Ile Arg Phe Tyr Asn Asp Leu Gln Gln Tyr Leu Asn Val Val Thr
   20 25 30
   Arg His Arg Tyr
   35
<210> 34
   <211> 36
   <212> PRT
   <213> Alligator sp.
<400> 34
   Thr Pro Leu Gln Pro Lys Tyr Pro Gly Asp Gly Ala Pro Val Glu Asp
   1 5 10 15
   Leu Ile Gln Phe Tyr Asn Asp Leu Gln Gln Tyr Leu Asn Val Val Thr
   20 25 30
   Arg Pro Arg Phe
   35
<210> 35
   <211> 36
   <212> PRT
   <213> Rana catesbeiana
<400> 35
   Ala Pro Ser Glu Pro His His Pro Gly Asp Gln Ala Thr Pro Asp Gln
   1 5 10 15
   Leu Ala Gln Tyr Tyr Ser Asp Leu Tyr Gln Tyr Ile Thr Phe Ile Thr
   20 25 30
   Arg Pro Arg Phe
   35
<210> 36
   <211> 12
   <212> PRT
   <213> Artificial Sequence
<220>
   <223> Polypeptide variation
<400> 36
   Lys His Tyr Leu Asn Leu Val Thr Arg Gln Arg Tyr
   1 5 10
<210> 37
   <211> 12
   <212> PRT
   <213> Artificial sequence
<220>
   <223> Polypeptide variation
<400> 37
   Arg His Thr Leu Asn Leu Val Thr Arg Gln Arg Tyr
   1 5 10
<210> 38
   <211> 12
   <212> PRT
   <213> Artificial sequence
<220>
   <223> Polypeptide variation
<400> 38
   Arg His Phe Leu Asn Leu Val Thr Arg Gln Arg Tyr
   1 5 10
<210> 39
   <211> 12
   <212> PRT
   <213> Artificial Sequence
<220>
   <223> Polypeptide variation
<400> 39
   Arg His Tyr Ile Asn Leu Val Thr Arg Gln Arg Tyr
   1 5 10
<210> 40
   <211> 12
   <212> PRT
   <213> Artificial Sequence
<220>
   <223> Polypeptide variation
<400> 40
   Arg His Tyr Val Asn Leu Val Thr Arg Gln Arg Tyr
   1 5 10
<210> 41
   <211> 12
   <212> PRT
   <213> Artificial Sequence
<220>
   <223> Polypeptide variation
<400> 41
   Arg His Tyr Leu Gln Leu Val Thr Arg Gln Arg Tyr
   1 5 10
<210> 42
   <211> 12
   <212> PRT
   <213> Artificial sequence
<220>
   <223> Polypeptide variation
<400> 42
   Arg His Tyr Leu Asn Ile Val Thr Arg Gln Arg Tyr
   1 5 10
<210> 43
   <211> 12
   <212> PRT
   <213> Artificial sequence
<220>
   <223> Polypeptide variation
<400> 43
   Arg His Tyr Leu Asn Val Val Thr Arg Gln Arg Tyr
   1 5 10
<210> 44
   <211> 12
   <212> PRT
   <213> Artificial sequence
<220>
   <223> Polypeptide variation
<400> 44
   Arg His Tyr Leu Asn Leu Ile Thr Arg Gln Arg Tyr
   1 5 10
<210> 45
   <211> 12
   <212> PRT
   <213> Artificial Sequence
<220>
   <223> Polypeptide variation
<400> 45
   Arg His Tyr Leu Asn Leu Leu Thr Arg Gln Arg Tyr
   1 5 10
<210> 46
   <211> 12
   <212> PRT
   <213> Artificial Sequence
<220>
   <223> Polypeptide variation
<400> 46
   Arg His Tyr Leu Asn Leu Val Ser Arg Gln Arg Tyr
   1 5 10
<210> 47
   <211> 12
   <212> PRT
   <213> Artificial Sequence
<220>
   <223> Polypeptide variation
<400> 47
   Arg His Tyr Leu Asn Leu Val Thr Lys Gln Arg Tyr
   1 5 10
<210> 48
   <211> 12
   <212> PRT
   <213> Artificial Sequence
<220>
   <223> Polypeptide variation
<400> 48
   Arg His Tyr Leu Asn Leu Val Thr Arg Asn Arg Tyr
   1 5 10
<210> 49
   <211> 12
   <212> PRT
   <213> Artificial Sequence
<220>
   <223> Polypeptide variation
<400> 49
   Arg His Tyr Leu Asn Leu Val Thr Arg Gln Lys Tyr
   1 5 10
<210> 50
   <211> 12
   <212> PRT
   <213> Artificial Sequence
<220>
   <223> Polypeptide variation
<400> 50
   Arg His Tyr Leu Asn Leu Val Thr Arg Gln Arg Thr
   1 5 10
<210> 51
   <211> 12
   <212> PRT
   <213> Artificial Sequence
<220>
   <223> Polypeptide variation
<400> 51
   Arg His Tyr Leu Asn Leu Val Thr Arg Gln Arg Phe
   1 5 10
<210> 52
   <211> 12
   <212> PRT
   <213> Artificial sequence
<220>
   <223> Polypeptide variation
<400> 52
   Lys His Thr Leu Asn Leu Val Thr Arg Gln Arg Tyr
   1 5 10
<210> 53
   <211> 12
   <212> PRT
   <213> Artificial sequence
<220>
   <223> Polypeptide variation
<400> 53
   Lys His Phe Leu Asn Leu Val Thr Arg Gln Arg Tyr
   1 5 10
<210> 54
   <211> 12
   <212> PRT
   <213> Artificial sequence
<220>
   <223> Polypeptide variation
<400> 54
   Lys His Tyr Ile Asn Leu Val Thr Arg Gln Arg Tyr
   1 5 10
<210> 55
   <211> 12
   <212> PRT
   <213> Artificial Sequence
<220>
   <223> Polypeptide variation
<400> 55
   Lys His Tyr Val Asn Leu Val Thr Arg Gln Arg Tyr
   1 5 10
<210> 56
   <211> 12
   <212> PRT
   <213> Artificial Sequence
<220>
   <223> Polypeptide variation
<400> 56
   Lys His Tyr Leu Gln Leu Val Thr Arg Gln Arg Tyr
   1 5 10
<210> 57
   <211> 12
   <212> PRT
   <213> Artificial Sequence
<220>
   <223> Polypeptide variation
<400> 57
   Lys His Tyr Leu Asn Ile Val Thr Arg Gln Arg Tyr
   1 5 10
<210> 58
   <211> 12
   <212> PRT
   <213> Artificial Sequence
<220>
   <223> Polypeptide variation
<400> 58
   Lys His Tyr Leu Asn Val Val Thr Arg Gln Arg Tyr
   1 5 10
<210> 59
   <211> 12
   <212> PRT
   <213> Artificial sequence
<220>
   <223> Polypeptide variation
<400> 59
   Lys His Tyr Leu Asn Leu Ile Thr Arg Gln Arg Tyr
   1 5 10
<210> 60
   <211> 12
   <212> PRT
   <213> Artificial Sequence
<220>
   <223> Polypeptide variation
<400> 60
   Lys His Tyr Leu Asn Leu Leu Thr Arg Gln Arg Tyr
   1 5 10
<210> 61
   <211> 12
   <212> PRT
   <213> Artificial Sequence
<220>
   <223> Polypeptide variation
<400> 61
   Lys His Tyr Leu Asn Leu Val Ser Arg Gln Arg Tyr
   1 5 10
<210> 62
   <211> 12
   <212> PRT
   <213> Artificial Sequence
<220>
   <223> Polypeptide variation
<400> 62
   Lys His Tyr Leu Asn Leu Val Thr Lys Gln Arg Tyr
   1 5 10
<210> 63
   <211> 12
   <212> PRT
   <213> Artificial Sequence
<220>
   <223> Polypeptide variation
<400> 63
   Lys His Tyr Leu Asn Leu Val Thr Arg Asn Arg Tyr
   1 5 10
<210> 64
   <211> 12
   <212> PRT
   <213> Artificial sequence
<220>
   <223> Polypeptide variation
<400> 64
   Lys His Tyr Leu Asn Leu Val Thr Arg Gln Lys Tyr
   1 5 10
<210> 65
   <211> 12
   <212> PRT
   <213> Artificial sequence
<220>
   <223> Polypeptide variation
<400> 65
   Lys His Tyr Leu Asn Leu Val Thr Arg Gln Arg Thr
   1 5 10
<210> 66
   <211> 12
   <212> PRT
   <213> Artificial sequence
<220>
   <223> Polypeptide variation
<400> 66
   Lys His Tyr Leu Asn Leu Val Thr Arg Gln Arg Phe
   1 5 10
<210> 67
   <211> 12
   <212> PRT
   <213> Artificial Sequence
<220>
   <223> Polypeptide variation
<400> 67
   Arg His Thr Ile Asn Leu Val Thr Arg Gln Arg Tyr
   1 5 10
<210> 68
   <211> 12
   <212> PRT
   <213> Artificial sequence
<220>
   <223> Polypeptide variation
<400> 68
   Arg His Thr Val Asn Leu Val Thr Arg Gln Arg Tyr
   1 5 10
<210> 69
   <211> 12
   <212> PRT
   <213> Artificial sequence
<220>
   <223> Polypeptide variation
<400> 69
   Arg His Thr Leu Gln Leu Val Thr Arg Gln Arg Tyr
   1 5 10
<210> 70
   <211> 12
   <212> PRT
   <213> Artificial Sequence
<220>
   <223> Polypeptide variation
<400> 70
   Arg His Thr Leu Asn Ile Val Thr Arg Gln Arg Tyr
   1 5 10
<210> 71
   <211> 12
   <212> PRT
   <213> Artificial Sequence
<220>
   <223> polypeptide variation
<400> 71
   Arg His Thr Leu Asn Val Val Thr Arg Gln Arg Tyr
   1 5 10
<210> 72
   <211> 12
   <212> PRT
   <213> Artificial sequence
<220>
   <223> Polypeptide variation
<400> 72
   Arg His Thr Leu Asn Leu Ile Thr Arg Gln Arg Tyr
   1 5 10
<210> 73
   <211> 12
   <212> PRT
   <213> Artificial Sequence
<220>
   <223> Polypeptide variation
<400> 73
   Arg His Thr Leu Asn Leu Leu Thr Arg Gln Arg Tyr
   1 5 10
<210> 74
   <211> 12
   <212> PRT
   <213> Artificial sequence
<220>
   <223> polypeptide variation
<400> 74
   Arg His Thr Leu Asn Leu Val Ser Arg Gln Arg Tyr
   1 5 10
<210> 75
   <211> 12
   <212> PRT
   <213> Artificial sequence
<220>
   <223> Polypeptide variation
<400> 75
   Arg His Thr Leu Asn Leu Val Thr Lys Gln Arg Tyr
   1 5 10
<210> 76
   <211> 12
   <212> PRT
   <213> Artificial Sequence
<220>
   <223> Polypeptide variation
<400> 76
   Arg His Thr Leu Asn Leu Val Thr Arg Asn Arg Tyr
   1 5 10
<210> 77
   <211> 12
   <212> PRT
   <213> Artificial Sequence
<220>
   <223> Polypeptide variation
<400> 77
   Arg His Thr Leu Asn Leu Val Thr Arg Gln Lys Tyr
   1 5 10
<210> 78
   <211> 12
   <212> PRT
   <213> Artificial sequence
<220>
   <223> Polypeptide variation
<400> 78
   Arg His Thr Leu Asn Leu Val Thr Arg Gln Arg Thr
   1 5 10
<210> 79
   <211> 12
   <212> PRT
   <213> Artificial sequence
<220>
   <223> Polypeptide variation
<400> 79
   Arg His Thr Leu Asn Leu Val Thr Arg Gln Arg Phe
   1 5 10
<210> 80
   <211> 12
   <212> PRT
   <213> Artificial Sequence
<220>
   <223> Polypeptide variation
<400> 80
   Arg His Phe Ile Asn Leu Val Thr Arg Gln Arg Tyr
   1               5                   10
<210> 81
   <211> 12
   <212> PRT
   <213> Artificial Sequence
<220>
   <223> Polypeptide variation
<400> 81
   Arg His Phe Val Asn Leu Val Thr Arg Gln Arg Tyr
   1 5 10
<210> 82
   <211> 12
   <212> PRT
   <213> Artificial Sequence
<220>
   <223> Polypeptide variation
<400> 82
   Arg His Phe Leu Gln Leu Val Thr Arg Gln Arg Tyr
   1 5 10
<210> 83
   <211> 12
   <212> PRT
   <213> Artificial Sequence
<220>
   <223> Polypeptide variation
<400> 83
   Arg His Phe Leu Asn Ile Val Thr Arg Gln Arg Tyr
   1 5 10
<210> 84
   <211> 12
   <212> PRT
   <213> Artificial Sequence
<220>
   <223> Polypeptide variation
<400> 84
   Arg His Phe Leu Asn Val Val Thr Arg Gln Arg Tyr
   1 5 10
<210> 85
   <211> 12
   <212> PRT
   <213> Artificial sequence
<220>
   <223> Polypeptide variation
<400> 85
   Arg His Phe Leu Asn Leu Ile Thr Arg Gln Arg Tyr
   1               5                   10
<210> 86
   <211> 12
   <212> PRT
   <213> Artificial Sequence
<220>
   <223> Polypeptide variation
<400> 86
   Arg His Phe Leu Asn Leu Leu Thr Arg Gln Arg Tyr
   1               5                   10
<210> 87
   <211> 12
   <212> PRT
   <213> Artificial sequence
<220>
   <223> Polypeptide variation
<400> 87
   Arg His Phe Leu Asn Leu Val Ser Arg Gln Arg Tyr
   1               5                   10
<210> 88
   <211> 12
   <212> PRT
   <213> Artificial Sequence
<220>
   <223> Polypeptide variation
<400> 88
   Arg His Phe Leu Asn Leu Val Thr Lys Gln Arg Tyr
   1               5                   10
<210> 89
   <211> 12
   <212> PRT
   <213> Artificial Sequence
<220>
   <223> Polypeptide variation
<400> 89
   Arg His Phe Leu Asn Leu Val Thr Arg Asn Arg Tyr
   1 5 10
<210> 90
   <211> 12
   <212> PRT
   <213> Artificial sequence
<220>
   <223> Polypeptide variation
<400> 90
   Arg His Phe Leu Asn Leu Val Thr Arg Gln Lys Tyr
   1 5 10
<210> 91
   <211> 12
   <212> PRT
   <213> Artificial Sequence
<220>
   <223> Polypeptide variation
<400> 91
   Arg His Phe Leu Asn Leu Val Thr Arg Gln Arg Thr
   1 5 10
<210> 92
   <211> 12
   <212> PRT
   <213> Artificial Sequence
<220>
   <223> Polypeptide variation
<400> 92
   Arg His Phe Leu Asn Leu Val Thr Arg Gln Arg Phe
   1 5 10
<210> 93
   <211> 12
   <212> PRT
   <213> Artificial sequence
<220>
   <223> Polypeptide variation
<400> 93
   Arg His Tyr Leu Gln Ile Val Thr Arg Gln Arg Tyr
   1 5 10
<210> 94
   <211> 12
   <212> PRT
   <213> Artificial Sequence
<220>
   <223> Polypeptide variation
<400> 94
   Arg His Tyr Leu Gln Val Val Thr Arg Gln Arg Tyr
   1 5 10
<210> 95
   <211> 12
   <212> PRT
   <213> Artificial sequence
<220>
   <223> Polypeptide variation
<400> 95
   Arg His Tyr Leu Gln Leu Ile Thr Arg Gln Arg Tyr
   1 5 10
<210> 96
   <211> 12
   <212> PRT
   <213> Artificial Sequence
<220>
   <223> Polypeptide variation
<400> 96
   Arg His Tyr Leu Gln Leu Leu Thr Arg Gln Arg Tyr
   1 5 10
<210> 97
   <211> 12
   <212> PRT
   <213> Artificial Sequence
<220>
   <223> Polypeptide variation
<400> 97
   Arg His Tyr Leu Gln Leu Val Ser Arg Gln Arg Tyr
   1 5 10
<210> 98
   <211> 12
   <212> PRT
   <213> Artificial Sequence
<220>
   <223> Polypeptide variation
<400> 98
   Arg His Tyr Leu Gln Leu Val Thr Lys Gln Arg Tyr
   1 5 10
<210> 99
   <211> 12
   <212> PRT
   <213> Artificial Sequence
<220>
   <223> Polypeptide variation
<400> 99
   Arg His Tyr Leu Gln Leu Val Thr Arg Asn Arg Tyr
   1 5 10
<210> 100
   <211> 12
   <212> PRT
   <213> Artificial Sequence
<220>
   <223> Polypeptide variation
<400> 100
   Arg His Tyr Leu Gln Leu Val Thr Arg Gln Lys Tyr
   1 5 10
<210> 101
   <211> 12
   <212> PRT
   <213> Artificial Sequence
<220>
   <223> Polypeptide variation
<400> 101
   Arg His Tyr Leu Gln Leu Val Thr Arg Gln Arg Thr
   1 5 10
<210> 102
   <211> 12
   <212> PRT
   <213> Artificial Sequence
<220>
   <223> Polypeptide variation
<400> 102
   Arg His Tyr Leu Gln Leu Val Thr Arg Gln Arg Phe
   1 5 10
<210> 103
   <211> 12
   <212> PRT
   <213> Artificial sequence
<220>
   <223> Polypeptide variation
<400> 103
   Arg His Tyr Leu Asn Ile Ile Thr Arg Gln Arg Tyr
   1 5 10
<210> 104
   <211> 12
   <212> PRT
   <213> Artificial Sequence
<220>
   <223> polypeptide variation
<400> 104
   Arg His Tyr Leu Asn Ile Leu Thr Arg Gln Arg Tyr
   1 5 10
<210> 105
   <211> 12
   <212> PRT
   <213> Artificial sequence
<220>
   <223> Polypeptide variation
<400> 105
   Arg His Tyr Leu Asn Ile Val Ser Arg Gln Arg Tyr
   1 5 10
<210> 106
   <211> 12
   <212> PRT
   <213> Artificial Sequence
<220>
   <223> Polypeptide variation
<400> 106
   Arg His Tyr Leu Asn Ile Val Thr Lys Gln Arg Tyr
   1 5 10
<210> 107
   <211> 12
   <212> PRT
   <213> Artificial Sequence
<220>
   <223> Polypeptide variation
<400> 107
   Arg His Tyr Leu Asn Ile Val Thr Arg Asn Arg Tyr
   1 5 10
<210> 108
   <211> 12
   <212> PRT
   <213> Artificial Sequence
<220>
   <223> Polypeptide variation
<400> 108
   Arg His Tyr Leu Asn Ile Val Thr Arg Gln Lys Tyr
   1 5 10
<210> 109
   <211> 12
   <212> PRT
   <213> Artificial Sequence
<220>
   <223> Polypeptide variation
<400> 109
   Arg His Tyr Leu Asn Ile Val Thr Arg Gln Arg Thr
   1 5 10
<210> 110
   <211> 12
   <212> PRT
   <213> Artificial Sequence
<220>
   <223> Polypeptide variation
<400> 110
   Arg His Tyr Leu Asn Ile Val Thr Arg Gln Arg Phe
   1 5 10
<210> 111
   <211> 12
   <212> PRT
   <213> Artificial Sequence
<220>
   <223> Polypeptide variation
<400> 111
   Arg His Tyr Leu Asn Val Ile Thr Arg Gln Arg Tyr
   1 5 10
<210> 112
   <211> 12
   <212> PRT
   <213> Artificial Sequence
<220>
   <223> Polypeptide variation
<400> 112
   Arg His Tyr Leu Asn Val Leu Thr Arg Gln Arg Tyr
   1 5 10
<210> 113
   <211> 12
   <212> PRT
   <213> Artificial Sequence
<220>
   <223> Polypeptide variation
<400> 113
   Arg His Tyr Leu Asn Val Val Ser Arg Gln Arg Tyr
   1 5 10
<210> 114
   <211> 12
   <212> PRT
   <213> Artificial sequence
<220>
   <223> Polypeptide variation
<400> 114
   Arg His Tyr Leu Asn Val Val Thr Lys Gln Arg Tyr
   1 5 10
<210> 115
   <211> 12
   <212> PRT
   <213> Artificial Sequence
<220>
   <223> Polypeptide variation
<400> 115
   Arg His Tyr Leu Asn Val Val Thr Arg Asn Arg Tyr
   1 5 10
<210> 116
   <211> 12
   <212> PRT
   <213> Artificial sequence
<220>
   <223> Polypeptide variation
<400> 116
   Arg His Tyr Leu Asn Val Val Thr Arg Gln Lys Tyr
   1               5                   10
<210> 117
   <211> 12
   <212> PRT
   <213> Artificial Sequence
<220>
   <223> Polypeptide variation
<400> 117
   Arg His Tyr Leu Asn Val Val Thr Arg Gln Arg Thr
   1               5                   10
<210> 118
   <211> 12
   <212> PRT
   <213> Artificial Sequence
<220>
   <223> Polypeptide variation
<400> 118
   Arg His Tyr Leu Asn Val Val Thr Arg Gln Arg Phe
   1               5                   10
<210> 119
   <211> 12
   <212> PRT
   <213> Artificial sequence
<220>
   <223> Polypeptide variation
<400> 119
   Arg His Tyr Leu Asn Leu Ile Ser Arg Gln Arg Tyr
   1               5                   10
<210> 120
   <211> 12
   <212> PRT
   <213> Artificial sequence
<220>
   <223> Polypeptide variation
<400> 120
   Arg His Tyr Leu Asn Leu Ile Thr Lys Gln Arg Tyr
   1               5                   10
<210> 121
   <211> 12
   <212> PRT
   <213> Artificial Sequence
<220>
   <223> Polypeptide variation
<400> 121
   Arg His Tyr Leu Asn Leu Ile Thr Arg Asn Arg Tyr
   1               5                   10
<210> 122
   <211> 12
   <212> PRT
   <213> Artificial Sequence
<220>
   <223> Polypeptide variation
<400> 122
   Arg His Tyr Leu Asn Leu Ile Thr Arg Gln Lys Tyr
   1               5                   10
<210> 123
   <211> 12
   <212> PRT
   <213> Artificial Sequence
<220>
   <223> Polypeptide variation
<400> 123
   Arg His Tyr Leu Asn Leu Ile Thr Arg Gln Arg Thr
   1               5                   10
<210> 124
   <211> 12
   <212> PRT
   <213> Artificial sequence
<220>
   <223> Polypeptide variation
<400> 124
   Arg His Tyr Leu Asn Leu Ile Thr Arg Gln Arg Phe
   1               5                   10
<210> 125
   <211> 12
   <212> PRT
   <213> Artificial sequence
<220>
   <223> Polypeptide variation
<400> 125
   Arg His Tyr Leu Asn Leu Leu Ser Arg Gln Arg Tyr
   1               5                   10
<210> 126
   <211> 12
   <212> PRT
   <213> Artificial sequence
<220>
   <223> Polypeptide variation
<400> 126
   Arg His Tyr Leu Asn Leu Leu Thr Lys Gln Arg Tyr
   1               5                   10
<210> 127
   <211> 12
   <212> PRT
   <213> Artificial Sequence
<220>
   <223> Polypeptide variation
<400> 127
   Arg His Tyr Leu Asn Leu Leu Thr Arg Asn Arg Tyr
   1               5                   10
<210> 128
   <211> 12
   <212> PRT
   <213> Artificial sequence
<220>
   <223> Polypeptide variation
<400> 128
   Arg His Tyr Leu Asn Leu Leu Thr Arg Gln Lys Tyr
   1               5                   10
<210> 129
   <211> 12
   <212> PRT
   <213> Artificial sequence
<220>
   <223> Polypeptide variation
<400> 129
   Arg His Tyr Leu Asn Leu Leu Thr Arg Gln Arg Thr
   1 5 10
<210> 130
   <211> 12
   <212> PRT
   <213> Artificial Sequence
<220>
   <223> Polypeptide variation
<400> 130
   Arg His Tyr Leu Asn Leu Leu Thr Arg Gln Arg Phe
   1 5 10
<210> 131
   <211> 12
   <212> PRT
   <213> Artificial sequence
<220>
   <223> Polypeptide variation
<400> 131
   Arg His Tyr Leu Asn Leu Val Ser Lys Gln Arg Tyr
   1 5 10
<210> 132
   <211> 12
   <212> PRT
   <213> Artificial Sequence
<220>
   <223> Polypeptide variation
<400> 132
   Arg His Tyr Leu Asn Leu Val Ser Arg Asn Arg Tyr
   1 5 10
<210> 133
   <211> 12
   <212> PRT
   <213> Artificial Sequence
<220>
   <223> Polypeptide variation
<400> 133
   Arg His Tyr Leu Asn Leu Val Ser Arg Gln Lys Tyr
   1 5 10
<210> 134
   <211> 12
   <212> PRT
   <213> Artificial sequence
<220>
   <223> Polypeptide variation
<400> 134
   Arg His Tyr Leu Asn Leu Val Ser Arg Gln Arg Thr
   1 5 10
<210> 135
   <211> 12
   <212> PRT
   <213> Artificial sequence
<220>
   <223> Polypeptide variation
<400> 135
   Arg His Tyr Leu Asn Leu Val Ser Arg Gln Arg Tyr
   1 5 10
<210> 136
   <211> 12
   <212> PRT
   <213> Artificial sequence
<220>
   <223> Polypeptide variation
<400> 136
   Arg His Tyr Leu Asn Leu Val Thr Lys Asn Arg Tyr
   1 5 10
<210> 137
   <211> 12
   <212> PRT
   <213> Artificial Sequence
<220>
   <223> Polypeptide variation
<400> 137
   Arg His Tyr Leu Asn Leu Val Thr Lys Gln Lys Tyr
   1 5 10
<210> 138
   <211> 12
   <212> PRT
   <213> Artificial Sequence
<220>
   <223> Polypeptide variation
<400> 138
   Arg His Tyr Leu Asn Leu Val Thr Lys Gln Arg Thr
   1 5 10
<210> 139
   <211> 12
   <212> PART
   <213> Artificial Sequence
<220>
   <223> Polypeptide variation
<400> 139
   Arg His Tyr Leu Asn Leu Val Thr Lys Gln Arg Phe
   1 5 10
<210> 140
   <211> 12
   <212> PRT
   <213> Artificial Sequence
<220>
   <223> Polypeptide variation
<400> 140
   Arg His Tyr Leu Asn Leu Val Thr Arg Asn Lys Tyr
   1 5 10
<210> 141
   <211> 12
   <212> PRT
   <213> Artificial sequence
<220>
   <223> Polypeptide variation
<400> 141
   Arg His Tyr Leu Asn Leu Val Thr Arg Asn Arg Thr
   1 5 10
<210> 142
   <211> 12
   <212> PRT
   <213> Artificial Sequence
<220>
   <223> Polypeptide variation
<400> 142
   Arg His Tyr Leu Asn Leu Val Thr Arg Asn Arg Phe
   1 5 10
<210> 143
   <211> 12
   <212> PRT
   <213> Artificial sequence
<220>
   <223> Polypeptide variation
<400> 143
   Arg His Tyr Leu Asn Leu Val Thr Arg Gln Lys Thr
   1 5 10
<210> 144
   <211> 12
   <212> PRT
   <213> Artificial sequence
<220>
   <223> Polypeptide variation
<400> 144
   Arg His Tyr Leu Asn Leu Val Thr Arg Gln Lys Phe
   1 5 10
<210> 145
   <211> 13
   <212> PRT
   <213> Artificial sequence
<220>
   <223> Polypeptide variation
<400> 145
   Leu Arg His Tyr Leu Asn Leu Val Thr Arg Gln Arg Tyr
   1 5 10
<210> 146
   <211> 13
   <212> PRT
   <213> Artificial Sequence
<220>
   <223> Polypeptide variation
<400> 146
   Ile Arg His Tyr Leu Asn Leu Val Thr Arg Gln Arg Tyr
   1 5 10
<210> 147
   <211> 13
   <212> PRT
   <213> Artificial Sequence
<220>
   <223> Polypeptide variation
<400> 147
   Val Arg His Tyr Leu Asn Leu Val Thr Arg Gln Arg Tyr
   1               5                   10
<210> 148
   <211> 14
   <212> PRT
   <213> Artificial sequence
<220>
   <223> Polypeptide variation
<400> 148
   Ser Leu Arg His Tyr Leu Asn Leu Val Thr Arg Gln Arg Tyr
   1               5                   10
<210> 149
   <211> 14
   <212> PRT
   <213> Artificial Sequence
<220>
   <223> Polypeptide variation
<400> 149
   Thr Leu Arg His Tyr Leu Asn Leu Val Thr Arg Gln Arg Tyr
   1               5                   10
<210> 150
   <211> 15
   <212> PRT
   <213> Artificial sequence
<220>
   <223> Polypeptide variation
<400> 150
   Ala Ser Leu Arg His Tyr Leu Asn Leu Val Thr Arg Gln Arg Tyr
   1               5                   10                  15
<210> 151
   <211> 15
   <212> PRT
   <213> Artificial Sequence
<220>
   <223> Polypeptide variation
<400> 151
   Ser Ser Leu Arg His Tyr Leu Asn Leu Val Thr Arg Gln Arg Tyr
   1               5                   10                  15
<210> 152
   <211> 16
   <212> PRT
   <213> Artificial Sequence
<220>
   <223> Polypeptide variation
<400> 152
   Tyr Ala Ser Leu Arg His Tyr Leu Asn Leu Val Thr Arg Gln Arg Tyr
   1               5                   10                  15
<210> 153
   <211> 16
   <212> PRT
   <213> Artificial sequence
<220>
   <223> Polypeptide variation
<400> 153
   Thr Ala Ser Leu Arg His Tyr Leu Asn Leu Val Thr Arg Gln Arg Tyr
   1               5                   10                  15
<210> 154
   <211> 16
   <212> PRT
   <213> Artificial Sequence
<220>
   <223> Polypeptide variation
<400> 154
   Phe Ala Ser Leu Arg His Tyr Leu Asn Leu Val Thr Arg Gln Arg Tyr
   1               5                   10                  15
<210> 155
   <211> 17
   <212> PRT
   <213> Artificial Sequence
<220>
   <223> Polypeptide variation
<400> 155
   Tyr Tyr Ala Ser Leu Arg His Tyr Leu Asn Leu Val Thr Arg Gln Arg
   1 5 10 15
   Tyr
<210> 156
   <211> 17
   <212> PRT
   <213> Artificial Sequence
<220>
   <223> Polypeptide variation
<400> 156
   Thr Tyr Ala Ser Leu Arg His Tyr Leu Asn Leu Val Thr Arg Gln Arg
   1 5 10 15
   Tyr
<210> 157
   <211> 17
   <212> PRT
   <213> Artificial sequence
<220>
   <223> Polypeptide variation
<400> 157
   Phe Tyr Ala Ser Leu Arg His Tyr Leu Asn Leu Val Thr Arg Gln Arg
   1 5 10 15
   Tyr
<210> 158
   <211> 18
   <212> PRT
   <213> Artificial Sequence
<220>
   <223> Polypeptide variation
<400> 158
   Arg Tyr Tyr Ala Ser Leu Arg His Tyr Leu Asn Leu Val Thr Arg Gln
   1 5 10 15
   Arg Tyr
<210> 159
   <211> 18
   <212> PRT
   <213> Artificial Sequence
<220>
   <223> Polypeptide variation
<400> 159
   Lys Tyr Tyr Ala Ser Leu Arg His Tyr Leu Asn Leu Val Thr Arg Gln
   1 5 10 15
   Arg Tyr
<210> 160
   <211> 19
   <212> PRT
   <213> Artificial Sequence
<220>
   <223> Polypeptide variation
<400> 160
   Asn Arg Tyr Tyr Ala Ser Leu Arg His Tyr Leu Asn Leu Val Thr Arg
   1 5 10 15
   Gln Arg Tyr
<210> 161
   <211> 19
   <212> PRT
   <213> Artificial Sequence
<220>
   <223> Polypeptide variation
<400> 161
   Gln Arg Tyr Tyr Ala Ser Leu Arg His Tyr Leu Asn Leu Val Thr Arg
   1 5 10 15
   Gln Arg Tyr
<210> 162
   <211> 20
   <212> PRT
   <213> Artificial Sequence
<220>
   <223> Polypeptide variation
<400> 162
   Leu Asn Arg Tyr Tyr Ala Ser Leu Arg His Tyr Leu Asn Leu Val Thr
   1 5 10 15
   Arg Gln Arg Tyr
   20
<210> 163
   <211> 20
   <212> PRT
   <213> Artificial sequence
<220>
   <223> Polypeptide variation
<400> 163
   Ile Asn Arg Tyr Tyr Ala Ser Leu Arg His Tyr Leu Asn Leu Val Thr
   1 5 10 15
   Arg Gln Arg Tyr
   20
<210> 164
   <211> 20
   <212> PRT
   <213> Artificial Sequence
<220>
   <223> Polypeptide variation
<400> 164
   Val Asn Arg Tyr Tyr Ala Ser Leu Arg His Tyr Leu Asn Leu Val Thr
   1 5 10 15
   Arg Gln Arg Tyr
   20
<210> 165
   <211> 21
   <212> PRT
   <213> Artificial Sequence
<220>
   <223> Polypeptide variation
<400> 165
   Glu Leu Asn Arg Tyr Tyr Ala Ser Leu Arg His Tyr Leu Asn Leu Val
   1 5 10 15
   Thr Arg Gln Arg Tyr
   20
<210> 166
   <211> 21
   <212> PRT
   <213> Artificial Sequence
<220>
   <223> Polypeptide variation
<400> 166
   Asp Leu Asn Arg Tyr Tyr Ala Ser Leu Arg His Tyr Leu Asn Leu Val
   1 5 10 15
   Thr Arg Gln Arg Tyr
   20
<210> 167
   <211> 22
   <212> PRT
   <213> Artificial Sequence
<220>
   <223> Polypeptide variation
<400> 167
   Glu Glu Leu Asn Arg Tyr Tyr Ala Ser Leu Arg His Tyr Leu Asn Leu
   1 5 10 15
   Val Thr Arg Gln Arg Tyr
   20
<210> 168
   <211> 22
   <212> PRT
   <213> Artificial Sequence
<220>
   <223> Polypeptide variation
<400> 168
   Asp Glu Leu Asn Arg Tyr Tyr Ala Ser Leu Arg His Tyr Leu Asn Leu
   1 5 10 15
   Val Thr Arg Gln Arg Tyr
   20
<210> 169
   <211> 23
   <212> PRT
   <213> Artificial Sequence
<220>
   <223> Polypeptide variation
<400> 169
   Pro Glu Glu Leu Asn Arg Tyr Tyr Ala Ser Leu Arg His Tyr Leu Asn
   1 5 10 15
   Leu Val Thr Arg Gln Arg Tyr
   20
<210> 170
   <211> 24
   <212> PRT
   <213> Artificial sequence
<220>
   <223> Polypeptide variation
<400> 170
   Ser Pro Glu Glu Leu Asn Arg Tyr Tyr Ala Ser Leu Arg His Tyr Leu
   1 5 10 15
   Asn Leu Val Thr Arg Gln Arg Tyr
   20
<210> 171
   <211> 24
   <212> PRT
   <213> Artificial sequence
<220>
   <223> Polypeptide variation
<400> 171
   Thr Pro Glu Glu Leu Asn Arg Tyr Tyr Ala Ser Leu Arg His Tyr Leu
   1 5 10 15
   Asn Leu Val Thr Arg Gln Arg Tyr
   20
<210> 172
   <211> 25
   <212> PRT
   <213> Artificial Sequence
<220>
   <223> Polypeptide variation
<400> 172
   Ala Ser Pro Glu Glu Leu Asn Arg Tyr Tyr Ala Ser Leu Arg His Tyr
   1 5 10 15
   Leu Asn Leu Val Thr Arg Gln Arg Tyr
   20 25
<210> 173
   <211> 25
   <212> PRT
   <213> Artificial Sequence
<220>
   <223> Polypeptide variation
<400> 173
   Ser Ser Pro Glu Glu Leu Asn Arg Tyr Tyr Ala Ser Leu Arg His Tyr
   1 5 10 15
   Leu Asn Leu Val Thr Arg Gln Arg Tyr
   20 25
<210> 174
   <211> 26
   <212> PRT
   <213> Artificial Sequence
<220>
   <223> Polypeptide variation
<400> 174
   Asp Ala Ser Pro Glu Glu Leu Asn Arg Tyr Tyr Ala Ser Leu Arg His
   1               5                   10                  15
   Tyr Leu Asn Leu Val Thr Arg Gln Arg Tyr
               20                  25
<210> 175
   <211> 26
   <212> PRT
   <213> Artificial sequence
<220>
   <223> Polypeptide variation
<400> 175
   Glu Ala Ser Pro Glu Glu Leu Asn Arg Tyr Tyr Ala Ser Leu Arg His
   1               5                   10                  15
   Tyr Leu Asn Leu Val Thr Arg Gln Arg Tyr
               20                  25
<210> 176
   <211> 27
   <212> PRT
   <213> Artificial sequence
<220>
   <223> Polypeptide variation
<400> 176
   Glu Asp Ala Ser Pro Glu Glu Leu Asn Arg Tyr Tyr Ala Ser Leu Arg
   1               5                   10                  15
   His Tyr Leu Asn Leu Val Thr Arg Gln Arg Tyr
               20                  25
<210> 177
   <211> 27
   <212> PRT
   <213> Artificial Sequence
<220>
   <223> Polypeptide variation
<400> 177
   Asp Asp Ala Ser Pro Glu Glu Leu Asn Arg Tyr Tyr Ala Ser Leu Arg
   1 5 10 15
   His Tyr Leu Asn Leu Val Thr Arg Gln Arg Tyr
   20 25
<210> 178
   <211> 28
   <212> PRT
   <213> Artificial sequence
<220>
   <223> Polypeptide variation
<400> 178
   Gly Glu Asp Ala Ser Pro Glu Glu Leu Asn Arg Tyr Tyr Ala Ser Leu
   1 5 10 15
   Arg His Tyr Leu Asn Leu Val Thr Arg Gln Arg Tyr
   20 25
<210> 179
   <211> 29
   <212> PRT
   <213> Artificial sequence
<220>
   <223> Polypeptide variation
<400> 179
   Pro Gly Glu Asp Ala Ser Pro Glu Glu Leu Asn Arg Tyr Tyr Ala Ser
   1 5 10 15
   Leu Arg His Tyr Leu Asn Leu Val Thr Arg Gln Arg Tyr
   20 25
<210> 180
   <211> 30
   <212> PRT
   <213> Artificial Sequence
<220>
   <223> Polypeptide variation
<400> 180
   Ala Pro Gly Glu Asp Ala Ser Pro Glu Glu Leu Asn Arg Tyr Tyr Ala
   1 5 10 15
   Ser Leu Arg His Tyr Leu Asn Leu Val Thr Arg Gln Arg Tyr
   20 25 30
<210> 181
   <211> 30
   <212> PRT
   <213> Artificial Sequence
<220>
   <223> Polypeptide variation
<400> 181 .
   Ser Pro Gly Glu Asp Ala Ser Pro Glu Glu Leu Asn Arg Tyr Tyr Ala
   1 5 10 15
   Ser Leu Arg His Tyr Leu Asn Leu Val Thr Arg Gln Arg Tyr
   20 25 30
<210> 182
   <211> 31
   <212> PRT
   <213> Artificial sequence
<220>
   <223> Polypeptide variation
<400> 182
   Glu Ala Pro Gly Glu Asp Ala Ser Pro Glu Glu Leu Asn Arg Tyr Tyr
   1 5 10 15
   Ala Ser Leu Arg His Tyr Leu Asn Leu Val Thr Arg Gln Arg Tyr
   20 25 30
<210> 183
   <211> 31
   <212> PRT
   <213> Artificial Sequence
<220>
   <223> Polypeptide variation
<400> 183
   Asp Ala Pro Gly Glu Asp Ala Ser Pro Glu Glu Leu Asn Arg Tyr Tyr
   1 5 10 15
   Ala Ser Leu Arg His Tyr Leu Asn Leu Val Thr Arg Gln Arg Tyr
   20 25 30
<210> 184
   <211> 32
   <212> PRT
   <213> Artificial Sequence
<220>
   <223> Polypeptide variation
<400> 184
   Pro Glu Ala Pro Gly Glu Asp Ala Ser Pro Glu Glu Leu Asn Arg Tyr
   1 5 10 15
   Tyr Ala Ser Leu Arg His Tyr Leu Asn Leu Val Thr Arg Gln Arg Tyr
   20 25 30
<210> 185
   <211> 33
   <212> PRT
   <213> Artificial Sequence
<220>
   <223> Polypeptide variation
<400> 185
   Lys Pro Glu Ala Pro Gly Glu Asp Ala Ser Pro Glu Glu Leu Asn Arg
   1 5 10 15
   Tyr Tyr Ala Ser Leu Arg His Tyr Leu Asn Leu Val Thr Arg Gln Arg
   20 25 30
   Tyr
<210> 186
   <211> 33
   <212> PRT
   <213> Artificial sequence
<220>
   <223> Polypeptide variation
<400> 186
   Arg Pro Glu Ala Pro Gly Glu Asp Ala Ser Pro Glu Glu Leu Asn Arg
   1 5 10 15
   Tyr Tyr Ala Ser Leu Arg His Tyr Leu Asn Leu Val Thr Arg Gln Arg
   20 25 30
   Tyr
<210> 187
   <211> 33
   <212> PRT
   <213> Artificial sequence
<220>
   <223> Polypeptide variation
<400> 187
   Gln Pro Glu Ala Pro Gly Glu Asp Ala Ser Pro Glu Glu Leu Asn Arg
   1 5 10 15
   Tyr Tyr Ala Ser Leu Arg His Tyr Leu Asn Leu Val Thr Arg Gln Arg
   20 25 30
   Tyr
<210> 188
   <211> 33
   <212> PRT
   <213> Artificial Sequence
<220>
   <223> Polypeptide variation
<400> 188
   Asn Pro Glu Ala Pro Gly Glu Asp Ala Ser Pro Glu Glu Leu Asn Arg
   1 5 10 15
   Tyr Tyr Ala Ser Leu Arg His Tyr Leu Asn Leu Val Thr Arg Gln Arg
   20 25 30
   Tyr
<210> 189
   <211> 34
   <212> PRT
   <213> Artificial sequence
<220>
   <223> Polypeptide variation
<400> 189
   Leu Lys Pro Glu Ala Pro Gly Glu Asp Ala Ser Pro Glu Glu Leu Asn
   1 5 10 15
   Arg Tyr Tyr Ala Ser Leu Arg His Tyr Leu Asn Leu Val Thr Arg Gln
   20 25 30
   Arg Tyr
<210> 190
   <211> 34
   <212> PRT
   <213> Artificial Sequence
<220>
   <223> Polypeptide variation
<400> 190
   Val Lys Pro Glu Ala Pro Gly Glu Asp Ala Ser Pro Glu Glu Leu Asn
   1 5 10 15
   Arg Tyr Tyr Ala Ser Leu Arg His Tyr Leu Asn Leu Val Thr Arg Gln
   20 25 30
   Arg Tyr
<210> 191
   <211> 13
   <212> PRT
   <213> Artificial Sequence
<220>
   <223> Polypeptide variation
<400> 191
   Leu Lys His Tyr Leu Asn Leu Val Thr Arg Gln Arg Tyr
   1               5                   10
<210> 192
   <211> 14
   <212> PRT
   <213> Artificial Sequence
<220>
   <223> Polypeptide variation
<400> 192
   Ser Leu Lys His Tyr Leu Asn Leu Val Thr Arg Gln Arg Tyr
   1               5                   10
<210> 193
   <211> 15
   <212> PRT
   <213> Artificial sequence
<220>
   <223> Polypeptide variation
<400> 193
   Ala Ser Leu Lys His Tyr Leu Asn Leu Val Thr Arg Gln Arg Tyr
   1               5                   10                  15
<210> 194
   <211> 16
   <212> PRT
   <213> Artificial Sequence
<220>
   <223> Polypeptide variation
<400> 194
   Tyr Ala Ser Leu Lys His Tyr Leu Asn Leu Val Thr Arg Gln Arg Tyr
   1               5                   10                  15
<210> 195
   <211> 17
   <212> PRT
   <213> Artificial Sequence
<220>
   <223> Polypeptide variation
<400> 195
   Tyr Tyr Ala Ser Leu Lys His Tyr Leu Asn Leu Val Thr Arg Gln Arg
   1               5                   10                  15
   Tyr
<210> 196
   <211> 18
   <212> PRT
   <213> Artificial sequence
<220>
   <223> Polypeptide variation
<400> 196
   Arg Tyr Tyr Ala Ser Leu Lys His Tyr Leu Asn Leu Val Thr Arg Gln
   1               5                   10                  15
   Arg Tyr
<210> 197
   <211> 19
   <212> PRT
   <213> Artificial Sequence
<220>
   <223> Polypeptide variation
<400> 197
   Asn Arg Tyr Tyr Ala Ser Leu Lys His Tyr Leu Asn Leu Val Thr Arg
   1               5                   10                  15
   Gln Arg Tyr
<210> 198
   <211> 20
   <212> PRT
   <213> Artificial Sequence
<220>
   <223> Polypeptide variation
<400> 198
   Leu Asn Arg Tyr Tyr Ala Ser Leu Lys His Tyr Leu Asn Leu Val Thr
   1               5                   10                  15
   Arg Gln Arg Tyr
               20
<210> 199
   <211> 21
   <212> PRT
   <213> Artificial Sequence
<220>
   <223> Polypeptide variation
<400> 199
   Glu Leu Asn Arg Tyr Tyr Ala Ser Leu Lys His Tyr Leu Asn Leu Val
   1               5                   10                  15
   Thr Arg Gln Arg Tyr
               20
<210> 200
   <211> 22
   <212> PRT
   <213> Artificial Sequence
<220>
   <223> Polypeptide variation
<400> 200
   Glu Glu Leu Asn Arg Tyr Tyr Ala Ser Leu Lys His Tyr Leu Asn Leu
   1               5                   10                  15
   Val Thr Arg Gln Arg Tyr
               20
<210> 201
   <211> 23
   <212> PRT
   <213> Artificial Sequence
<220>
   <223> Polypeptide variation
<400> 201
   Pro Glu Glu Leu Asn Arg Tyr Tyr Ala Ser Leu Lys His Tyr Leu Asn
   1               5                   10                  15
   Leu Val Thr Arg Gln Arg Tyr
               20
<210> 202
   <211> 23
   <212> PRT
   <213> Artificial Sequence
<220>
   <223> Polypeptide variation
<400> 202
   Ser Glu Glu Leu Asn Arg Tyr Tyr Ala Ser Leu Lys His Tyr Leu Asn
   1               5                   10                  15
   Leu Val Thr Arg Gln Arg Tyr
               20
<210> 203
   <211> 24
   <212> PRT
   <213> Artificial sequence
<220>
   <223> Polypeptide variation
<400> 203
   Ala Ser Glu Glu Leu Asn Arg Tyr Tyr Ala Ser Leu Lys His Tyr Leu
   1               5                   10                  15
   Asn Leu Val Thr Arg Gln Arg Tyr
               20
<210> 204
   <211> 25
   <212> PRT
   <213> Artificial Sequence
<220>
   <223> Polypeptide variation
<400> 204
   Asp Ala Ser Glu Glu Leu Asn Arg Tyr Tyr Ala Ser Leu Lys His Tyr
   1               5                   10                  15
   Leu Asn Leu Val Thr Arg Gln Arg Tyr
               20                  25
<210> 205
   <211> 26
   <212> PRT
   <213> Artificial Sequence
<220>
   <223> Polypeptide variation
<400> 205
   Glu Asp Ala Ser Glu Glu Leu Asn Arg Tyr Tyr Ala Ser Leu Lys His
   1               5                   10                  15
   Tyr Leu Asn Leu Val Thr Arg Gln Arg Tyr
               20                  25
<210> 206
   <211> 28
   <212> PRT
   <213> Artificial sequence
<220>
   <223> Polypeptide variation
<400> 206
   Gly Glu Asp Ala Ser Pro Glu Glu Leu Asn Arg Tyr Tyr Ala Ser Leu
   1               5                   10                  15
   Lys His Tyr Leu Asn Leu Val Thr Arg Gln Arg Tyr
               20                  25
<210> 207
   <211> 29
   <212> PRT
   <213> Artificial sequence
<220>
   <223> Polypeptide variation
<400> 207
   Pro Gly Glu Asp Ala Ser Pro Glu Glu Leu Asn Arg Tyr Tyr Ala Ser
   1               5                   10                  15
   Leu Lys His Tyr Leu Asn Leu Val Thr Arg Gln Arg Tyr
               20                  25
<210> 208
   <211> 29
   <212> PRT
   <213> Artificial Sequence
<220>
   <223> Polypeptide variation
<400> 208
   Ala Pro Gly Glu Asp Ala Ser Glu Glu Leu Asn Arg Tyr Tyr Ala Ser
   1               5                   10                  15
   Leu Lys His Tyr Leu Asn Leu Val Thr Arg Gln Arg Tyr
               20                  25
<210> 209
   <211> 30
   <212> PRT
   <213> Artificial Sequence
<220>
   <223> Polypeptide variation
<400> 209
   Glu Ala Pro Gly Glu Asp Ala Ser Glu Glu Leu Asn Arg Tyr Tyr Ala
   1               5                   10                  15
   Ser Leu Lys His Tyr Leu Asn Leu Val Thr Arg Gln Arg Tyr
               20                  25                  30
<210> 210
   <211> 32
   <212> PRT
   <213> Artificial Sequence
<220>
   <223> Polypeptide variation
<400> 210
   Pro Glu Ala Pro Gly Glu Asp Ala Ser Pro Glu Glu Leu Asn Arg Tyr
   1               5                   10                  15
   Tyr Ala Ser Leu Lys His Tyr Leu Asn Leu Val Thr Arg Gln Arg Tyr
               20                  25                  30
<210> 211
   <211> 32
   <212> PRT
   <213> Artificial Sequence
<220>
   <223> Polypeptide variation
<400> 211
   Lys Pro Glu Ala Pro Gly Glu Asp Ala Ser Glu Glu Leu Asn Arg Tyr
   1               5                   10                  15
   Tyr Ala Ser Leu Lys His Tyr Leu Asn Leu Val Thr Arg Gln Arg Tyr
               20                  25                  30
<210> 212
   <211> 33
   <212> PRT
   <213> Artificial sequence
<220>
   <223> Polypeptide variation
<400> 212
   Ile Lys Pro Glu Ala Pro Gly Glu Asp Ala Ser Glu Glu Leu Asn Arg
   1               5                   10                  15
   Tyr Tyr Ala Ser Leu Lys His Tyr Leu Asn Leu Val Thr Arg Gln Arg
               20                  25                  30
   Tyr
<210> 213
   <211> 13
   <212> PRT
   <213> Artificial Sequence
<220>
   <223> Polypeptide variation
<220>
   <221> MOD_RES
   <222> (1)..(1)
   <223> ACETYLATION
<400> 213
   Leu Arg His Tyr Ile Asn Leu Ile Thr Arg Gln Arg Tyr
   1               5                   10
<210> 214
   <211> 13
   <212> PRT
   <213> Artificial sequence
<220>
   <223> Polypeptide variation
<220>
   <221> MOD_RES
   <222> (1)..(1)
   <223> ACETYLATION
<400> 214
   Leu Arg His Tyr Leu Asn Leu Leu Thr Arg Gln Arg Tyr
   1               5                   10
<210> 215
   <211> 13
   <212> PRT
   <213> Artificial Sequence
<220>
   <223> Polypeptide variation
<400> 215
   Leu Arg His Tyr Leu Asn Leu Leu Thr Arg Gln Arg Tyr
   1               5                   10
<210> 216
   <211> 24
   <212> PRT
   <213> Artificial Sequence
<220>
   <223> Polypeptide variation
<400> 216
   Pro Ala Glu Asp Leu Ala Gln Tyr Ala Ala Glu Leu Arg His Tyr Leu
   1               5                   10                  15
   Asn Leu Leu Thr Arg Gln Arg Tyr
               20
<210> 217
   <211> 20
   <212> PRT
   <213> Artificial Sequence
<220>
   <223> Polypeptide variation
<220>
   <221> MISC_FEATURE
   <222> (1)..(1)
   <223> H
<220>
   <221> MOD_RES
   <222> (20)..(20)
   <223> AMIDATION
<400> 217
   Leu Ala Arg Tyr Tyr Ser Ala Leu Arg His Tyr Ile Asn Leu Ile Thr
   1               5                   10                  15
   Arg Gln Arg Tyr
               20
<210> 218
   <211> 20
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (20)..(20)
   <223> AMIDATION
<400> 218
   Met Ala Arg Tyr Tyr Ser Ala Leu Arg His Tyr Ile Asn Leu Ile Thr
   1               5                   10                  15
   Arg Gln Arg Tyr
               20
<210> 219
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 219
   Ala Arg Tyr Tyr Ser Ala Leu Arg His Phe Ile Asn Leu Ile Thr Arg
   1               5                   10                  15
   Gln Arg Tyr
<210> 220
   <211> 20
   <212> PRT
   <213> Artificial Sequence
<220>
   <223> Polypeptide variation
<220>
   <221> MISC_FEATURE
   <222> (1)..(1)
   <223> D Ala
<220>
   <221> MOD_RES
   <222> (1)..(1)
   <223> ACETYLATION
<220>
   <221> MOD_RES
   <222> (20)..(20)
   <223> AMIDATION
<400> 220
   Xaa Ala Arg Tyr Tyr Ser Ala Leu Arg His Tyr Ile Asn Leu Ile Thr
   1               5                   10                  15
   Arg Gln Arg Tyr
               20
<210> 221
   <211> 18
   <212> PRT
   <213> Artificial Sequence
<220>
   <223> Polypeptide variation
<220>
   <221> MIST_FEATURE
   <222> (1)..(1)
   <223> N terminus is bonded to H
<220>
   <221> MOD_RES
   <222> (18)..(18)
   <223> AMIDATION
<400> 221
   Arg Tyr Tyr Ser Ala Leu Arg His Tyr Ile Asn Leu Ile Thr Arg Gln
   1               5                   10                  15
   Arg Tyr
<210> 222
   <211> 20
   <212> PRT
   <213> Artificial Sequence
<220>
   <223> Polypeptide variation
<220>
   <221> MOD_RES
   <222> (1)..(1)
   <223> Nle
<220>
   <221> MISC_FEATURE
   <222> (1)..(1)
   <223> N terminus is bonded to H
<220>
   <221> MOD_RES
   <222> (20)..(20)
   <223> AMIDATION
<400> 222
   Xaa Ala Arg Tyr Tyr Ser Ala Leu Arg His Tyr Ile Asn Leu Ile Thr
   1               5                   10                  15
   Arg Gln Arg Tyr
               20
<210> 223
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MIST_FEATURE
   <222> (1)..(1)
   <223> D ser
<220>
   <221> MOD_RES
   <222> (19)..(19)
   <223> AMIDATION
<400> 223
   Xaa Arg Tyr Tyr Ser Ala Leu Arg His Tyr Ile Asn Leu Ile Thr Arg
   1 5 10 15
   Gln Arg Tyr
<210> 224
   <211> 20
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (20)..(20)
   <223> AMIDATION
<400> 224
   Ala Ala Arg Tyr Ser His Ala Leu Arg His Tyr Ile Asn Leu Ile Thr
   1 5 10 15
   Arg Gln Arg Tyr
   20
<210> 225
   <211> 19
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MISC_FEATURE
   <222> (1)..(1)
   <223> D Ile
<220>
   <221> MOD_RES
   <222> (19)..(19)
   <223> AMIDATION
<400> 225
   Xaa Arg Tyr Tyr Ser Ala Leu Arg His Tyr Ile Asn Leu Ile Thr Arg
   1 5 10 15
   Gln Arg Tyr
<210> 226
   <211> 20
   <212> PRT
   <213> Artificial Sequence
<220>
   <223> Polypeptide variation
<220>
   <221> MOD_RES
   <222> (20)..(20)
   <223> AMIDATION
<220>
   <221> MOD_RES
   <222> (1) .. (1)
   <223> ACETYLATION
<400> 226
   Arg Ala Arg Tyr Tyr Ser Ala Leu Arg His Tyr Ile Asn Leu Ile Thr
   1 5 10 15
   Arg Gln Arg Tyr
   20
<210> 227
   <211> 18
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (18)..(18)
   <223> AMIDATION
<400> 227
   Gln Tyr Tyr Ser Ala Leu Arg His Tyr Ile Asn Leu Ile Thr Arg Gln
   1 5 10 15
   Arg Tyr
<210> 228
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 228
   Ala Arg Phe Tyr Ser Ala Leu Arg His Tyr Ile Asn Leu Ile Thr Arg
   1 5 10 15
   Gln Arg Tyr
<210> 229
   <211> 20
   <212> PRT
   <213> Artificial Sequence
<220>
   <223> Polypeptide variation
<220>
   <221> MISC_FEATURE
   <222> (1)..(1)
   <223> MeLeu
<220>
   <221> MISC_FEATURE
   <222> (1)..(1)
   <223> N terminus is bonded to H
<220>
   <221> MOD_RES
   <222> (20)..(20)
   <223> AMIDATION
<400> 229
   Xaa Ala Arg Tyr Tyr Ser Ala Leu Arg His Tyr Ile Asn Leu Ile Thr
   1 5 10 15
   Arg Gln Arg Tyr
   20
<210> 230
   <211> 20
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (20)..(20)
   <223> AMIDATION
<220>
   <221> MOD_RES
   <222> (1)..(1)
   <223> METHYLATION
<400> 230
   Leu Ala Arg Tyr Tyr Ser Ala Leu Arg His Tyr Ile Asn Leu Ile Thr
   1 5 10 15
   Arg Gln Arg Tyr
   20
<210> 231
   <211> 20
   <212> PRT
   <213> Artificial Sequence
<220>
   <223> Polypeptide variation
<220>
   <221> MISC_FEATURE
   <222> (1)..(1)
   <223> desamino
<220>
   <221> MOD_RES
   <222> (19)..(19)
   <223> AMIDATION
<400> 231
   Xaa Ala Arg Tyr Tyr Ser Ala Leu Arg His Tyr Ile Asn Leu Ile Thr
   1               5                   10                  15
   Arg Gln Arg Tyr
               20
<210> 232
   <211> 19
   <212> PRT
   <213> Artificial Sequence
<220>
   <223> Polypeptide variation
<220>
   <221> MOD_RES
   <222> (19)..(19)
   <223> AMIDATION
<220>
   <221> MOD_RES
   <222> (1)..(1)
   <223> FORMYLATION
<400> 232
   Ala Arg Tyr Tyr Ser Glu Leu Arg Arg Tyr Ile Asn Leu Ile Thr Arg
   1               5                   10                  15
   Gln Arg Tyr
<210> 233
   <211> 20
   <212> PRT
   <213> Artificial sequence
<220>
   <223> Polypeptide variation
<220>
   <221> MOD_RES
   <222> (1)..(1)
   <223> Nva
<220>
   <221> MISC_FEATURE
   <222> (1)..(1)
   <223> N terminus is bonded to H
<220>
   <221> MOD_RES
   <222> (20)..(20)
   <223> AMIDATION
<400> 233
   Xaa Ala Arg Tyr Ala Ser Ala Leu Arg His Tyr Leu Asn Leu Ile Thr
   1               5                   10                  15
   Arg Gln Arg Tyr
               20
<210> 234
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 234
   Ala Arg Tyr Tyr Thr Gln Leu Arg His Tyr Ile Asn Leu Ile Thr Arg
   1               5                   10                  15
   Gln Arg Tyr
<210> 235
   <211> 20
   <212> PRT
   <213> Artificial Sequence
<220>
   <223> Polypeptide variation
<220>
   <221> MISC_FEATURE
   <222> (1)..(1)
   <223> desamino
<220>
   <221> MISC_FEATURE
   <222> (1)..(1)
   <223> N terminus is bonded to H
<220>
   <221> MOD_RES
   <222> (20)..(20)
   <223> AMIDATION
<400> 235
   Leu Ala Arg Tyr Tyr Ser Asn Leu Arg His Tyr Ile Asn Val Ile Thr
   1 5 10 15
   Arg Gln Arg Tyr
   20
<210> 236
   <211> 19
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 236
   Ala Arg Tyr Tyr Asp Ser Leu Arg His Tyr Ile Asn Thr Ile Thr Arg
   1 5 10 15
   Gln Arg Tyr
<210> 237
   <211> 19
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 237
   Ala Arg Tyr Tyr Ser Ala Leu Gln His Tyr Ile Asn Leu Leu Thr Arg
   1 5 10 15
   Pro Arg Tyr
<210> 238
   <211> 20
   <212> PRT
   <213> Artificial Sequence
<220>
   <223> Polypeptide variation
<220>
   <221> MISC_EATURE
   <222> (1)..(1)
   <223> N terminus is bonded to H
<220>
   <221> MOD_RES
   <222> (20)..(20)
   <223> AMIDATION
<400> 238
   Leu Ala Arg Tyr Tyr Ser Ala Leu Arg Gln Tyr Arg Asn Leu Ile Thr
   1               5                   10                  15
   Arg Gln Arg Phe
               20
<210> 239
   <211> 18
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (18)..(18)
   <223> AMIDATION
<400> 239
   Arg Tyr Tyr Ala Ser Leu Arg His Tyr Leu Asn Leu Val Thr Arg Gln
   1               5                   10                  15
   Arg Phe
<210> 240
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 240
   Ser Arg Tyr Tyr Ala Ser Leu Arg His Tyr Leu Asn Leu Val Thr Arg
   1               5                   10                  15
   Gln Arg Tyr
<210> 241
   <211> 19
   <212> PRT
   <213> Artificial Sequence
<220>
   <223> Polypeptide variation
<220>
   <221> MOD_RES
   <222> (1)..(1)
   <223> ACETYLATION
<220>
   <221> MOD_RES
   <222> (19)..(19)
   <223> AMIDATION
<400> 241
   Ser Arg Tyr Tyr Ala Ser Leu Arg His Phe Leu Asn Leu Val Thr Arg
   1               5                   10                  15
   Gln Arg Tyr
<210> 242
   <211> 20
   <212> PRT
   <213> Artificial Sequence
<220>
   <223> Polypeptide variation
<220>
   <221> MOD_RES
   <222> (1)..(1)
   <223> Nle
<220>
   <221> MISC_FEATURE
   <222> (1) .. (1)
   <223> N terminus is bonded to H
<220>
   <221> MOD_RES
   <222> (20)..(20)
   <223> AMIDATION
<400> 242
   Xaa Ala Arg Tyr Tyr Asn Ala Leu Arg His Phe Ile Asn Leu Ile Thr
   1               5                   10                  15
   Arg Gln Arg Tyr
               20
<210> 243
   <211> 19
   <212> PRT
   <213> Artificial sequence
<220>
   <223> Polypeptide variation
<220>
   <221> MISC_FEATURE
   <222> (1) .. (1)
   <223> D isomer of Ala
<220>
   <221> MISC_FEATURE
   <222> (1)..(1)
   <223> N terminus is bonded to H
<220>
   <221> MOD_RES
   <222> (19)..(19)
   <223> AMIDATION
<400> 243
   Xaa Arg Tyr Glu Ser Ala Leu Arg His Tyr Ile Asn Leu Ile Thr Arg
   1               5                   10                  15
   His Arg Tyr
<210> 244
   <211> 21
   <212> PRT
   <213> Artificial Sequence
<220>
   <223> Polypeptide variation
<220>
   <221> MOD_RES
   <222> (21)..(21)
   <223> AMIDATION
<220>
   <221> MISC_FEATURE
   <222> (1)..(1)
   <223> Bz
<400> 244
   Xaa Leu Ala Arg Tyr Tyr Ser Ala Leu Arg His Tyr Ile Asn Leu Ile
   1 5 10 15
   Thr Arg Pro Arg Phe
   20
<210> 245
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 245
   Ala Leu Tyr Tyr Ser Ala Leu Arg His Phe Val Asn Leu Ile Thr Arg
   1 5 10 15
   Gln Arg Tyr
<210> 246
   <211> 19
   <212> PRT
   <213> Artificial sequence
<220>
   <223> Polypeptide variation
<220>
   <221> MISC_FEATURE
   <222> (1)..(1)
   <223> D Ala
<220>
   <221> MOD_RES
   <222> (19)..(19)
   <223> AMIDATION
<400> 246
   Xaa Arg Tyr Tyr Ser Ala Leu Arg His Tyr Val Asn Leu Ile Phe Arg
   1               5                   10                  15
   Gln Arg Tyr
<210> 247
   <211> 18
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MISC_FEATURE
   <222> (1)..(1)
   <223> MeSer
<220>
   <221> MOD_RES
   <222> (18)..(18)
   <223> AMIDATION
<400> 247
   Xaa Tyr Tyr Ser Ala Leu Arg His Tyr Ile Asn Met Ile Thr Arg Gln
   1               5                   10                  15
   Arg Phe
<210> 248
   <211> 20
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MOD_RES
   <222> (20)..(20)
   <223> AMIDATION
<400> 248
   Arg Ile Arg Tyr Tyr Ser Ala Leu Arg His Phe Ile Asn Leu Ile Thr
   1 5 10 15
   Arg Gln Arg Phe
   20
<210> 249
   <211> 20
   <212> PRT
   <213> Artificial sequence
<220>
   <223> Polypeptide variation
<220>
   <221> MISC_FEATURE
   <222> (1)..(1)
   <223> N terminal is bonded to H
<220>
   <221> MOD_RES
   <222> (20)..(20)
   <223> AMIDATION
<400> 249
   Leu Ser Arg Tyr Tyr Ser Ala Leu Arg His Phe Ile Asn Leu Ile Thr
   1 5 10 15
   Arg Gln Arg Tyr
   20
<210> 250
   <211> 19
   <212> PRT
   <213> Artificial Sequence
<220>
   <223> Polypeptide variation
<220>
   <221> MOD_RES
   <222> (19)..(19)
   <223> AMIDATION
<220>
   <221> MISC_FEATURE
   <222> (1)..(1)
   <223> Xaa is MeIle
<400> 250
<210> 251
   <211> 19
   <212> PRT
   <213> Artificial Sequence
<220>
   <223> Polypeptide variation
<220>
   <221> MISC_FEATURE
   <222> (1)..(1)
   <223> D Ser
<220>
   <221> MISC_FEATURE
   <222> (1)..(1)
   <223> N terminus is bonded to H
<220>
   <221> MOD_RES
   <222> (19)..(19)
   <223> AMIDATION
<400> 251
   Xaa Arg Tyr Tyr Ser Ala Leu Arg His Tyr Ile Asn Leu Ile Thr Arg
   1               5                   10                  15
   Gln Arg Phe
<210> 252
   <211> 20
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (20)..(20)
   <223> AMIDATION
<400> 252
   Met Ala Arg Tyr Tyr Ser Asp Leu Arg Arg Tyr Ile Asn Leu Ile Thr
   1               5                   10                  15
   Arg Gln Arg Tyr
               20
<210> 253
   <211> 19
   <212> PRT
   <213> Artificial sequence
<220>
   <223> Polypeptide variation
<220>
   <221> MISC_FEATURE
   <222> (1)..(1)
   <223> N terminus is bonded to H
<220>
   <221> MOD_RES
   <222> (19)..(19)
   <223> AMIDATION
<400> 253
   Ala Arg Tyr Tyr Ser Glu Leu Arg His Tyr Ile Ile Leu Ile Thr Arg
   1               5                   10                  15
   Gln Arg Tyr
<210> 254
   <211> 20
   <212> PRT
   <213> Artificial Sequence
<220>
   <223> Polypeptide variation
<220>
   <221> MISC_FEATURE
   <222> (1)..(1)
   <223> D Ala
<220>
   <221> MOD_RES
   <222> (20)..(20)
   <223> AMIDATION
<400> 254
   Xaa Ala Arg Tyr Tyr Ser Ala Leu Arg His Tyr Ile Asn Leu Ile Thr
   1               5                   10                  15
   Arg Gln Arg Tyr
               20
<210> 255
   <211> 15
   <212> PRT
   <213> Artificial Sequence
<220>
   <223> Polypeptide variation
<400> 255
   Ala Ser Leu Arg His Trp Leu Asn Leu Val Thr Arg Gln Arg Tyr
   1 5 10 15
<210> 256
   <211> 35
   <212> PRT
   <213> Artificial sequence
<220>
   <223> Polypeptide variation
<220>
   <221> MISC_FEATURE
   <222> (25)..(25)
   <223> im DNP HIS; 2,2 diphenylalanine Hisitidine
<220>
   <221> MOD_RES
   <222> (35)..(35)
   <223> AMIDATION
<400> 256
   Tyr Pro Ala Lys Glu Ala Pro Gly Glu Asp Ala Ser Pro Glu Glu Leu
   1 5 10 15
   Ser Thr Tyr Tyr Ala Ser Leu Arg Xaa Tyr Leu Asn Leu Val Thr Arg
   20 25 30
   Glx Arg Tyr
   35
<210> 257
   <211> 15
   <212> PRT
   <213> Artificial sequence
<220>
   <223> Polypeptide variation
<220>
   <221> MOD_RES
   <222> (15)..(15)
   <223> AMIDATION
<400> 257
   Ala Ser Leu Arg His Tyr Leu Asn Leu Val Thr Arg Gln Arg Tyr
   1 5 10 15
<210> 258
   <211> 15
   <212> PRT
   <213> Artificial Sequence
<220>
   <223> Polypeptide variation
<220>
   <221> MOD_RES
   <222> (15)..(15)
   <223> AMIDATION
<400> 258
   Ala Ser Leu Arg His Tyr Leu Asn Leu Val Ala Arg Gln Arg Tyr
   1 5 10 15
<210> 259
   <211> 15
   <212> PRT
   <213> Artificial sequence
<220>
   <223> Polypeptide variation
<220>
   <221> MOD_RES
   <222> (15)..(15)
   <223> AMIDATION
<400> 259
   Ala Ala Leu Arg His Tyr Leu Asn Leu Val Ala Arg Gln Arg Tyr
   1 5 10 15
<210> 260
   <211> 15
   <212> PRT
   <213> Artificial Sequence
<220>
   <223> Polypeptide variation
<220>
   <221> MOD_RES
   <222> (15)..(15)
   <223> AMIDATION
<400> 260
   Ala Ser Leu Arg His Tyr Glu Asn Leu Val Thr Arg Gln Arg Tyr
   1 5 10 15
<210> 261
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MISC_FEATURE
   <222> (13)..(13)
   <223> Xaa is ornithine
<220>
   <221> MOD_RES
   <222> (15)..(15)
   <223> AMIDATION
<400> 261
   Ala Ser Leu Arg His Tyr Leu Asn Leu Val Thr Arg Xaa Arg Tyr
   1               5                   10                  15
<210> 262
   <211> 15
   <212> PRT
   <213> Artificial sequence
<220>
   <223> Polypeptide variation
<220>
   <221> MISC_FEATURE
   <222> (5)..(5)
   <223> Xaa is p.cl.Pro; 4 chlorophenylalanine
<220>
   <221> MOD_RES
   <222> (1)..(1)
   <223> N alpha ACETYLATION
<220>
   <221> MOD_RES
   <222> (15)..(15)
   <223> AMIDATION
<400> 262
   Ala Ser Leu Arg Xaa Tyr Leu Asn Leu Val Thr Arg Gln Arg Tyr
   1               5                   10                  15
<210> 263
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 263
   Ala Ser Leu Arg His Tyr Glu Asn Leu Val Thr Arg Gln Arg Tyr
   1 5 10 15
<210> 264
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is N Me Tyr
<400> 264
   Ala Ser Leu Arg His Phe Glu Asn Leu Val Thr Arg Gln Arg Xaa
   1 5 10 15
<210> 265
   <211> 15
   <212> PRT
   <213> Artificial Sequence
<220>
   <223> Polypeptide variation
<220>
   <221> MISC_FEATURE
   <222> (13)..(13)
   <223> Xaa is Ornithine
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is N Me Tyr
<400> 265
   Ala Ser Leu Arg His Tyr Glu Asn Leu Val Thr Arg Xaa Arg Xaa
   1 5 10 15
<210> 266
   <211> 15
   <212> PRT
   <213> Artificial Sequence
<220>
   <223> Polypeptide variation
<220>
   <221> LIPID
   <222> (1)..(1)
   <223> N alpha myristoyl
<220>
   <221> MOD_RES
   <222> (15)..(15)
   <223> AMIDATION
<400> 266
   Ala Ser Leu Arg His Tyr Leu Asn Leu Val Thr Arg Gln Arg Tyr
   1 5 10 15
<210> 267
   <211> 15
   <212> PRT
   <213> Artificial Sequence
<220>
   <223> Polypeptide variation
<220>
   <221> MISC_FEATURE
   <222> (1)..(1)
   <223> N alpha naphthateneacetyl
<220>
   <221> MOD_RES
   <222> (15)..(15)
   <223> AMIDATION
<400> 267
   Ala Ser Leu Arg His Tyr Leu Asn Leu Val Thr Arg Gln Arg Tyr
   1 5 10 15
<210> 268
   <211> 15
   <212> PRT
   <213> Artificial Sequence
<220>
   <223> Polypeptide variation
<220>
   <221> MISC_FEATURE
   <222> (15)..(15)
   <223> Xaa is N Me Tyr
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (13)..(13)
   <223> Xaa is ornithine
<400> 268
   Ala Ser Leu Arg His Phe Glu Asn Leu Val Thr Arg Xaa Arg Xaa
   1               5                   10                  15
<210> 269
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 269
   Ala Ser Leu Arg His Tyr Leu Asn Leu Val Thr Arg Gln Arg Tyr
   1               5                   10                  15
<210> 270
   <211> 15
   <212> PRT
   <213> Artificial Sequence
<220>
   <223> Polypeptide variation
<220>
   <221> MISC_FEATURE
   <222> (6)..(6)
   <223> Xaa is 3 benzothienyalanine
<220>
   <221> MOD_RES
   <222> (7)..(7)
   <223> N alpha ACETYLATION
<220>
   <221> MOD_RES
   <222> (1)..(1)
   <223> N alpha ACETYLATION
<400> 270
   Ala Ser Leu Arg His Xaa Leu Asn Leu Val Thr Arg Gln Arg Tyr
   1               5                   10                  15
<210> 271
   <211> 16
   <212> PRT
   <213> Artificial Sequence
<220>
   <223> Polypeptide variation
<220>
   <221> MISC_FEATURE
   <222> (1)..(1)
   <223> Xaa is 4,4' biphenylalanine
<220>
   <221> MOD_RES
   <222> (1)..(1)
   <223> N alpha ACETYLATION
<220>
   <221> MOD_RES
   <222> (16)..(16)
   <223> AMIDATION
<400> 271
   Xaa Ala Ser Leu Arg His Tyr Leu Asn Leu Val Thr Arg Gln Arg Tyr
   1               5                   10                  15
<210> 272
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (6)..(6)
   <223> Xaa is 3 benzothienyalanine
<400> 272
   Ala Ser Leu Arg His Xaa Leu Asn Leu Val Thr Arg Gln Arg Tyr
   1               5                   10                  15
<210> 273
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (6)..(6)
   <223> Xaa is 3 benzothienyalanine
<400> 273
   Ala Ser Leu Arg His Xaa Leu Asn Leu Val Thr Arg Gln Arg Tyr
   1               5                   10                  15
<210> 274
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 274
   Ala Ser Leu Arg His Trp Leu Asn Leu Val Thr Arg Gln Arg Tyr
   1 5 10 15
<210> 275
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 275
   Ala Ser Leu Arg His Trp Leu Asn Leu Val Thr Arg Gln Arg Tyr
   1 5 10 15
<210> 276
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (6)..(6)
   <223> Xaa is 2 thienylalanine
<400> 276
   Ala Ser Leu Arg Asn Xaa Leu Asn Leu Val Thr Arg Gln Arg Tyr
   1               5                   10                  15
<210> 277
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (6)..(6)
   <223> Xaa is tetrahydroisoquinoline
<400> 277
   Ala Ser Leu Arg His Xaa Leu Asn Leu Val Thr Arg Gln Arg Tyr
   1               5                   10                  15
<210> 278
   <211> 3
   <212> PRT
   <213> Homo sapiens
<400> 278
<210> 279
   <211> 11
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (11)..(11)
   <223> AMIDATION
<400> 279
   His Phe Leu Asn Leu Val Thr Arg Gln Arg Tyr
   1 5 10
<210> 280
   <211> 15
   <212> PRT
   <213> Artificial Sequence
<220>
   <223> Polypeptide variation
<220>
   <221> MOD_RES
   <222> (15)..(15)
   <223> AMIDATION
<220>
   <221> MOD_RES
   <222> (1)..(1)
   <223> ACETYLATION
<220>
   <221> MISC_FEATURE
   <222> (15)..(15)
   <223> Xaa is 2 thienylalanine
<400> 280
   Ala Ser Leu Arg His Phe Leu Asn Leu Val Thr Arg Gln Arg Xaa
   1 5 10 15
<210> 281
   <211> 16
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (16)..(16)
   <223> AMIDATION
<220>
   <221> MISC_FEATURE
   <222> (6)..(6)
   <223> Xaa is 4 Thiazolylalanine
<400> 281
   Ala Ser Leu Arg His Xaa Phe Leu Asn Leu Val Thr Arg Gln Arg Tyr
   1               5                   10                  15
<210> 282
   <211> 16
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (16)..(16)
   <223> AMIDATION
<220>
   <221> ISC_FEATURE
   <222> (6)..(6)
   <223> Xaa is 4 Thiazolylalanine
<400> 282
   Ala Ser Leu Arg His Xaa Phe Leu Asn Leu Val Thr Arg Gln Arg Tyr
   1               5                   10                  15
<210> 283
   <211> 3
   <212> PRT
   <213> Homo sapiens
<400> 283
<210> 284
   <211> 3
   <212> PRT
   <213> Homo sapiens
<400> 284
<210> 285
   <211> 3
   <212> PRT
   <213> Homo sapiens
<400> 285
<210> 286
   <211> 3
   <212> PRT
   <213> Homo sapiens
<400> 286
<210> 287
   <211> 3
   <212> PRT
   <213> Homo sapiens
<400> 287
<210> 288
   <211> 3
   <212> PRT
   <213> Homo sapiens
<400> 288
<210> 289
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 289
   Phe Ser Leu Arg Asn Phe Leu Asn Leu Val Thr Arg Gln Arg Tyr
   1 5 10 15
<210> 290
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 290
   Tyr Ser Leu Arg His Phe Leu Asn Leu Val Thr Arg Gln Arg Tyr
   1 5 10 15
<210> 291
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 291
   Ala Ser Leu Arg His Tyr Trp Asn Leu Val Thr Arg Gln Arg Tyr
   1 5 10 15
<210> 292
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 292
   Ala Ser Leu Arg His Tyr Leu Asn Trp Val Thr Arg Gln Arg Tyr
   1 5 10 15
<210> 293
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 293
   Ala Ser Leu Arg Ala Phe Leu Asn Leu Val Thr Arg Gln Arg Tyr
   1 5 10 15
<210> 294
   <211> 14
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (14)..(14)
   <223> AMIDATION
<220>
   <221> MISC_FEATURE
   <222> (5)..(5)
   <223> Xaa is 3' benzothienyalanine
<400> 294
   Ala Ser Leu Arg Xaa Leu Asn Leu Val Thr Arg Gln Arg Tyr
   1               5                   10
<210> 295
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 295
   Ala Ser Leu Arg His Phe Leu Asn Leu Val Thr Arg Gln Arg Tyr
   1               5                   10                  15
<210> 296
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 296
   Ala Ser Leu Arg His Phe Leu Asn Leu Val Thr Arg Gln Arg Phe
   1 5 10 15
<210> 297
   <211> 15
   <212> PRT
   <213> Artificial sequence
<220>
   <223> Polypeptide variation
<220>
   <221> MISC_FEATURE
   <222> (11)..(11)
   <223> Xaa is D form of Trp
<220>
   <221> MOD_RES
   <222> (11)..(11)
   <223> AMIDATION
<220>
   <221> MOD_RES
   <222> (11)..(11)
   <223> N alpha ACETYLATION
<400> 297
   Ala Ser Leu Arg His Phe Leu Asn Leu Val Xaa Arg Gln Arg Tyr
   1 5 10 15
<210> 298
   <211> 13
   <212> PRT
   <213> Artificial sequence
<220>
   <223> Polypeptide variation
<220>
   <221> MOD_RES
   <222> (13)..(13)
   <223> AMIDATION
<220>
   <221> MISC_FEATURE
   <222> (1)..(1)
   <223> N terminus is bonded to CH3CO
<400> 298
   Leu Arg His Tyr Leu Asn Leu Leu Thr Arg Gln Arg Tyr
   1 5 10
<210> 299
   <211> 13
   <212> PRT
   <213> Artificial Sequence
<220>
   <223> Polypeptide variation
<220>
   <221> MOD_RES
   <222> (13)..(13)
   <223> AMIDATION
<220>
   <221> MISC_FEATURE
   <222> (1)..(1)
   <223> N terminus is bonded to CH3CO
<400> 299
   Leu Arg His Tyr Ile Asn Leu Ile Thr Arg Gln Arg Tyr
   1 5 10
<210> 300
   <211> 13
   <212> PRT
   <213> Artificial Sequence
<220>
   <223> Polypeptide variation
<220>
   <221> MOD_RES
   <222> (1)..(1)
   <223> AMIDATION
<220>
   <221> MOD_RES
   <222> (13)..(13)
   <223> AMIDATION
<400> 300
   Leu Arg His Tyr Leu Asn Leu Leu Thr Arg Gln Arg Tyr
   1 5 10
<210> 301
   <211> 13
   <212> PRT
   <213> Artificial Sequence
<220>
   <223> Polypeptide variation
<220>
   <221> MOD_RES
   <222> (1)..(1)
   <223> AMIDATION
<220>
   <221> MOD_RES
   <222> (13)..(13)
   <223> AMIDATION
<400> 301
   Leu Arg His Tyr Ile Asn Leu Ile Thr Arg Gln Arg Tyr
   1 5 10
<210> 302
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is a pseudopeptide bond consisting of CH2 NH
<220>
   <221> MISC_FEATURE
   <222> (14)..(14)
   <223> Xaa is a pseudopeptide bond consisting of CH2 NH
<220>
   <221> MISC_FEATURE
   <222> (10)..(10)
   <223> Xaa is Norvaline
<220>
   <221> MISC_FEATURE
   <222> (3)..(3)
   <223> Xaa is Norleucine
<220>
   <221> MISC_FEATURE
   <222> (7)..(7)
   <223> Xaa is Norleucine
<220>
   <221> MISC_FEATURE
   <222> (9)..(9)
   <223> Xaa is Norleucine
<400> 302
   Ala Ser Xaa Arg His Trp Xaa Asn Xaa Xaa Thr Arg Gln Xaa Xaa
   1 5 10 15
<210> 303
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is a pseudopeptide bond consisting of CH2 NH
<220>
   <221> MISC_FEATURE
   <222> (14)..(14)
   <223> Xaa is a pseudopeptide bond consisting of CH2 NH
<220>
   <221> MISC_FEATURE
   <222> (3)..(3)
   <223> Xaa is Norleucine
<220>
   <221> MISC_FEATURE
   <222> (7)..(7)
   <223> Xaa is Norleucine
<220>
   <221> MTSC_FEATURE
   <222> (10)..(10)
   <223> Xaa is Norvaline
<400> 303
   Ala Ser Xaa Arg His Trp Xaa Asn Trp Xaa Thr Arg Gln Xaa Xaa
   1 5 10 15
<210> 304
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is a pseudopeptide bond consisting of CH2 NH
<220>
   <221> MISC_FEATURE
   <222> (14)..(14)
   <223> Xaa is a pseudopeptide bond consisting of CH2 NH
<220>
   <221> MISC_FEATURE
   <222> (3)..(3)
   <223> Xaa is Norleucine
<220>
   <221> MISC_FEATURE
   <222> (7)..(7)
   <223> Xaa is Norleucine
<220>
   <221> MISC_FEATURE
   <222> (9)..(9)
   <223> Xaa is Norleucine
<220>
   <221> MISC_FEATURE
   <222> (10)..(10)
   <223> Xaa is Norvaline
<400> 304
   Ala Ser Xaa Arg His Phe Xaa Asn Xaa Xaa Thr Arg Gln Xaa Xaa
   1               5                   10                  15
<210> 305
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is a pseudopeptide bond consisting of CH2 NH
<220>
   <221> MISC_FEATURE
   <222> (14)..(14)
   <223> Xaa is a pseudopeptide bond consisting of CH2 NH
<220>
   <221> MISC_FEATURE
   <222> (3)..(3)
   <223> Xaa is Norleucine
<220>
   <221> MISC_FEATURE
   <222> (7)..(7)
   <223> Xaa is Norleucine
<220>
   <221> MISC_FEATURE
   <222> (10)..(10)
   <223> Xaa is Norvaline
<400> 305
   Ala Ser Xaa Arg His Phe Xaa Asn Trp Xaa Thr Arg Gln Xaa Xaa
   1               5                   10                  15
<210> 306
   <211> 12
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (12)..(12)
   <223> AMIDATION
<220>
   <221> MISC_FEATURE
   <222> (12)..(12)
   <223> Xaa is a pseudopeptide bond consisting of CH2 NH
<220>
   <221> MISC_FEATURE
   <222> (11)..(11)
   <223> Xaa is a pseudopeptide bond consisting of CH2 NH
<400> 306
   Arg His Tyr Leu Asn Trp Val Thr Arg Gln Xaa Xaa
   1 5 10
<210> 307
   <211> 12
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (12)..(12)
   <223> AMIDATION
<400> 307
   Arg His Tyr Leu Asn Trp Val Thr Arg Gln Arg Tyr
   1 5 10
<210> 308
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (14)..(14)
   <223> Xaa is a psuedopeptide bond consisting of CH2 NH2
<220>
   <221> MISC_FEATURE
   <222> (15)..(15)
   <223> Xaa is a psuedopeptide bond consisting of CH2 NH2
<220>
   <221> MISC_FEATURE
   <222> (7)..(7)
   <223> Xaa is Norleucine
<220>
   <221> MISC_FEATURE
   <222> (10)..(10)
   <223> Xaa is Norvaline
<400> 308
   Ala Ser Leu Arg His Tyr Xaa Asn Trp Xaa Thr Arg Gln Xaa Xaa
   1               5                   10                  15
<210> 309
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<220>
   <221> MISC_FEATURE
   <222> (15)..(15)
   <223> Xaa is a pseudopeptide bond consisting of CH2 NH2
<220>
   <221> MISC_FEATURE
   <222> (14)..(14)
   <223> Xaa is a pseudopeptide bond consisting of CH2 NH2
<220>
   <221> MISC_FEATURE
   <222> (3)..(3)
   <223> Xaa is Norleucine
<220>
   <221> MISC_FEATURE
   <222> (7)..(7)
   <223> Xaa is Norleucine
<220>
   <221> MISC_FEATURE
   <222> (10)..(10)
   <223> Xaa is Norvaline
<400> 309
   Ala Ser Xaa Arg His Tyr Xaa Asn Trp Xaa Thr Arg Gln Xaa Xaa
   1               5                   10                  15
<210> 310
   <211> 9
   <212> PRT
   <213> Artificial Sequence
<220>
   <223> Polypeptide variation
<220>
   <221> MISC_FEATURE
   <222> (9)..(9)
   <223> bonded to OCH3
<220>
   <221> MISC_FEATURE
   <222> (1)..(1)
   <223> N terminus is bonded to H
<400> 310
   Ile Asn Pro Ile Tyr Arg Leu Arg Tyr
   1               5
<210> 311
   <211> 9
   <212> PRT
   <213> Artificial Sequence
<220>
   <223> Polypeptide variation
<220>
   <221> DISULFID
   <222> (4)..(4)
   <223> sequence is linked to identical sequence by a disulfide bond
<220>
   <221> MISC_FEATURE
   <222> (1)..(1)
   <223> N terminus is bonded to H
<220>
   <221> MISC_FEATURE
   <222> (9)..(9)
   <223> C terminus is bonded to NH2
<400> 311
   Ile Asn Pro Cys Tyr Arg Leu Arg Tyr
   1 5
<210> 312
   <211> 6
   <212> PRT
   <213> Artificial Sequence
<220>
   <223> Polypeptide variation
<220>
   <221> MISC_FEATURE
   <222> (6)..(6)
   <223> C terminus is bonded to OCH3
<220>
   <221> DISULFID
   <222> (1)..(1)
   <223> sequence is linked to an identical sequence
<220>
   <221> MISC_FEATURE
   <222> (1)..(1)
   <223> N terminus is bonded to H
<400> 312
   Cys Tyr Arg Leu Arg Tyr
   1 5
<210> 313
   <211> 8
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MISC_FEATURE
   <222> (3)..(4)
   <223> Connected by NH CH CO
<220>
   <221> MISC_FEATURE
   <222> (3)..(4)
   <223> Identical peptide chains are connected by (CH2)4 at the CH o f NH CH CO
<400> 313
   Ile Asn Pro Tyr Arg Leu Arg Tyr
   1 5
<210> 314
   <211> 10
   <212> PRT
   <213> Artificial Sequence
<220>
   <223> Polypeptide variation
<220>
   <221> MISC_FEATURE
   <222> (1)..(1)
   <223> N terminus is bonded to H
<220>
   <221> MISC_FEATURE
   <222> (5)..(5)
   <223> C terminus is bonded to OCH3
<400> 314
   Tyr Arg Leu Arg Tyr Tyr Arg Leu Arg Tyr
   1               5                   10
<210> 315
   <211> 34
   <212> PRT
   <213> Artificial Sequence
<220>
   <223> Polypeptide variation
<220>
   <221> DISULFID
   <222> (18)..(22)
   <223>
<400> 315
   Ser Lys Pro Asp Asn Pro Gly Glu Asp Ala Pro Ala Glu Asp Met Ala
   1               5                   10                  15
   Arg Cys Tyr Ser Ala Cys Arg His Tyr Ile Asn Leu Ile Thr Arg Gln
               20                  25                  30
   Arg Tyr
<210> 316
   <211> 12
   <212> PRT
   <213> Artificial sequence
<220>
   <223> Polypeptide variation
<400> 316
   Arg His Tyr Leu Asn Leu Ile Gly Arg Gln Arg Tyr
   1 5 10
<210> 317
   <211> 12
   <212> PRT
   <213> Artificial Sequence
<220>
   <223> Polypeptide variation
<220>
   <221> MOD_RES
   <222> (3)..(7)
   <223> ACETYLATION
<400> 317
   Arg His Gly Leu Asn Leu Leu Gly Arg Gln Arg Tyr
   1 5 10
<210> 318
   <211> 10
   <212> PRT
   <213> Artificial Sequence
<220>
   <223> Polypeptide variation
<400> 318
   Tyr Ile Asn Leu Ile Tyr Arg Leu Arg Tyr
   1 5 10
<210> 319
   <211> 11
   <212> PRT
   <213> Artificial Sequence
<220>
   <223> Polypeptide variation
<400> 319
   His Tyr Ile Asn Leu Ile Tyr Arg Leu Arg Tyr
   1 5 10
<210> 320
   <211> 12
   <212> PRT
   <213> Artificial Sequence
<220>
   <223> Polypeptide variation
<400> 320
   Arg His Tyr Ile Asn Leu Ile Tyr Arg Leu Arg Tyr
   1 5 10
<210> 321
   <211> 10
   <212> PRT
   <213> Artificial Sequence
<220>
   <223> Polypeptide variation
<400> 321
   Tyr Ile Asn Leu Leu Tyr Arg Gln Arg Tyr
   1 5 10
<210> 322
   <211> 15
   <212> PRT
   <213> Artificial sequence
<220>
   <223> Polypeptide variation
<220>
   <221> MISC_FEATURE
   <222> (5)..(5)
   <223> Xaa is 6 amino hexanoic acid
<400> 322
   Tyr Pro Ser Leu Xaa Tyr Ile Asn Leu Ile Tyr Arg Leu Arg Tyr
   1 5 10 15
<210> 323
   <211> 9
   <212> PRT
   <213> Artificial Sequence
<220>
   <223> Polypeptide variation
<400> 323
   Ile Asn Leu Ile Tyr Arg Leu Arg Tyr
   1 5
<210> 324
   <211> 12
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (12)..(12)
   <223> AMIDATION
<400> 324
   Arg His Phe Leu Asn Leu Val Thr Arg Gln Arg Tyr
   1               5                   10
<210> 325
   <211> 15
   <212> PRT
   <213> Artificial sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 325
   Ala Ser Leu Arg His Phe Leu Asn Leu Val Thr Arg Gln Arg Tyr
   1               5                   10                  15
<210> 326
   <211> 15
   <212> PRT
   <213> Artificial Sequence
<220>
   <223> Polypeptide variation
<220>
   <221> MISC_FEATURE
   <222> (1)..(1)
   <223> N terminal is bonded to H
<220>
   <221> MOD_RES
   <222> (15)..(15)
   <223> AMIDATION
<400> 326
   Ala Ser Leu Arg His Phe Leu Asn Leu Val Thr Arg Gln Arg Tyr
   1 5 10 15
<210> 327
   <211> 9
   <212> PRT
   <213> Artificial Sequence
<220>
   <223> Polypeptide variation
<220>
   <221> MOD_RES
   <222> (9)..(9)
   <223> AMIDATION
<220>
   <221> MOD_RES
   <222> (1)..(1)
   <223> N alpha ACETYLATION
<400> 327
   Ala Ser Leu Arg Thr Arg Gln Arg Tyr
   1 5
<210> 328
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MISC_FEATURE
   <222> (6)..(6)
   <223> Xaa is 2 thienylalanine
<220>
   <221> MOD_RES
   <222> (15)..(15)
   <223> AMIDATION
<400> 328
   Ala Ser Leu Arg His Xaa Leu Asn Leu Val Thr Arg Gln Arg Tyr
   1 5 10 15
<210> 329
   <211> 15
   <212> PRT
   <213> Artificial Sequence
<220>
   <223> Polypeptide variation
<220>
   <221> MOD_RES
   <222> (1)..(1)
   <223> N alpha ACETYLATION
<220>
   <221> MOD_RES
   <222> (15)..(15)
   <223> AMIDATION
<400> 329
   Tyr Ser Leu Arg His Phe Leu Asn Leu Val Thr Arg Gln Arg Tyr
   1 5 10 15
<210> 330
   <211> 5
   <212> PRT
   <213> Artificial sequence
<220>
   <223> Polypeptide variation
<400> 330
   Asp Asp Asp Asp Tyr
   1 5
<210> 331
   <211> 3
   <212> PRT
   <213> Artificial Sequence
<220>
   <223> Polypeptide variation
<400> 331
   Gly Pro Arg
   1
<210> 332
   <211> 3
   <212> PRT
   <213> Artificial Sequence
<220>
   <223> Polypeptide variation
<400> 332
   Ala Gly Gly
   1
<210> 333
   <211> 5
   <212> PRT
   <213> Artificial sequence
<220>
   <223> Polypeptide variation
<400> 333
   His Pro Phe His Leu
   1               5
<210> 334
   <211> 34
   <212> PRT
   <213> Homo sapiens
<400> 334
   Ile Lys Pro Glu Ala Pro Gly Glu Asp Ala Ser Pro Glu Glu Leu Asn
   1               5                   10                  15
   Arg Tyr Tyr Ala Ser Leu Arg His Tyr Leu Asn Leu Val Thr Arg Gln
               20                  25                  30
   Arg Tyr
<210> 335
   <211> 34
   <212> PRT
   <213> Artificial Sequence
<220>
   <223> Polypeptide variation
<400> 335
   Ser Lys Pro Asp Asn Pro Gly Glu Asp Ala Pro Ala Glu Asp Met Ala
   1               5                   10                  15
   Arg Tyr Tyr Ser Ala Leu Arg His Tyr Ile Asn Leu Ile Thr Arg Gln
               20                  25                  30
   Arg Tyr
<210> 336
   <211> 37
   <212> PRT
   <213> Homo sapiens
<400> 336
<210> 337
   <211> 36
   <212> PRT
   <213> Homo sapiens
<220>
   <221> MISC_FEATURE
   <222> (36)..(36)
   <223> c terminus is bonded to NH2
<400> 337
<210> 338
   <211> 31
   <212> PRT
   <213> Homo sapiens
<400> 338
<210> 339
   <211> 30
   <212> PRT
   <213> Homo sapiens
<220>
   <221> MISC_FEATURE
   <222> (30)..(30)
   <223> C terminus is bonded to NH2
<400> 339
<210> 340
   <211> 37
   <212> PRT
   <213> Homo sapiens
<400> 340
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asp Thr Lys Arg Asn
               20                  25                  30
   Lys Asn Asn Ile Ala
           35
<210> 341
   <211> 463
   <212> PRT
   <213> homo sapiens
<400> 341